# EUROPEAN PATENT APPLICATION

(11) **EP 4 324 923 A1**
(43) Date of publication of application: **21.02.2024**
(21) Application number: 22787390.8
(22) Date of filing: 31.03.2022
(51) Int. Cl.: C12N 15/113, A61K 31/713, A61P 31/20, A61P 35/00, A61P 3/10, A61P 9/10, A61P 9/12, A61P 3/06, A61P 7/00

(54) **RNA INHIBITOR FOR INHIBITING HEPATITIS B VIRUS GENE EXPRESSION AND APPLICATION THEREOF**

(30) Priority: 13.04.2021 CN 202110394458
(71) Applicant: Kylonova (Xiamen) Biopharma Co., Ltd., Xiamen, Fujian 361022 (CN)
(72) Inventor: MU, Zhuo, Xiamen, Fujian 361022 (CN); WANG, Shengjun, Xiamen, Fujian 361022 (CN)
(74) Representative: Cabinet Chaillot
(86) International application number: PCT/CN2022/084381
(87) International publication number: WO 2022/218163

(57) **Abstract**

Provided are an RNA inhibitor for inhibiting hepatitis B virus (HBV) gene expression and an application thereof. The RNA inhibitor is formed of a sense strand and an antisense strand by means of base pairing; the sense strand and the antisense strand are at least 85% complementary to each other; and - OH at 2' position of glycosyl of some or all of nucleotides is replaced by fluorine or methoxy, and phosphates between at least 3 consecutive nucleotides at the end are thioated. In the structure of the RNA inhibitor, 5'MVIP and 3'MVIP are also comprised, such that the RNA inhibitor has specific liver targeting. The RNA inhibitor can continuously inhibit the synthesis of an HBV surface antigen (HBsAg), can promote the production of HBV surface antibody (HBsAb), has an inhibitory effect on the most common types of HBV, i.e., A, B, C and D, and can be used in combination with a nucleoside analog or an interferon, and can cure hepatitis B functionally.

## Description

### Technical field

The invention belongs to the field of biochemistry, and specifically relates to an RNA inhibitor for inhibiting gene expression of hepatitis B virus and an application thereof. The RNA inhibitor is formed of a sense strand and an antisense strand by means of base pairing, wherein the sense strand and the antisense strand are at least 85% base complementary to each other, and the -OH at 2' position of glycosyl of some or all of nucleotides is replaced by fluorine or methoxy, and phosphates between at least 3 consecutive nucleotides at the end are thioated. The RNA inhibitor of the present invention further comprises 5'MVIP and 3'MVIP in structure so that the RNA inhibitor have liver targeting specificity, wherein the 5'MVIP is coupled to the 5' end of the sense strand and/or the antisense strand of the RNA inhibitor, the 3'MVIP is coupled to the 3' end of the antisense strand and/or the sense strand of the RNA inhibitor, both the 5'MVIP and the 3'MVIP comprises a liver targeting specific ligand X, a branched chain L, a linker B and a linking chain D, the 5'MVIP further comprises a transition point R₁ connected with the 5' end of the sense strand or antisense strand of the RNA inhibitor, the 3'MVIP further comprises a transition point R₂ connected with the 3' end of the sense strand or antisense strand of the RNA inhibitor, the liver targeting specific ligands X, the branched chains L or the linkers B within each of the 5'MVIP and the 3'MVIP or between the 5'MVIP and the 3'MVIP may be the same or different. The RNA inhibitor provided by the present invention has an efficacy that the current clinical first-line drugs for hepatitis B do not have, can directly destroy the function of HBV mRNA as a translation template, and prevent the synthesis of HBV surface antigen (HBsAg). In addition, the RNA inhibitor of the present invention has a significant inhibitory effect on the most common types of HBV, i.e., A, B, C and D, can be used in combination with a nucleoside analog or an interferon, can continuously and efficiently reduce the expression level of HBsAg in HBV mice and promote the production of HBV surface antibody (HBsAb), and can cure hepatitis B functionally.

### Background Art

### RNAi

RNAi (RNA interference) was discovered in an antisense RNA inhibition experiment on *Caenorhabditis elegans* carried out by Andrew Z. Fire et al. in 1998, and this process was named as RNAi. This discovery was recognized by *Science* as one of the top ten scientific advances in 2001, and ranked the first on the list of the top ten scientific advances in 2002. Since then, siRNA with the mechanism of RNAi has attracted much attention as a potential genetic therapeutic drug. In 2006, Andrew Z. Fire and Craig C. Mello won the Nobel Prize for Physiology or Medicine for their contribution in the study of RNAi mechanism. RNAi can be triggered by double stranded RNA (dsRNA) in many organisms, including animals, plants and fungi. In the process of RNAi, a long-chain dsRNA is cleaved or "diced" into small fragments of 21 to 25 nucleotides in length by an endonuclease known as "Dicer". These small fragments are known as small interfering RNA (siRNA), in which the antisense strand (Guide strand) is loaded onto Argonaute protein (AGO2). AGO2 loading occurs in a RISC-loading complex, which is a ternary complex composed of an Argonaute protein, a Dicer and a dsRNA binding protein (briefly referred as TRBP). In the process of loading, the sense strands (Passenger strand) are cleaved by AGO2 and discharged. Then, AGO2 utilizes the antisense strands to bind to mRNAs containing complete complementary sequences, and catalyzes the cleavage of these mRNAs, such that mRNAs are cleaved to lose their function of translation template, which in turn prevents the synthesis of related proteins. After cleavage, the cleaved mRNAs are released, and the RISC-loading complex loaded with the antisense strand was recycled into another round of cleavage.

Hepatitis B is a disease in which pyroptosis (or) fibrosis in varying degrees occurs in the liver due to continuous infection with hepatitis B virus for more than 6 months. According to the World Health Organization, there are about 2 billion people infected in the world, of which about 4 million people are acutely infected every year, and about 350 to 400 million people are infected with hepatitis B, of which 68% are in the African and the Western Pacific regions. There are about 1 million people died from hepatitis B infection-related diseases every year in the word, of which 30% is due to liver cirrhosis, and 45% is due to primary hepatocellular carcinoma. Among the patients with liver cirrhosis and primary hepatocellular carcinoma in China, 77% and 84% were caused by hepatitis B virus respectively. So far, the clinical first-line drugs include nucleoside (NUC) and interferon drugs, and the most important drugs are still nucleoside drugs such as lamivudine, entecavir, adefovir, telbivudine, etc. Tenofovir alafenamide is a new NUC drug latestly marketed, but its application is limited to an extent because it may cause renal damage. Nucleoside drugs have the advantages of high bioavailability and relatively safe oral administration. However, although nucleoside drugs can effectively control the disease, long-term use may lead to drug resistance, HBV DNA, ALT, and liver histology rebound to different extents after drug withdrawal, and long-term administration of the nucleoside drugs leads to obvious side effects, such as kidney damage, infant teratogenicity, etc. The emergence of drug-resistant virus strains is another adverse effect that must be faced with long-term application of nucleoside drugs. The emergence of drug-resistant strains results in greatly reduced cure rate, or even drug failure. Because the inhibition of nucleoside drugs against virus replication is reversible, the course of treatment must be more than one year in order to achieve the maximum curative effect for most of patients, so that drug resistance will appear, and the expected effect will not be achieved. NUC drugs need to be taken every day, and patients' compliance is poor.

Hepatitis B surface antigen (HBsAg), which is the coat protein of hepatitis B virus (HBV), is the first detectable marker of the virus. HBsAg positive is the gold standard for judging HBV infection. For hepatitis B patients, if HBsAg is cleared before cirrhosis, the incidence of cirrhosis and hepatocellular carcinoma will be reduced by 60 times. HBsAg serum clearance is used as one of the treatment endpoint criteria in each of the guidelines of the American Association for the Study of Liver Diseases (AASLD), the Asia Pacific Association for the Study of the Liver (APASL), and the European Association for the Study of the Liver (EASL). In addition, a high level of antigen induces immune tolerance, and a reduced HBsAg level can restore the immunological control of HBV infection. So far, the clinical first-line drugs including nucleoside (NUC) and interferon drugs do not have the effect of reducing HBsAg level, let alone clearing HBsAg.

Treatment of hepatitis B remains a global health challenge. Therefore, there is an urgent need in this field to develop an anti-HBV drug with a new therapeutic mechanism, which can effectively and permanently reduce HBsAg level, so that hepatitis B patients can regenerate HBsAb, and finally can be functionally cured.

### Summary of the Invention

The invention relates to an RNA inhibitor for inhibiting gene expression of hepatitis B virus and an application thereof. The RNA inhibitor is formed of a sense strand and an antisense strand by means of base pairing, wherein the sense strand and the antisense strand are at least 85% base complementary to each other, and the -OH at 2' position of glycosyl of some or all of nucleotides is replaced by fluorine or methoxy, and phosphates between at least 3 consecutive nucleotides at the end are thioated, to enhance its stability *in vivo.* The RNA inhibitor of the present invention further comprises 5'MVIP and 3'MVIP in structure so that the RNA inhibitor have liver targeting specificity, wherein the 5'MVIP is coupled to the 5' end of the sense strand and/or the antisense strand of the RNA inhibitor, the 3'MVIP is coupled to the 3' end of the antisense strand and/or the sense strand of the RNA inhibitor, both the 5'MVIP and the 3'MVIP comprises a liver targeting specific ligand X, a branched chain L, a linker B and a linking chain D, the 5'MVIP further comprises a transition point R₁ connected with the 5' end of the sense strand or antisense strand of the RNA inhibitor, the 3'MVIP further comprises a transition point R₂ connected with the 3' end of the sense strand or antisense strand of the RNA inhibitor, the liver targeting specific ligands X, the branched chains L or the linkers B within each of 5'MVIP and 3'MVIP or between 5'MVIP and 3'MVIP may be the same or different. The RNA inhibitor provided by the present invention has an efficacy that the current clinical first-line drugs for hepatitis B do not have, can directly destroy the function of HBV mRNA as a translation template, and prevent the synthesis of HBV surface antigen (HBsAg). In addition, the RNA inhibitor of the present invention has a significant inhibitory effect on the most common types of HBV, i.e., A, B, C and D, can be used in combination with a nucleoside analog or an interferon, can continuously and efficiently reduce the expression level of HBsAg in HBV mice and promote the production of HBV surface antibody (HBsAb), and can cure hepatitis B functionally. Compared with the disclosed techniques of the same kind, the RNA inhibitor described in the present invention is mainly characterized in that it can promote the production of HBsAb *in vivo,* stimulate to regenerate immunity to HBV *in vivo,* and achieve functional cure of hepatitis B.

In one aspect, the present invention provides an RNA inhibitor for inhibiting gene expression of hepatitis B virus or a pharmaceutically acceptable salt thereof, wherein,
the RNA inhibitor is formed of a sense strand and an antisense strand with a chain length of 15-30, preferably 19-23, by means of base pairing.

In the above-mentioned embodiments, preferably, the sense strand and the antisense strand are at least 85% base complementary to each other,
the -OH at 2' position of glycosyl of some or all of nucleotides of the sense strand or the antisense strand may be replaced, wherein the replacing group is fluorine or methoxy, and
phosphate bonds between at least 3 adjacent nucleotides at the end of the sense strand or antisense strand may be thioated.

More preferably, the sense strand is SEQ ID NO. 1 as shown below or a sequence that differs from SEQ ID NO. 1 by one, two or three nucleotides, and the antisense strand is SEQ ID NO. 58 as shown below or a sequence that differs from SEQ ID NO. 58 by one, two or three nucleotides:

| | | |
|---|---|---|
| sense strand: | 5' ggguuuuucucguugacaa 3' | SEQ ID NO. 1 |
| antisense strand: | 5' uugucaacgagaaaaacccuu 3' | SEQ ID NO. 58 |

wherein, g = guanosine, a = adenosine, u = uridine, c = cytidine.

Alternatively, more preferably, the sense strand is SEQ ID NO. 140 as shown below or a sequence that differs from SEQ ID NO. 140 by one, two or three nucleotides, and the antisense strand is SEQ ID NO. 141 as shown below or a sequence that differs from SEQ ID no. 141 by one, two or three nucleotides:

| | | |
|---|---|---|
| sense strand: | 5'ggguuuuucuuguugacaa 3' | SEQ ID NO. 140 |
| antisense strand: | 5' uugucaacaagaaaaacccuu 3' | SEQ ID NO. 141 |

wherein, g = guanosine, a = adenosine, u = uridine, c = cytidine.

In order to enhance the stability of the above-mentioned RNA inhibitor *in vivo,* the sense strand and antisense strand of the above-mentioned RNA inhibitor may be modified, wherein the nucleotides therein may have a modifying group and the strand may be modified in whole or in part, as long as its activity is not affected or even enhanced.

In a preferred embodiment, the modified sense strand of the RNA inhibitor is SEQ ID NO. 2 as shown below or a sequence that differs from SEQ ID NO. 2 by one, two or three nucleotides, and the modified antisense strand of the RNA inhibitor is SEQ ID NO. 59 as shown below or a sequence that differs from SEQ ID NO. 59 by one, two or three nucleotides:

| | | |
|---|---|---|
| sense strand: | 5' Gs fGs G U fU U fU fU fC U C G U U G A Cs As A 3' | SEQ ID NO. 2 |
| antisense strand: | 5' Us Us G U C A fA C G A G fA A fA fA A C C Cs Us U 3' | SEQ ID NO. 59 |

wherein, G=2'-O-methylguanosine, A=2'-O-methyladenosine, U=2'-O-methyluridine, C=2'-O-methylcytidine; Gs=2'-O-methylguanosine-3'-phosphorothioate, As=2'-O-methyladenosine-3'-phosphorothioate, Us=2'-O-methyluridine-3'-phosphorothioate, Cs=2'-O-methylcytidine-3'-phosphorothioate; fG=2'-fluoroguanosine, fA=2'-fluoroadenosine, fU=2'-fluorouridine, fC=2'-fluorocytidine; fGs=2'-fluoroguanosine-3'-phosphorothioate, fAs=2'-fluoroadenosine-3'-phosphorothioate, fUs=2'-fluorouridine-3'-phosphorothioate, fCs=2'-fluorocytidine-3'-phosphorothioate.

In another preferred embodiment, the modified sense strand of the RNA inhibitor is SEQ ID NO. 142 as shown below or a sequence that differs from SEQ ID NO. 142 by one, two or three nucleotides, and the modified antisense strand of the RNA inhibitor is SEQ ID NO. 143 as shown below or a sequence that differs from SEQ ID NO. 143 by one, two or three nucleotides:

| | | |
|---|---|---|
| sense strand: | 5' Gs Gs G U fU U fU fU fC U U G U U G A Cs As A 3' | SEQ ID NO. 142 |
| antisense strand: | 5'Us Us G U C A fA C A A G fA A fA A A C C Cs Us U 3' | SEQ ID NO. 143 |

wherein, G=2'-O-methylguanosine, A=2'-O-methyladenosine, U=2'-O-methyluridine, C=2'-O-methylcytidine; Gs=2'-O-methylguanosine-3'-phosphorothioate, As=2'-O-methyladenosine-3'-phosphorothioate, Us=2'-O-methyluridine-3'-phosphorothioate, Cs=2'-O-methylcytidine-3'-phosphorothioate; fG=2'-fluoroguanosine, fA=2'-fluoroadenosine, fU=2'-fluorouridine, fC=2'-fluorocytidine; fGs=2'-fluoroguanosine-3'-phosphorothioate, fAs=2'-fluoroadenosine-3'-phosphorothioate, fUs=2'-fluorouridine-3'-phosphorothioate, fCs=2'-fluorocytidine-3'-phosphorothioate.

In the above embodiments, preferably, the RNA inhibitor or a pharmaceutically acceptable salt thereof further comprises a combination of 5'MVIP and 3'MVIP, wherein,
the 5'MVIP and 3'MVIP are ligand structures with a liver targeting specific ligand X, and further comprise a branched chain L, a linker B and a linking chain D;
the 5'MVIP is coupled to the 5' end of the sense strand and/or the antisense strand, and further comprises a transition point R₁ connected to the 5' end of the sense strand or antisense strand;
the 3'MVIP is coupled to the 3' end of the antisense strand and/or the sense strand, and further comprises a transition point R₂ connected to the 3' end of the sense strand or antisense strand;
the 5'MVIP has a structure as shown in general formula I, and the 3'MVIP has a structure as shown in general formula II,

   (X-L)ₙ-13-D-R₁- I

   (X-L)ₘ-B-D-R₂- II
wherein,
n and m are respectively an integer of 0 to 4, preferably 1 to 3, and n+m is an integer of 2 to 6, preferably 2, 3 or 4;
the transition points R₁ and R₂ have a structure containing -NH-, sulfur atom or oxygen atom, and generally at least one -NH-, sulfur atom or oxygen atom is in the structure, R₁ and R₂ are linked to the linking chain D of 5'MVIP and 3'MVIP, and the 5' end and the 3' end of the sense strand and/or the antisense strand respectively through the -NH-, sulfur atom or oxygen atom in the structure, thereby introducing the liver targeting specific ligand X; the transition points R₁ and R₂ may be a straight chain; a straight chain with a branch, or various cyclic structures, the cyclic structure may be, for example, saturated or unsaturated aliphatic carbocyclyl, or 5- or 6-membered heterocyclyl or aromatic hydrocarbonyl containing sulfur, oxygen or nitrogen atom, etc.;
R₁ is preferably -NH(CH₂)ₓCH₂O-, wherein x is an integer of 3 to 12, preferably 4 to 6;
R₂ is preferably -NH(CH₂)ₓ₁CH(OH)(CH₂)ₓ₂CH₂O-, wherein x1 is an integer of 1 to 4, and x2 is an integer of 0 to 4;
the liver targeting specific ligand X is selected from galactose, galactosamine, N-acetylgalactosamine and derivatives thereof, preferably selected from N-acetylgalactosamine and derivatives thereof, and the liver target specific ligands X within each of the 5'MVIP and the 3'MVIP or between the 5'MVIP and the 3'MVIP may be the same or different;
the branched chain L is a C4-C18 straight chain containing -NH-, C=O, O, S, amide group, phosphoryl, thiophosphoryl, C4-C10 aliphatic carbocyclyl, phenyl or a combination thereof, which may have a side chain of ethyl alcohols or carboxylic acids, the branched chain L is preferably a C7-C18 straight chain containing an amide group or a six-membered aliphatic carbocyclyl, and the branched chains L within each of the 5'MVIP and the 3'MVIP or between the 5'MVIP and the 3'MVIP may be the same or different;
the linker B is selected from the following structural formulae: 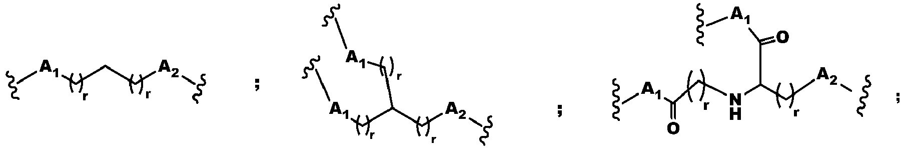 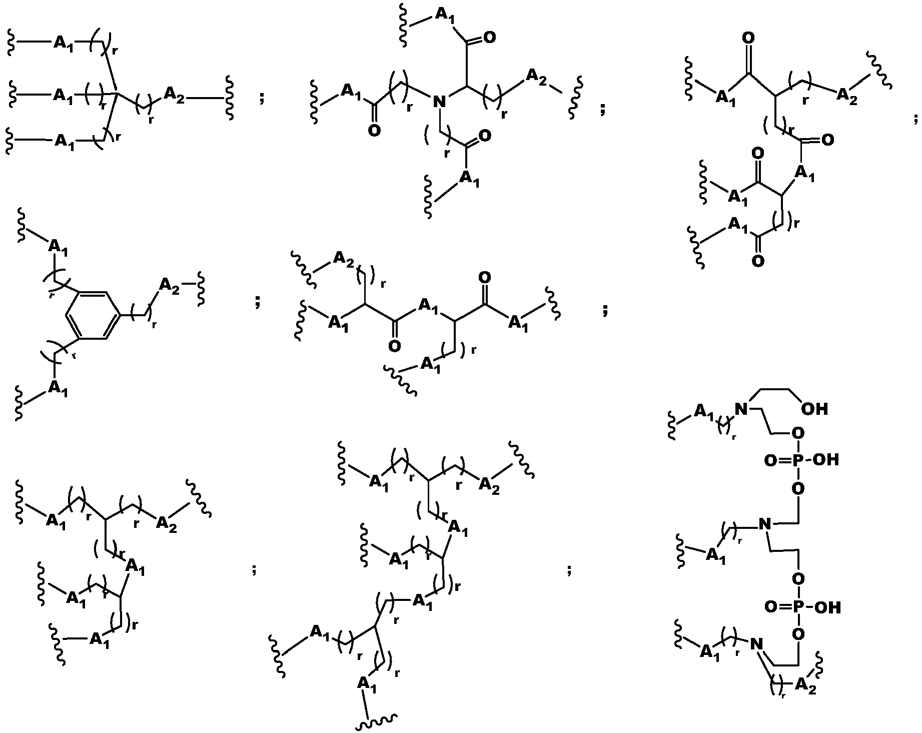
wherein, A₁ and A₂ are each independently C, O, S, -NH-, carbonyl, amide group, phosphoryl or thiophosphoryl, r is an integer of 0 to 4, and the linkers B between the 5'MVIP and the 3'MVIP may be the same or different;
the linking chain D is a C3-C18 straight chain containing -NH-, C=O, O, S, amide group, phosphoryl, thiophosphoryl, aromatic hydrocarbonyl, C4-C10 aliphatic carbocyclyl, 5- or 6-membered heterocyclyl containing 1 to 3 nitrogens or a combination thereof, the C3-C18 straight chain may further have a side chain of methyl alcohols, methyl tert-butyl, methyl phenol, or C5-C6 alicyclyl, the linking chain D is preferably a C3-C10 straight chain containing two C=O, 6-membered aliphatic carbocyclyl or phenyl.

Specifically, in some embodiments, when n=0 (that is, there is no 5'MVIP), the MVIP may have a structure of:

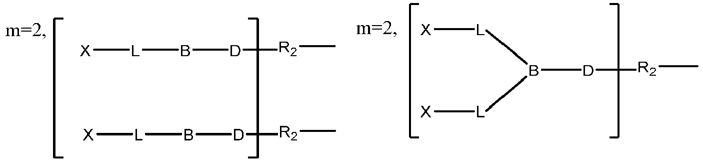

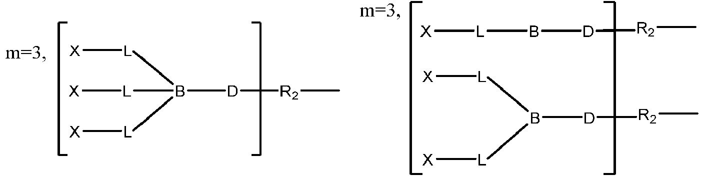

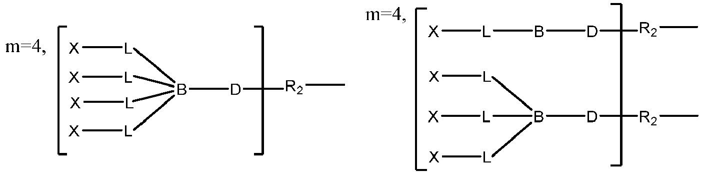

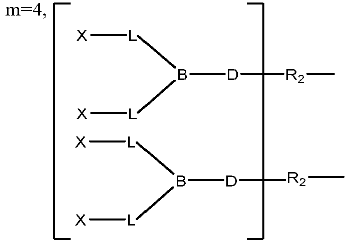

In some embodiments, when n=1, the MVIP may have a structure of:

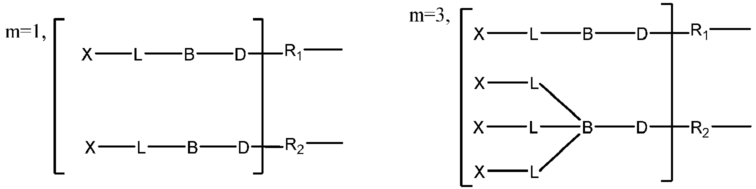

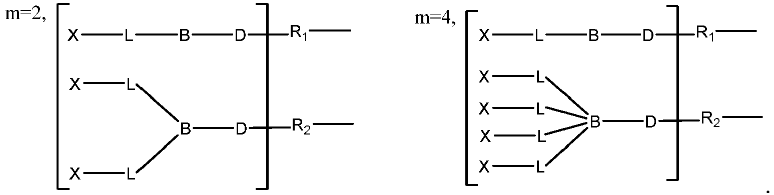

In some embodiments, when n=2, the MVIP may have a structure of:

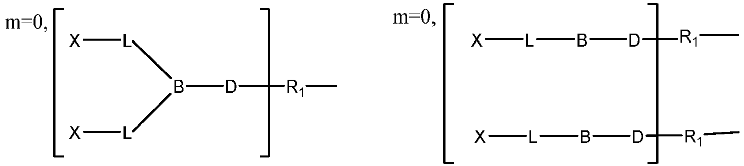

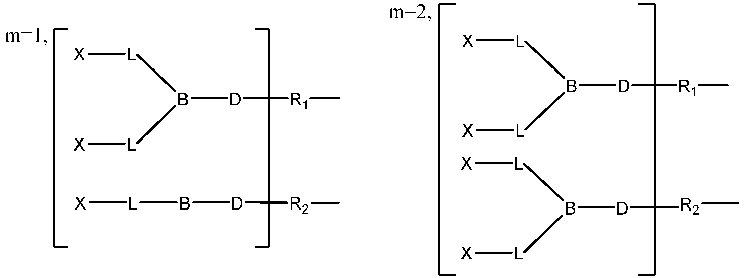

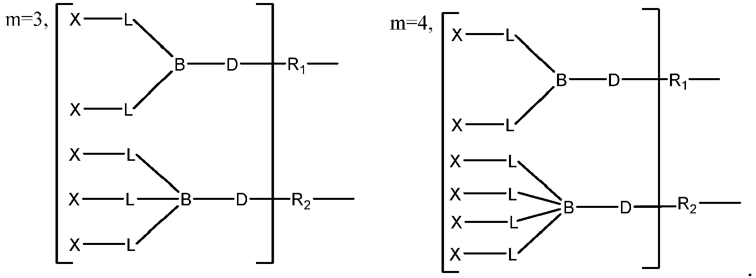

In some embodiments, when n=3, the MVIP may have a structure of:

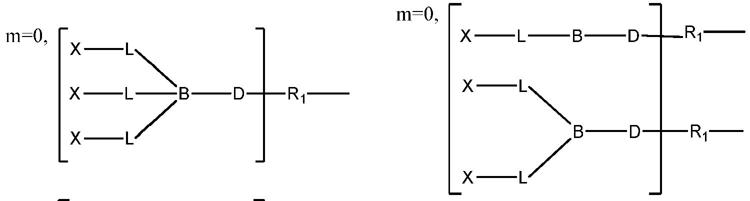

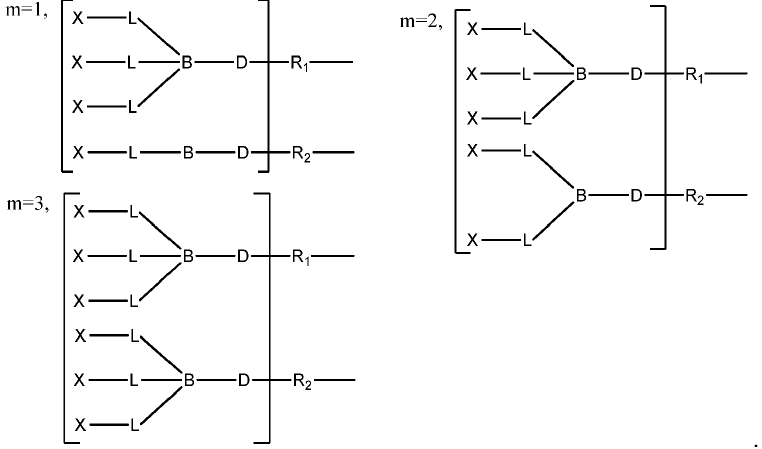

In some embodiments, when n=4, the MVIP may have a structure of:

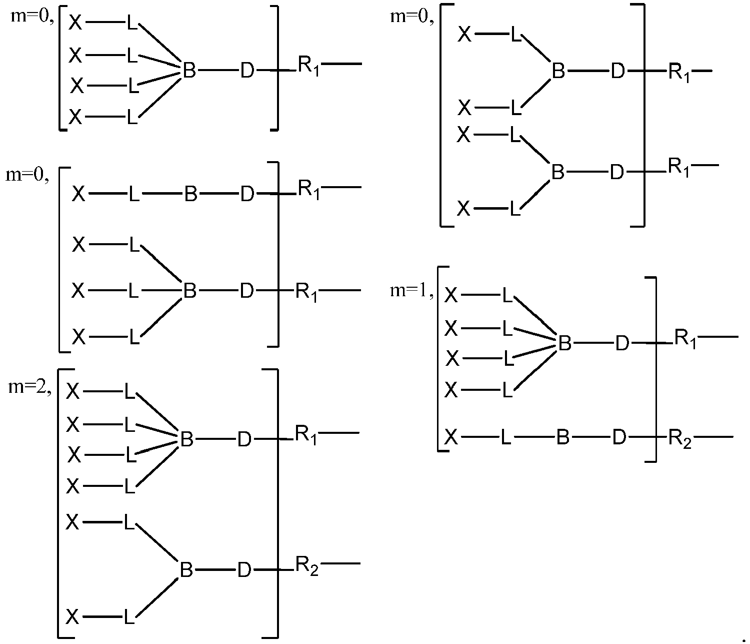

In some embodiments, the n refers to the sum of n in 5'MVIPs at the 5' ends of the sense and antisense strands of the RNA inhibitor, and the m refers to the sum of m in 3'MVIPs at the 3' ends of the sense and antisense strands of the RNA inhibitor.

The liver targeting specific ligand X is selected from structures for enhancing the uptake of RNA inhibitors by liver cells, and may be a lipid, a steroids, a vitamin, a sugar, a protein, a peptide, a polyamine or a peptide mimic moiety. In the RNA inhibitor provided by the present invention, the liver targeting specific ligands X introduced at the end of the sense strand or antisense strand of the RNA inhibitor may be the same or different, for example, in terms of performance, some may be for enhancing liver targeting, some may be moieties for regulating the pharmacokinetics of the RNA inhibitor *in vivo,* and some may be moieties having *in vivo* dissolving activity. In some embodiments, the liver targeting specific ligand X is one or more monosaccharides and derivatives thereof selected from the following structures.

The monosaccharide is one or more selected from the following structures: mannose, galactose, D-arabinose, glucose, fructose, xylose, glucosamine, ribose. Mannose is one or more selected from the following structures: D-mannopyranose, L-mannopyranose, α-D-mannofuranose, β-D-mannofuranose, α-D-mannopyranose, β-D-mannopyranose. Galactose is one or more selected from the following structures: L-galactose, D-galactose, α-D-galactopyranose, β-D-galactopyranose, α-D-galactofuranose, β-D-galactofuranose. Glucose is one or more selected from the following structures: D-glucose, L-glucose, α-D-glucopyranose, β-D-glucopyranose, α-D-glucofuranose, β-D-glucopyranose. Fructose is one or more selected from the following structures: α-D-fructofuranose, α-D-fructopyranose. Xylose is one or more selected from the following structures: D-xylofuranose, L-xylofuranose. Ribose is one or more selected from the following structures: ribose, D-ribose, L-ribose. The monosaccharide derivative is selected from mannose derivatives, galactose derivatives, glucose derivatives, ribose derivatives and other derivatives. The galactose derivative may be selected from α-D-galactosamine, N-acetylgalactosamine, 4-thio-β-D-galactopyranose. The glucose derivative may be selected from 2-amino-3-O-[(R)-1-carboxyethyl]-2-deoxy-P-D-glucopyranose, 2-deoxy-2-methylamino-L-glucopyranose, 2-deoxy-2-sulfoamino-D-glucopyranose, 5-thio-β-D-glucopyranose, methyl 2,3,4-tri-O-acetyl-1-thio-6-O-trityl-α-D-glucopyranoside. The ribose derivative is one or more selected from D-4-thioribose and L-4-thioribose.

In some preferred embodiments, the liver targeting specific ligand X is selected from galactose, galactosamine, N-acetylgalactosamine and derivatives thereof, and has the following general formula: 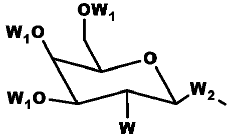 wherein, W₁s are hydrogen or a hydroxyl protecting group, and may be the same or different; W is - OH, -NHCOOH or -NHCO(CH₂)_{q}CH₃, wherein q is an integer of 0 to 4; W₂ is -NH-, O, S or C.

In some embodiments, the liver targeting specific ligand X is preferably one or more selected from the following structural formulae: 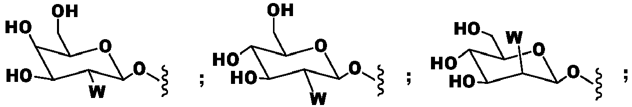 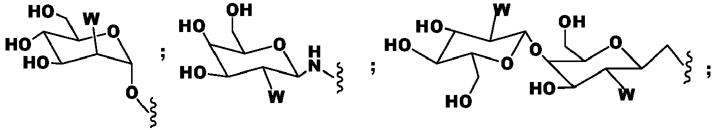 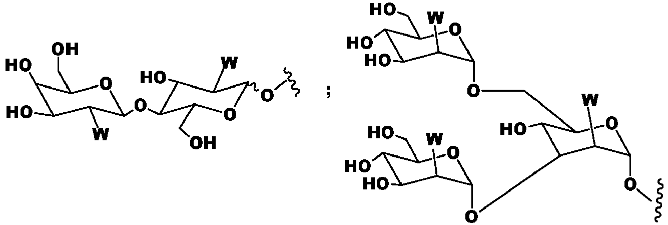 wherein, W is one or two selected from-OH, -NHCOOH or -NHCO(CH₂)_{q}CH₃, wherein q is an integer of 0 to 4.

In some embodiments, the liver targeting specific ligands X in the same 5'MVIP or 3'MVIP structure may be the same or different.

In some embodiments, Xs between the 5'MVIP and the 3'MVIP may be the same or different.

The branched chain L is a C4-C18 straight chain containing -NH-, C=O, O, S, amide group, phosphoryl, thiophosphoryl, C4-C10 aliphatic carbocyclyl, phenyl or a combination thereof, which may have a side chain of ethyl alcohols or carboxylic acids, the branched chain L is preferably a C7-C18 straight chain containing amide group or 6-membered aliphatic carbocyclyl. The length or structure of the branched chain L will affect the activity of the RNA inhibitors of the present invention.

In some embodiments, the branched chains L in the same 5'MVIP or 3'MVIP structure may be the same or different.

In some embodiments, the branched chains L between the 5'MVIP and the 3'MVIP may be the same or different.

In some embodiments, the branched chain L may be one or more selected from the following structural formulae: 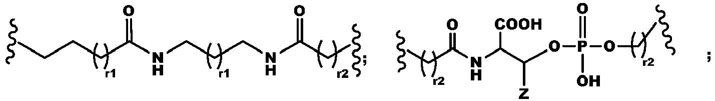 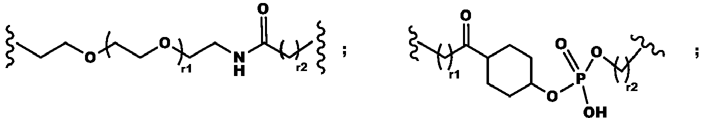 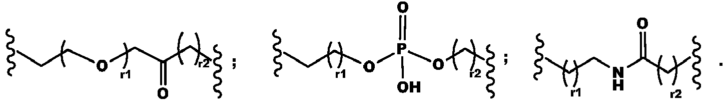 wherein, r1 is a positive integer of 1 to 12, r2 is an integer of 0 to 20, Z is H, an alkyl or an amide group, such as a C1-C5 alkyl, a C1-C5 amide group, such as formamido, etc.

The structure of the linker B is related to the number of the specific ligand X that may be introduced, and the linker B contains -NH-, C, O, S, amide group, phosphoryl, thiophosphoryl, and it is a straight chain when n or m is 1, and has the number of forks of 2, 3 or 4 respectively when n or m is 2, 3 or 4. The linker B may be selected from the following structural formulae: 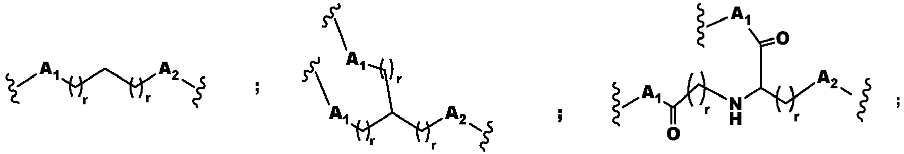 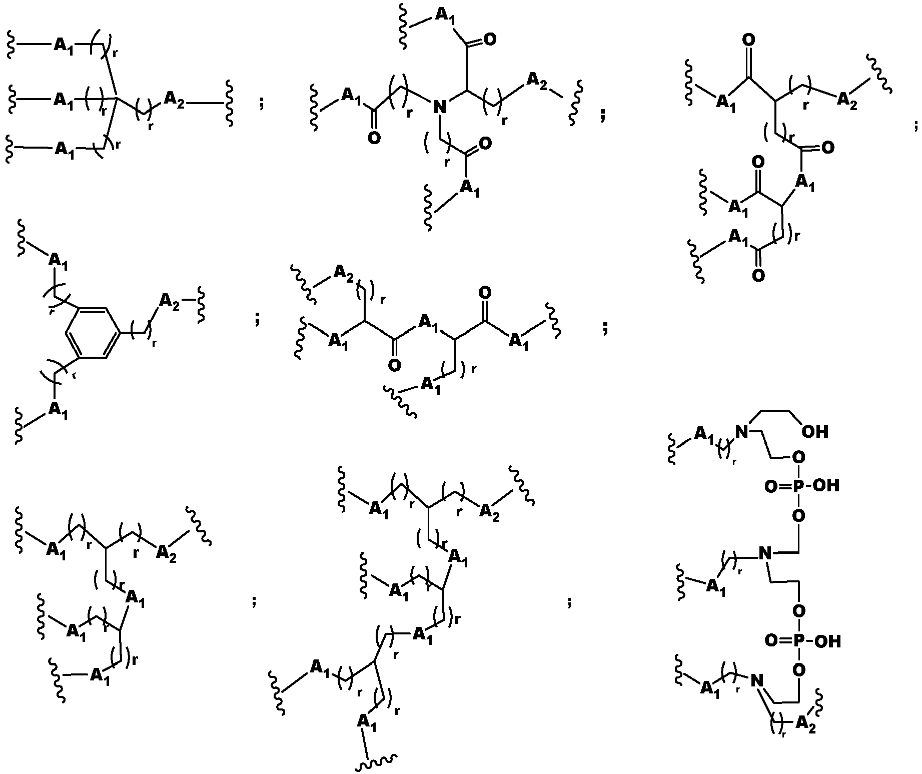 wherein, A₁ and A₂ are each independently C, O, S, -NH-, carbonyl, amide group, phosphoryl or thiophosphoryl, and r is an integer of 0 to 4.

In some embodiments, when n or m is 1, 2, 3 or 4, the linker B is selected from the following structural formulae: 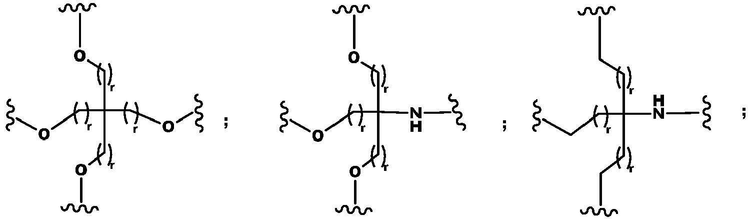 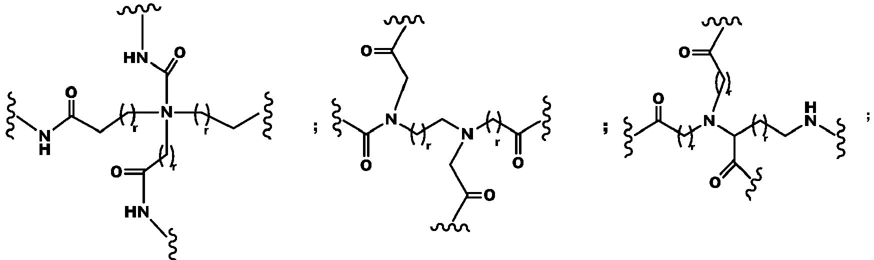 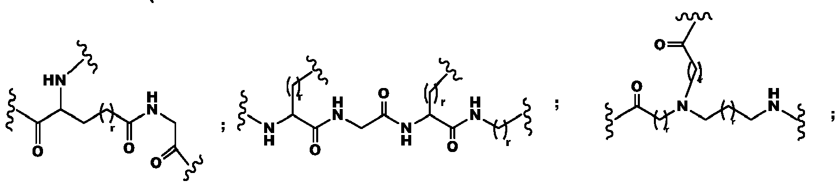 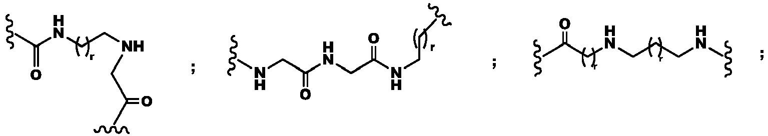 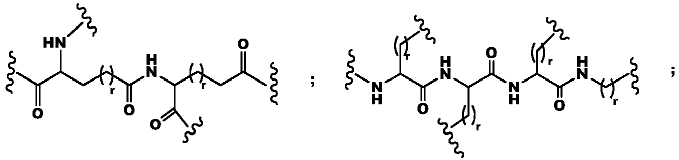 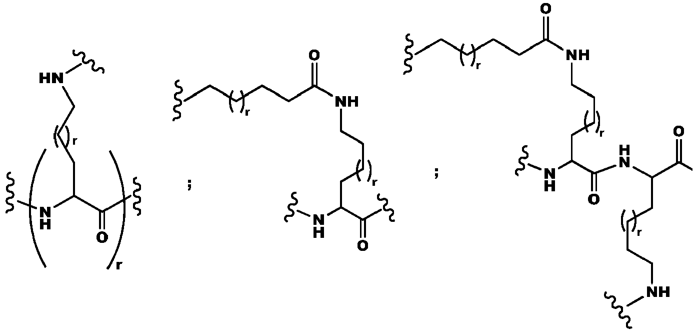 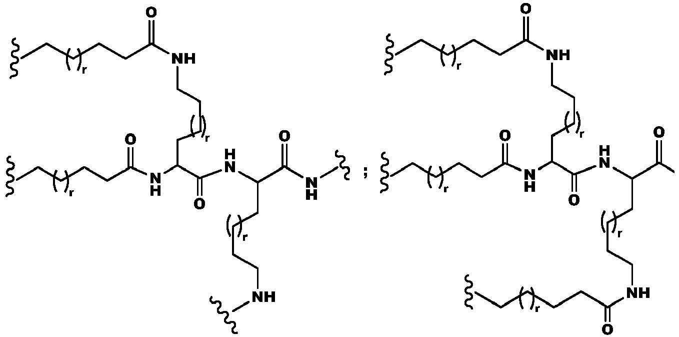 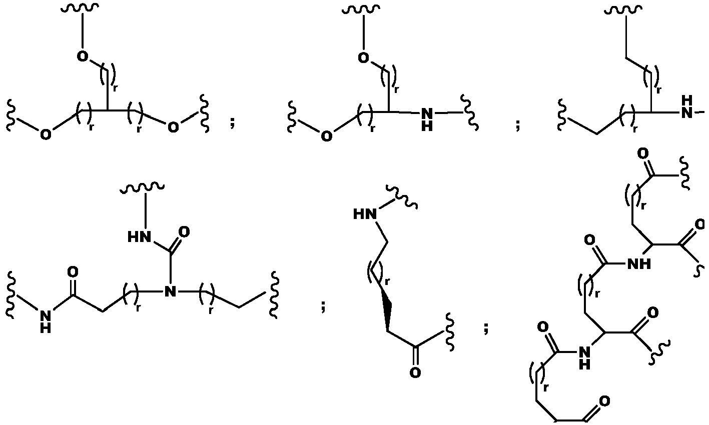 wherein, r is an integer of 0 to 4.

In some embodiments, when n or m is 1, 2, 3 or 4, the linker B is selected from the following structural formulae:

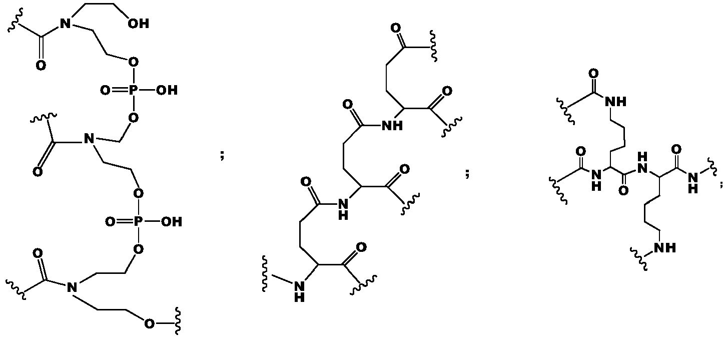

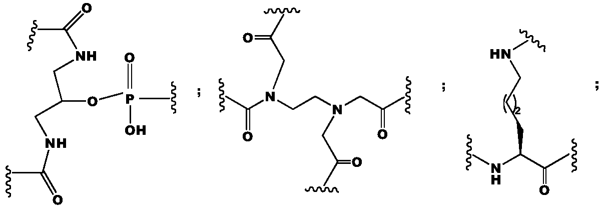

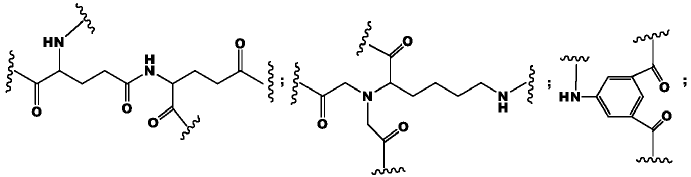

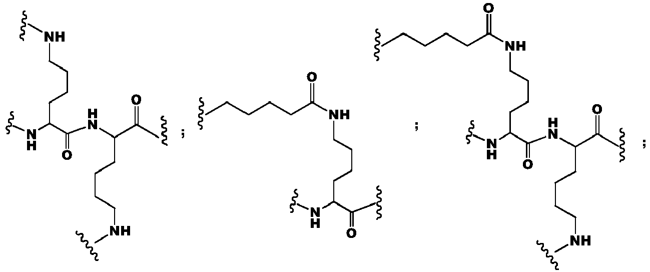

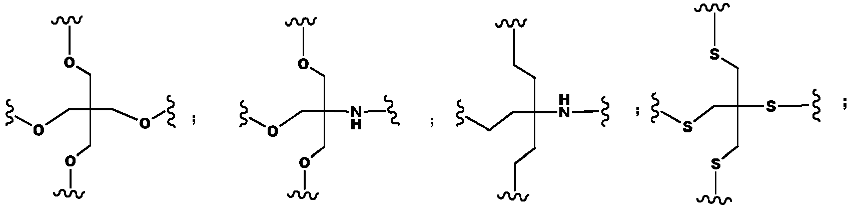

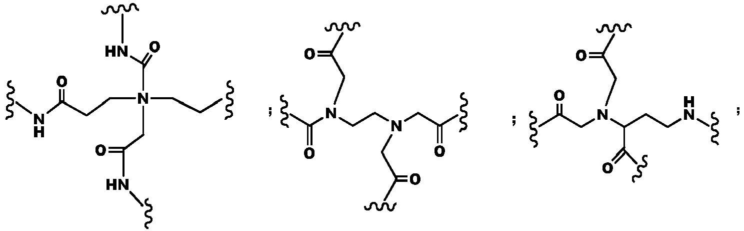

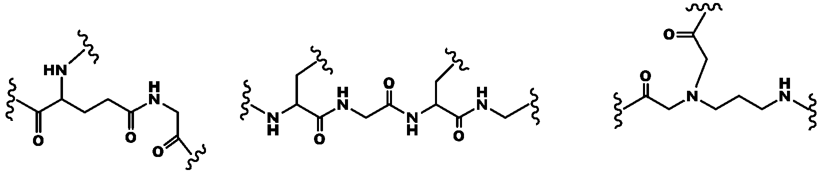

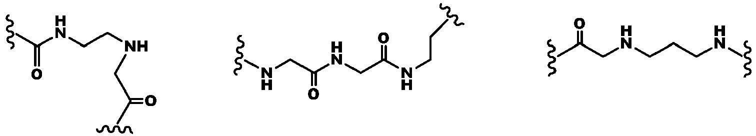

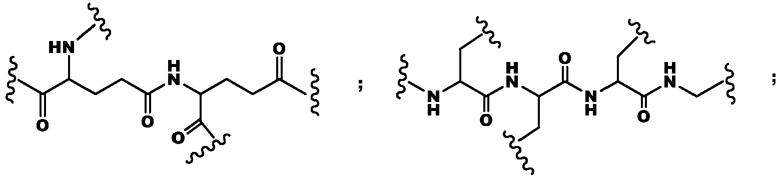

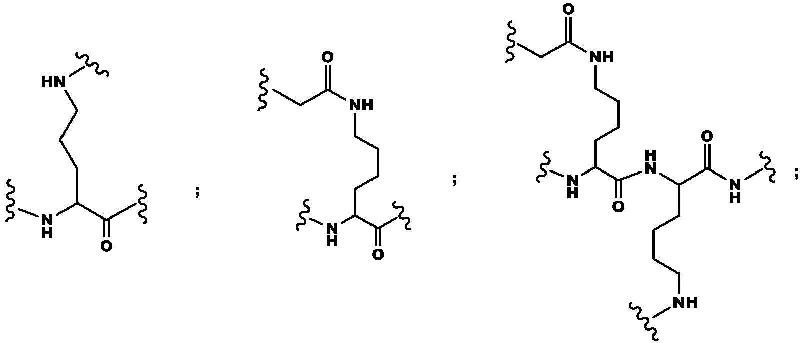

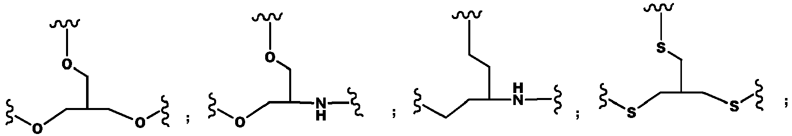

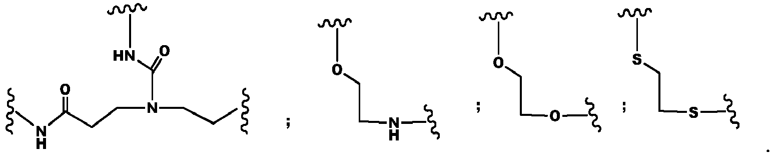

In some embodiments, the linker B is preferably one or more selected from the following structural formulae:

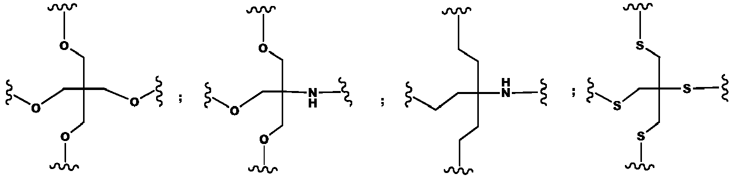

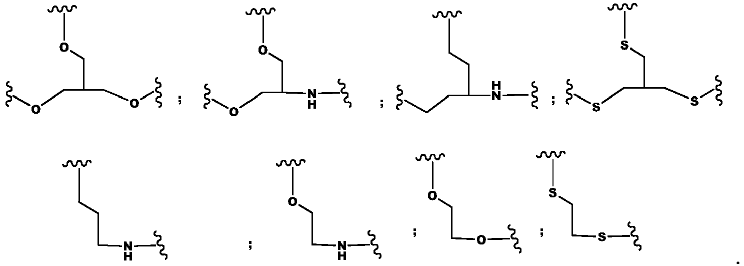

The linking chain D is a C3-C18 straight chain containing -NH-, C=O, O, S, amide group, phosphoryl, thiophosphoryl, aromatic hydrocarbonyl, C4-C10 aliphatic carbocyclyl, 5- or 6-membered heterocyclyl containing 1 to 3 nitrogens or a combination thereof, the C3-C18 straight chain may further have a side chain of methyl alcohols, methyl tert-butyl, methyl phenol, or C5-C6 alicyclyl, the linking chain D is preferably a C3-C10 straight chain containing two C=O, 6-membered aliphatic carbocyclyl or phenyl.

In some embodiments, the linking chain D is one or more selected from the following structural formulae: 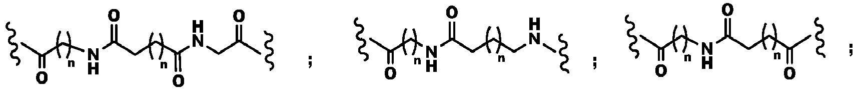 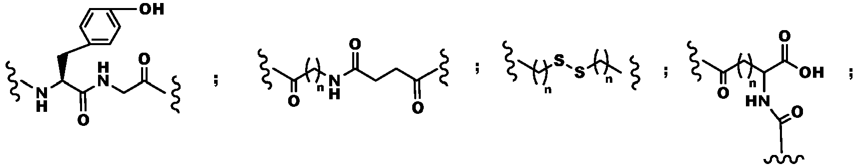 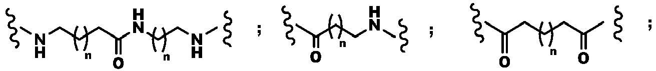 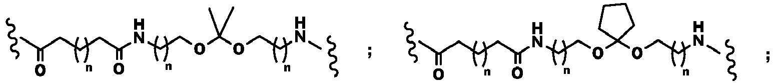 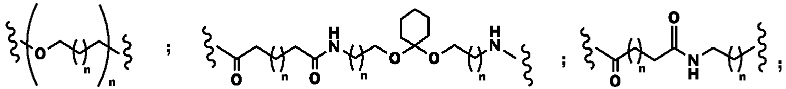 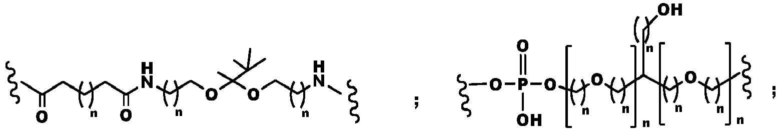 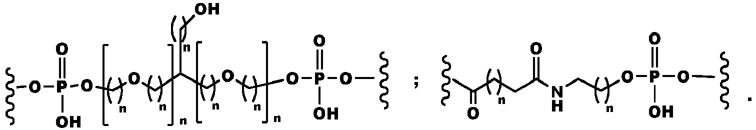 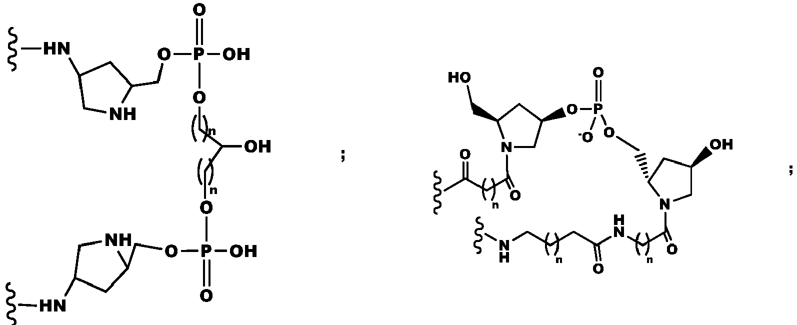 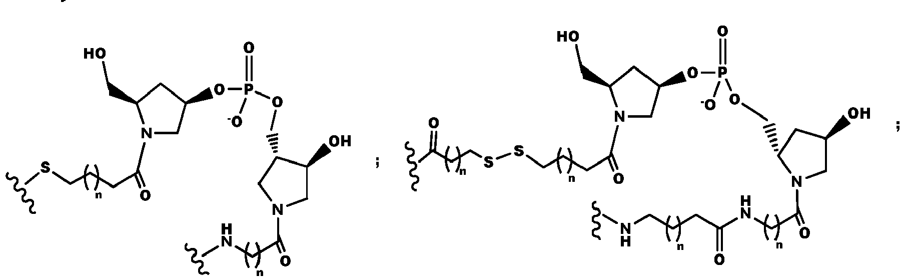 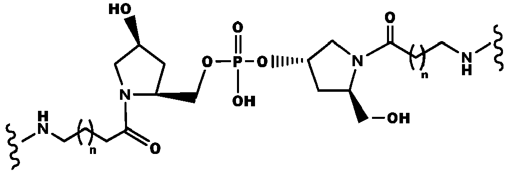 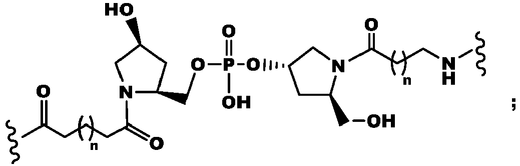 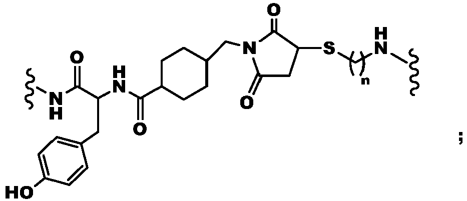 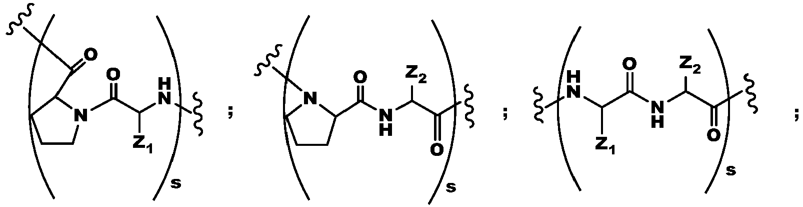 wherein, each n is a positive integer of 1 to 20, and each n is the same or different integers; s is an integer of 2 to 13; Z₁ and Z₂ are the same or different substituents, such as C3-C10 alkyl.

In some embodiments, the linking chain D is preferably one selected from the following structural formulae:

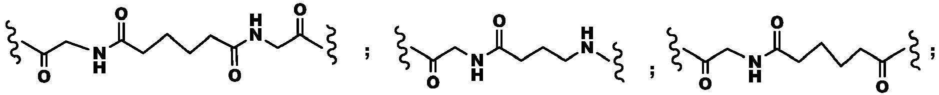

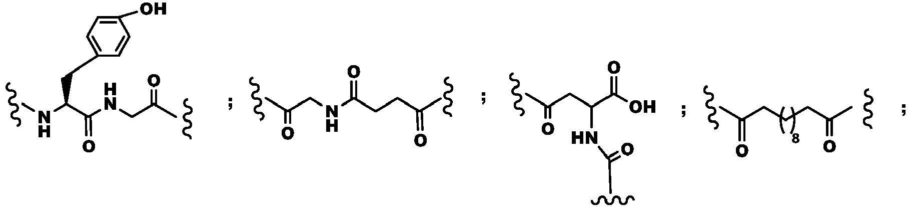

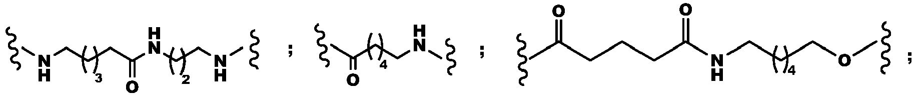

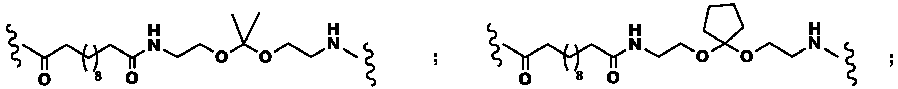

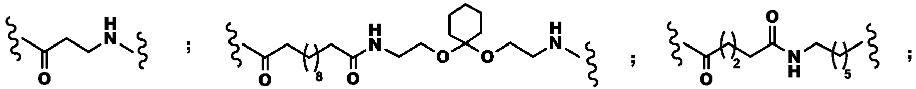

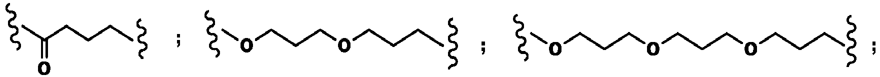

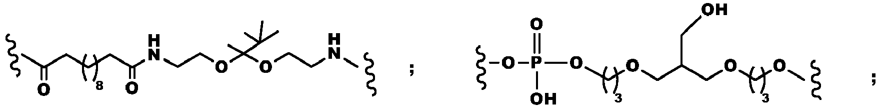

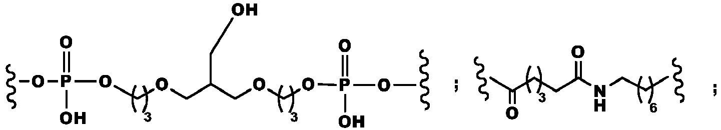

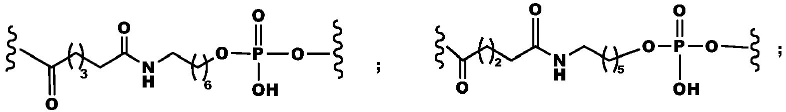

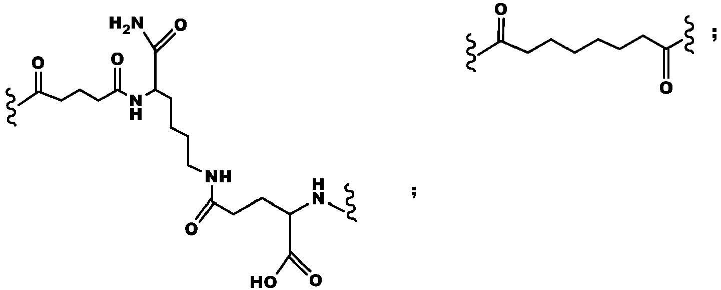

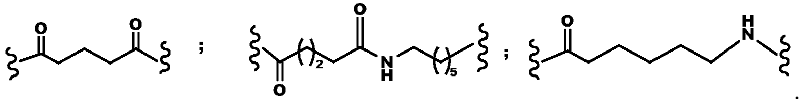

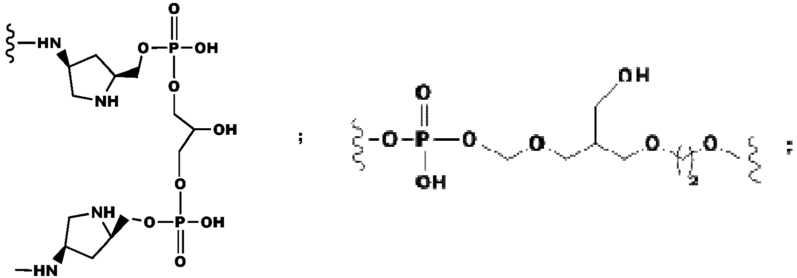

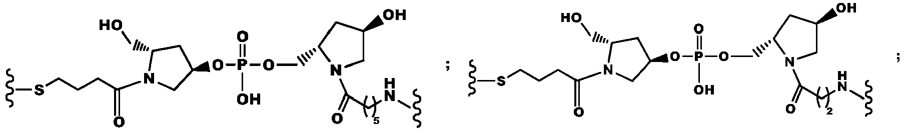

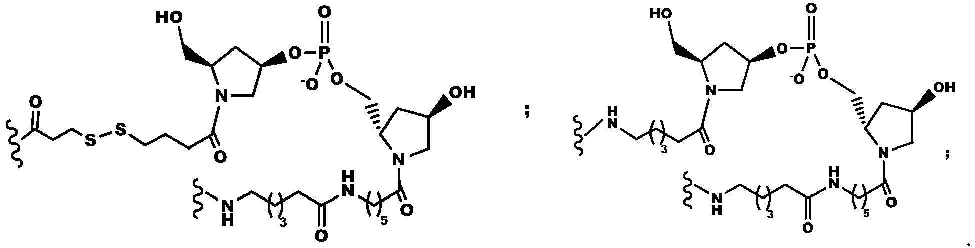

In some embodiments, the linking chain D is preferably one or more selected from the following structural formulae

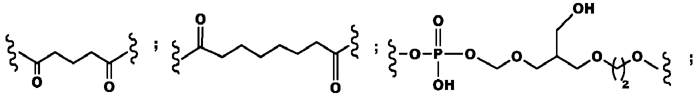

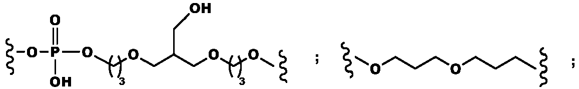

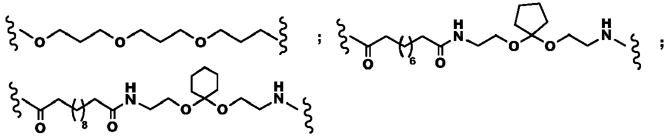

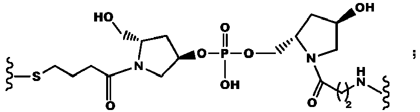

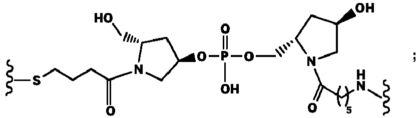

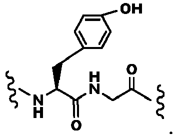

In some of the most preferred embodiments, the linking chain D is a C3-C10 straight chain containing two C=O.

In some embodiments, the (X-L)ₙ-B-D- in the structure of 5'MVIP and (X-L)ₘ₋B-D- in the structure of 3'MVIP are one or more selected from the following structural formulae:

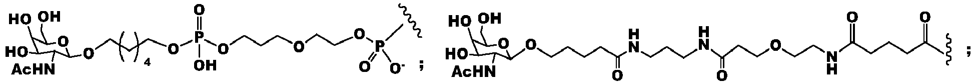

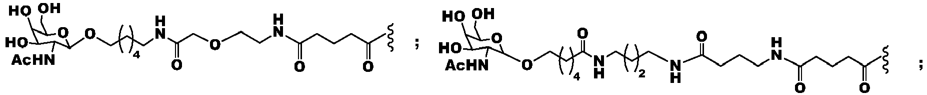

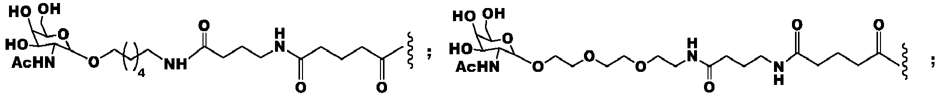

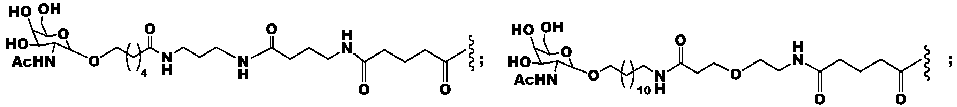

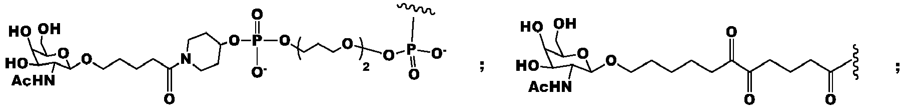

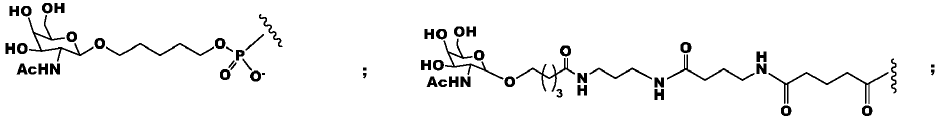

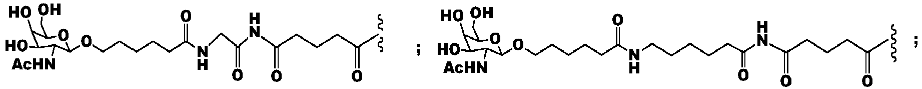

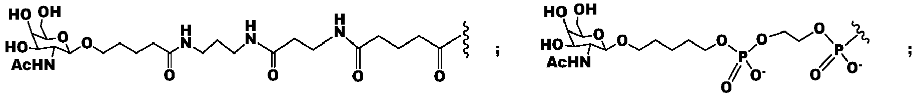

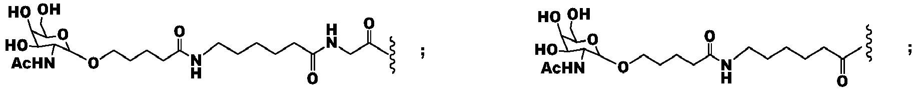

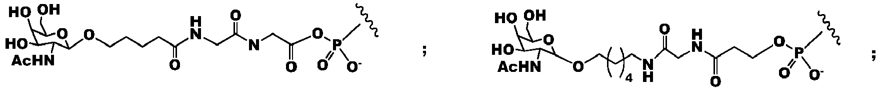

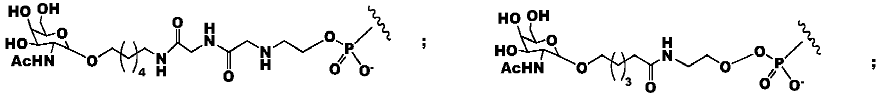

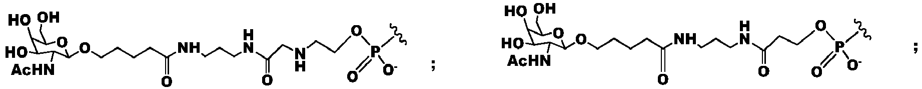

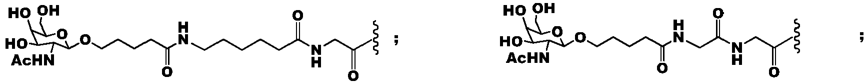

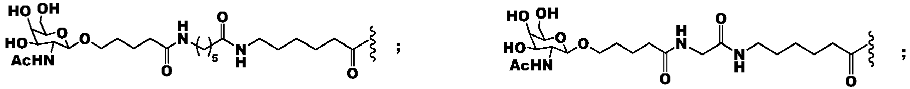

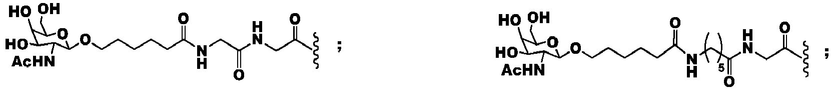

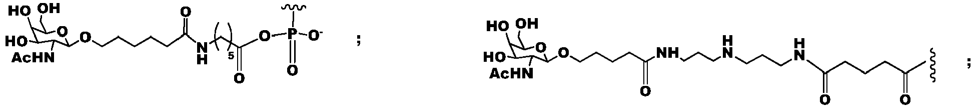

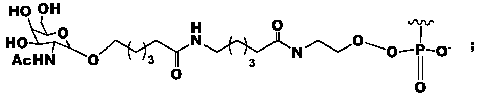

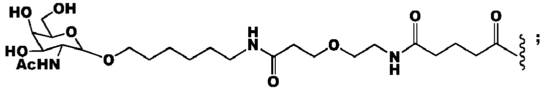

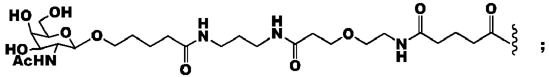

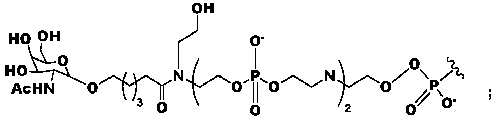

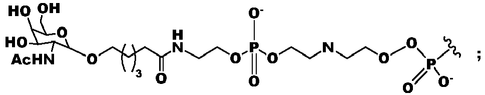

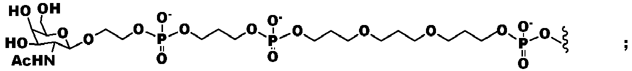

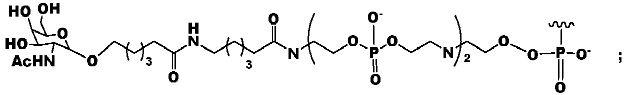

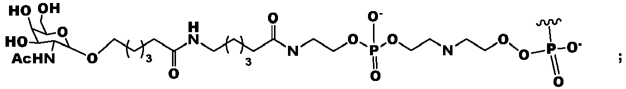

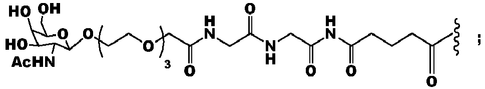

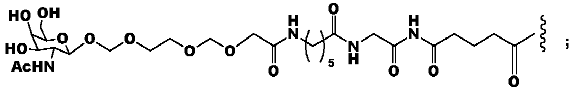

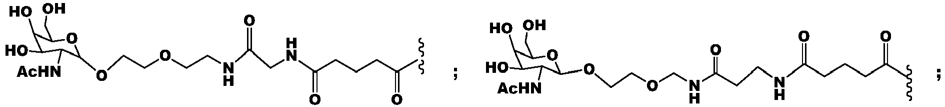

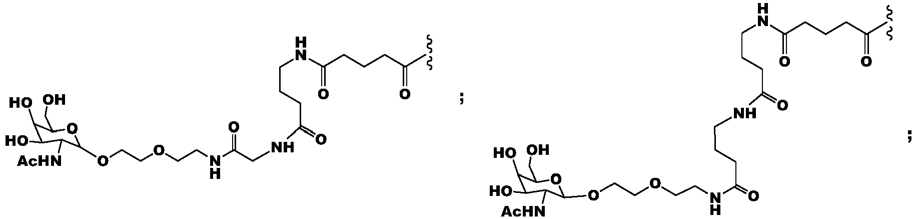

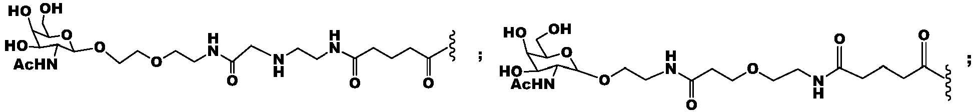

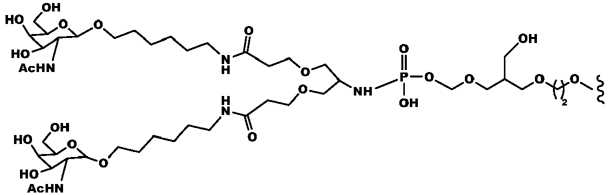

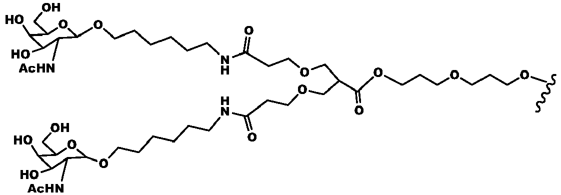

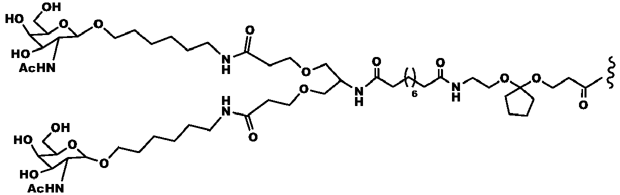

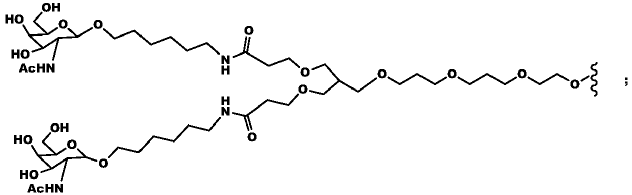

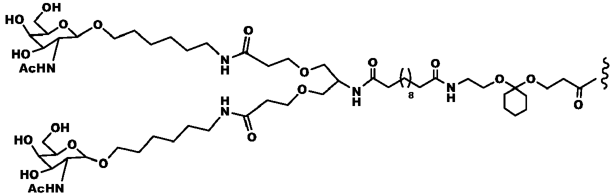

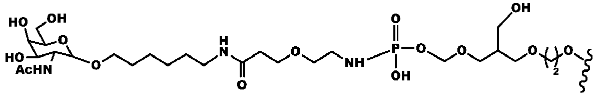

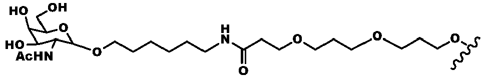

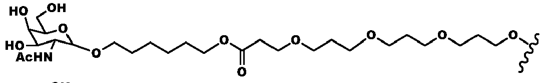

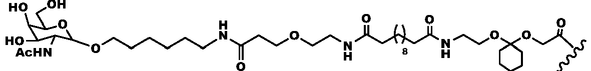

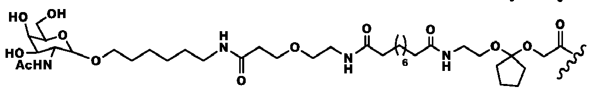

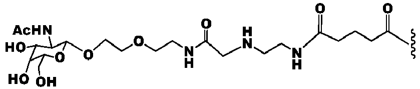

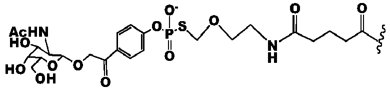

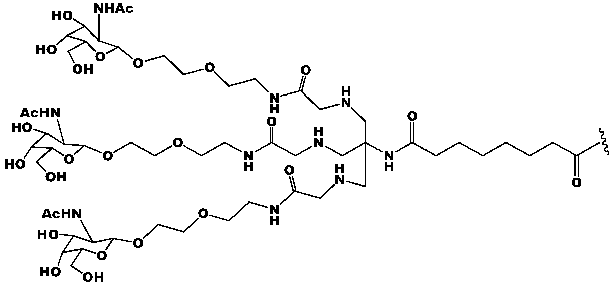

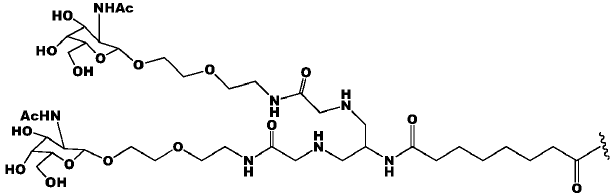

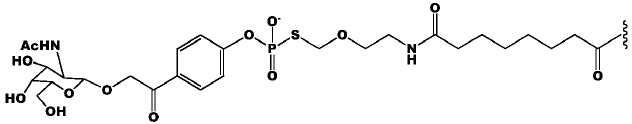

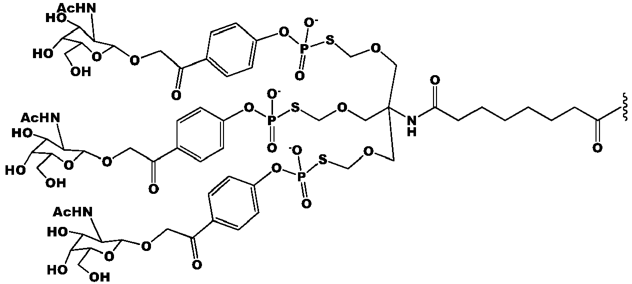

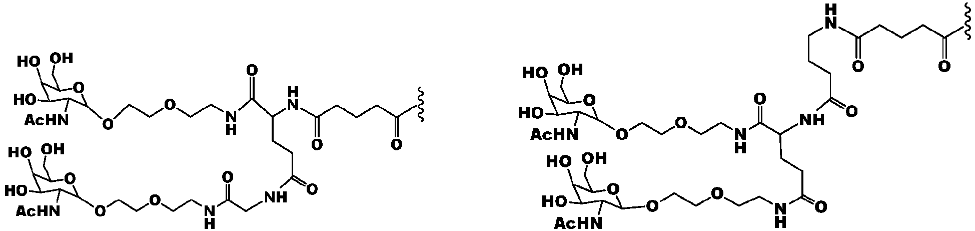

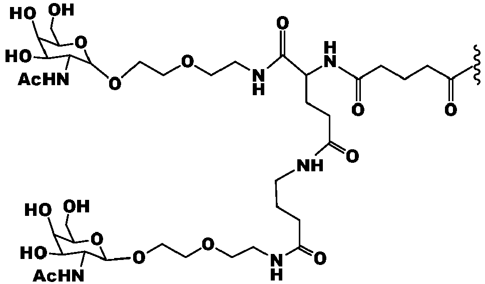

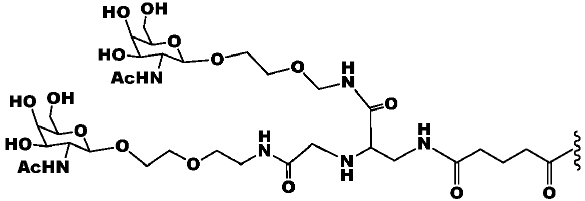

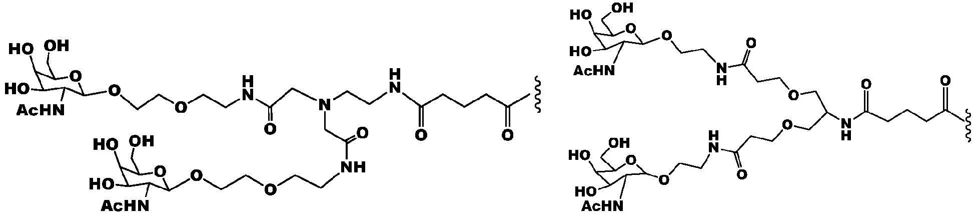

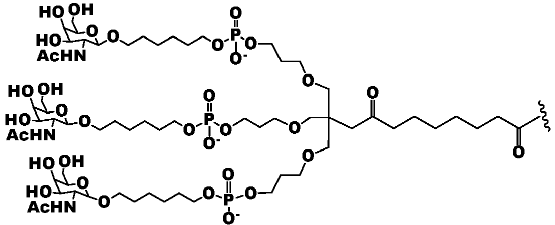

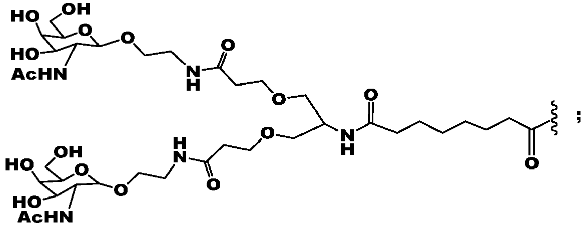

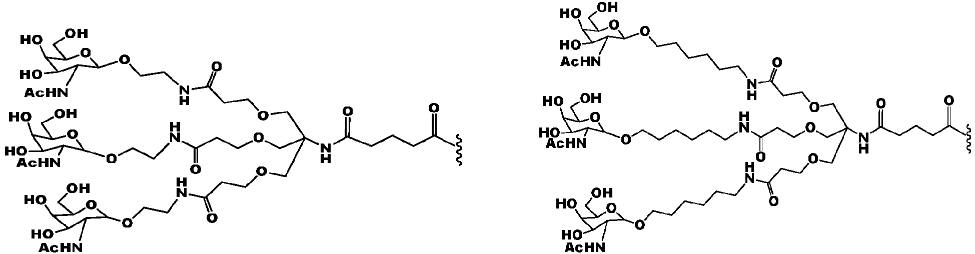

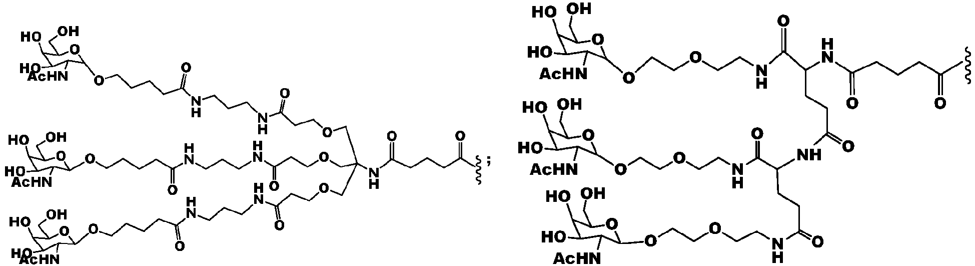

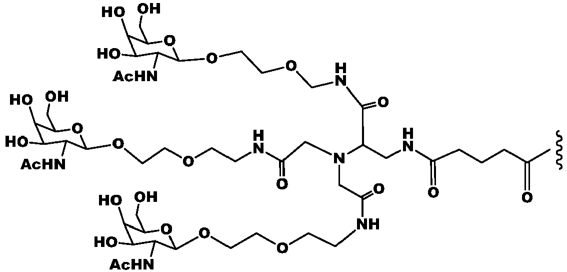

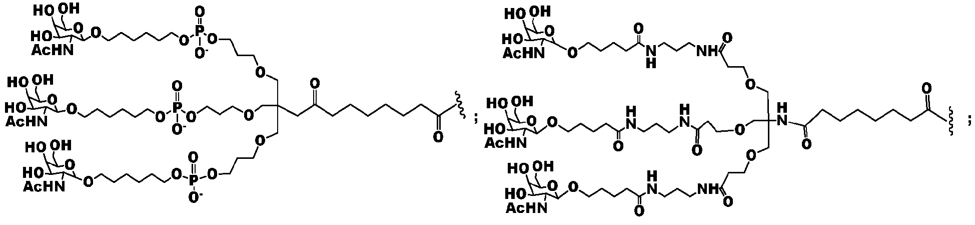

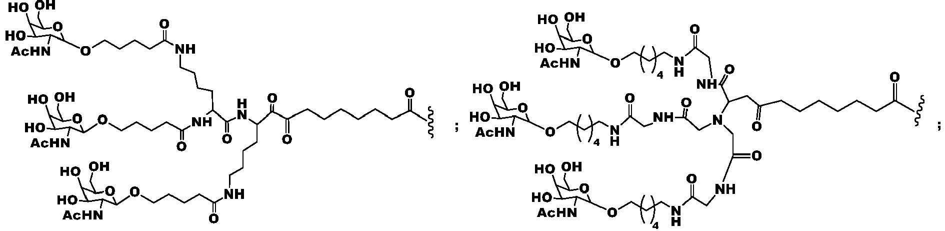

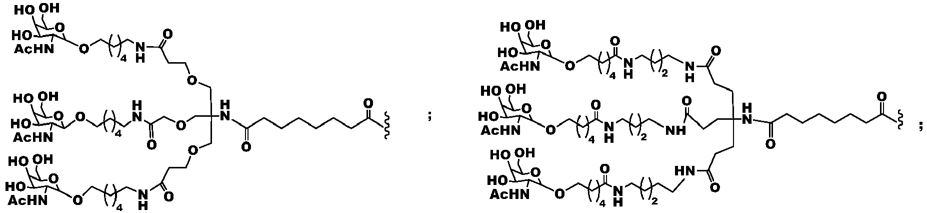

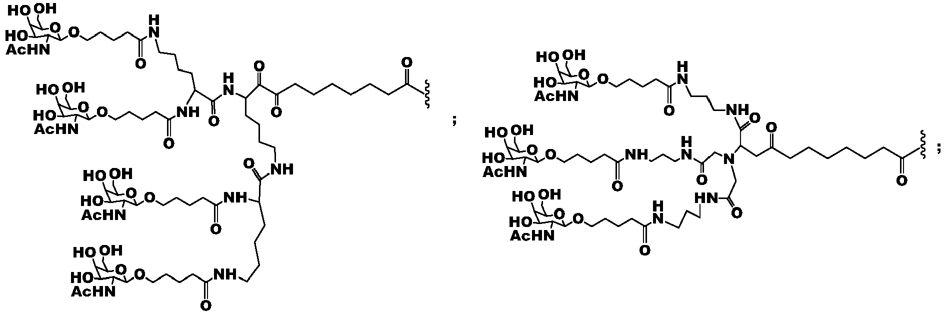

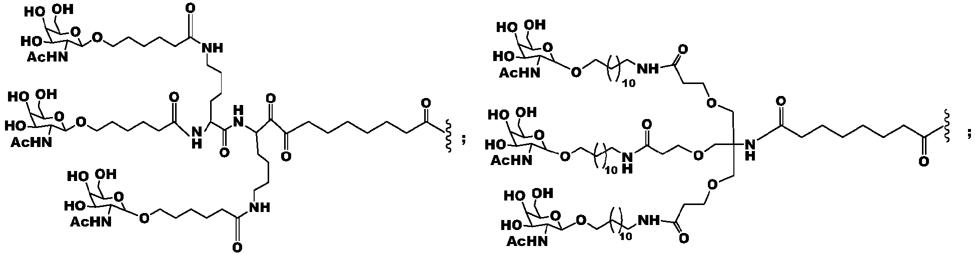

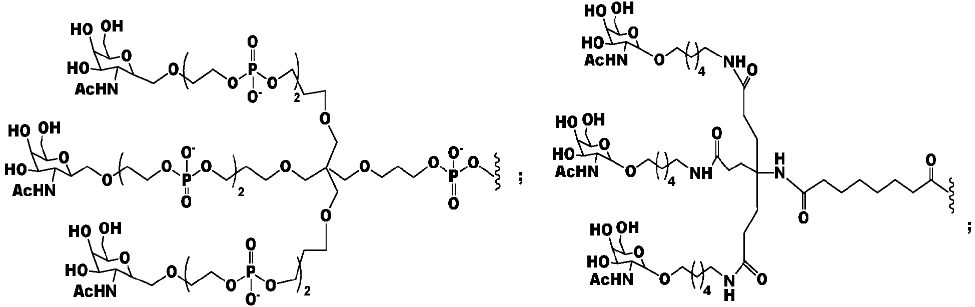

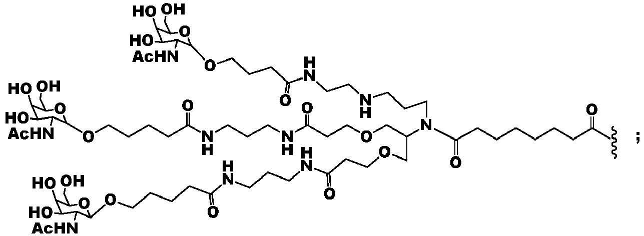

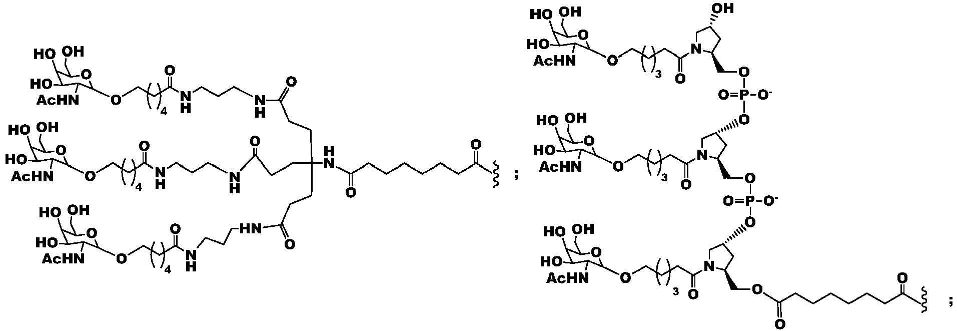

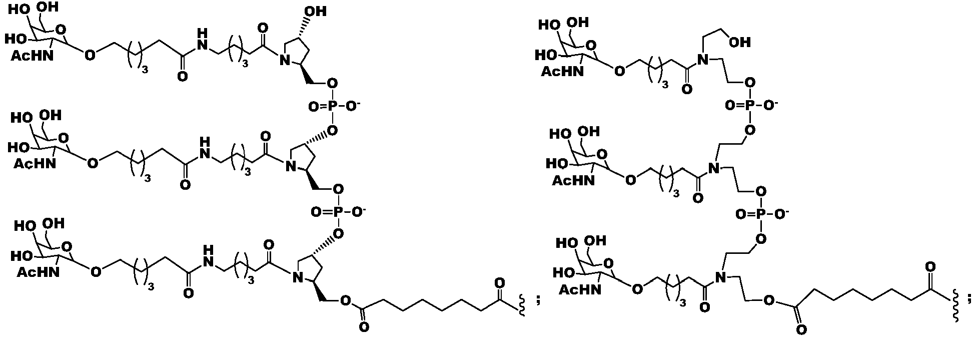

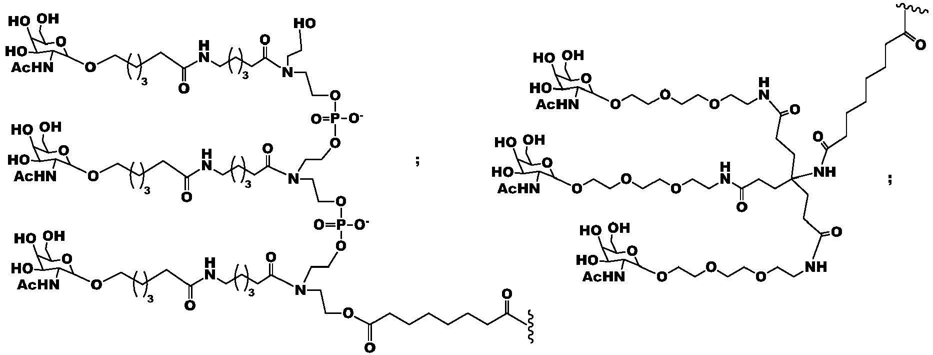

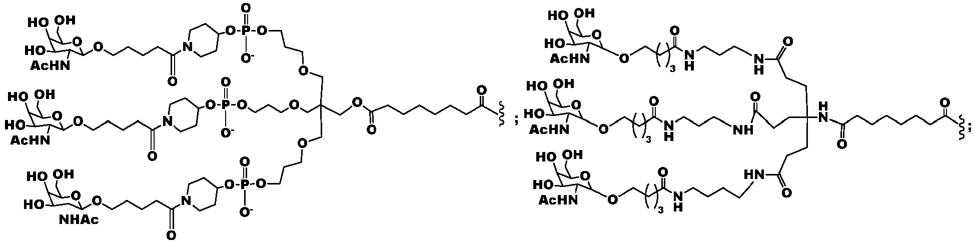

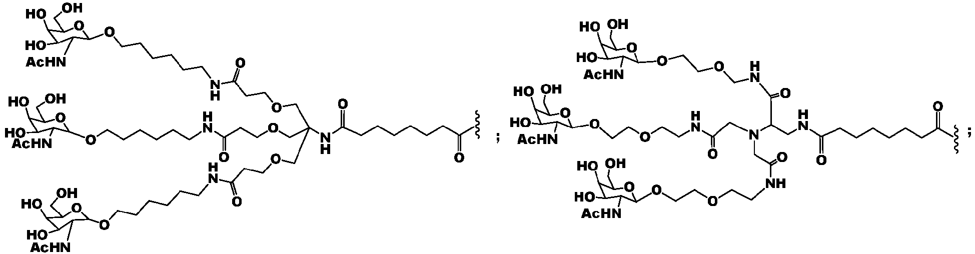

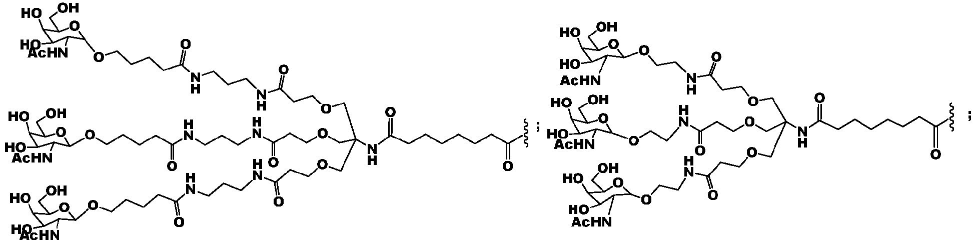

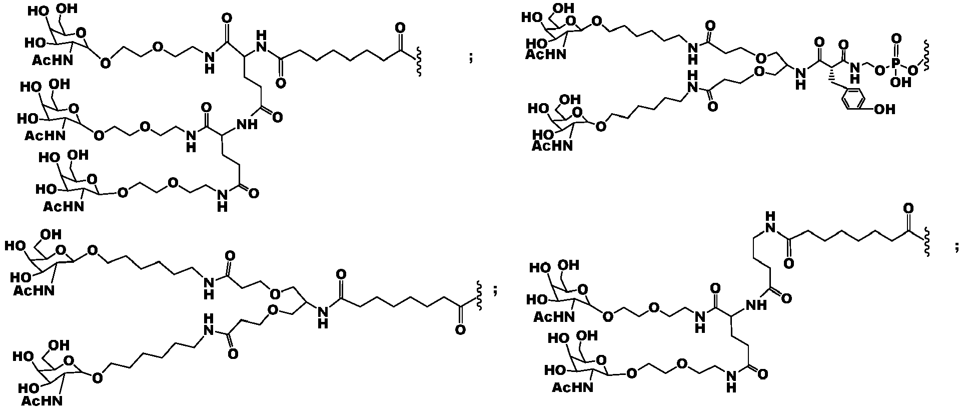

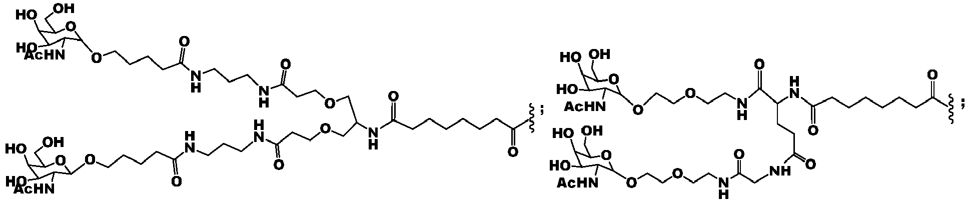

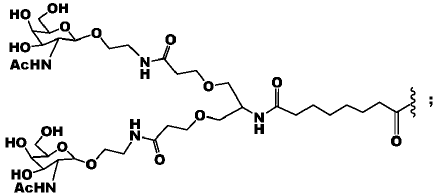

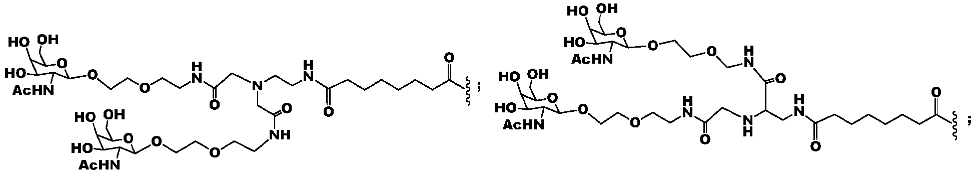

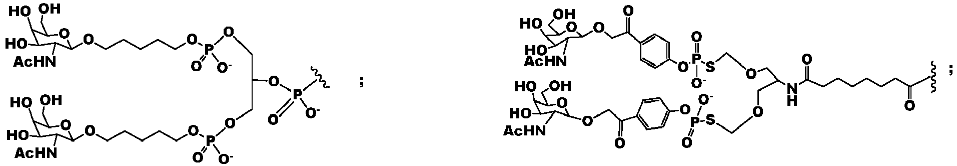

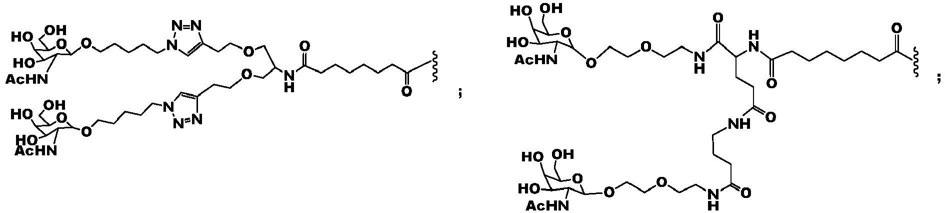

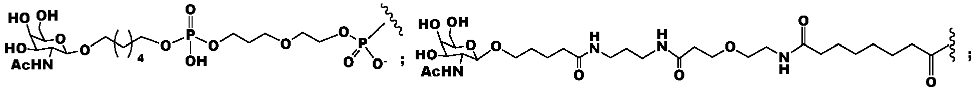

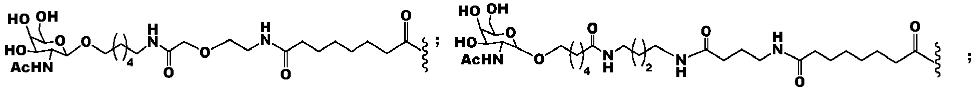

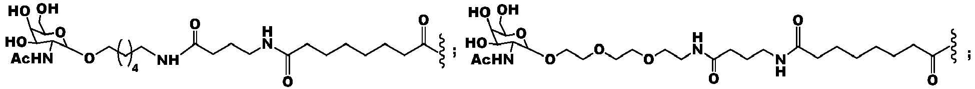

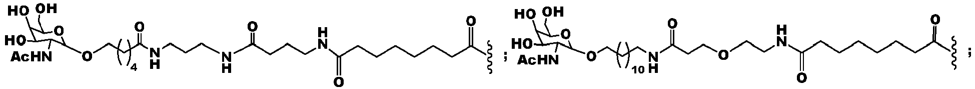

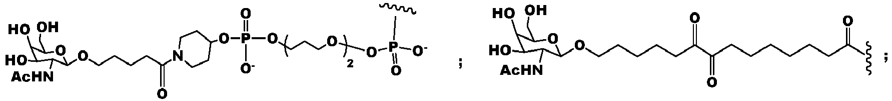

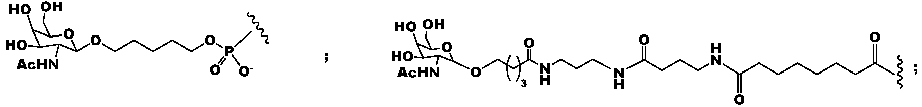

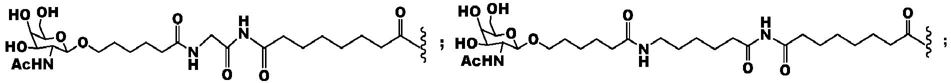

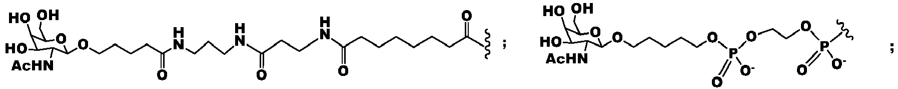

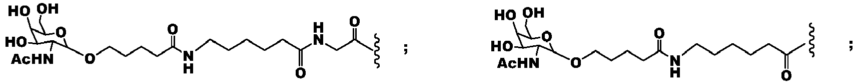

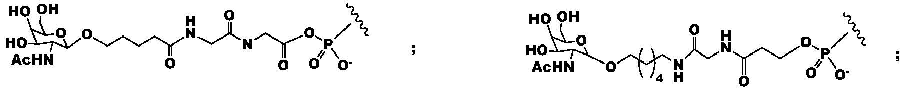

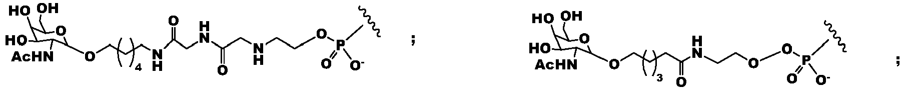

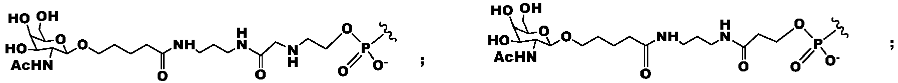

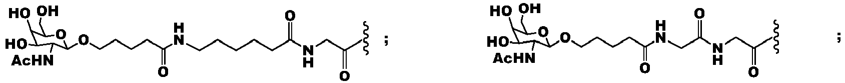

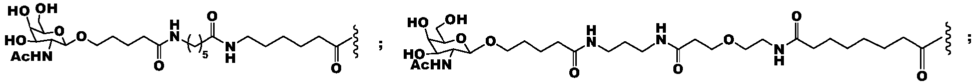

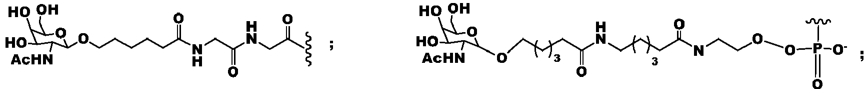

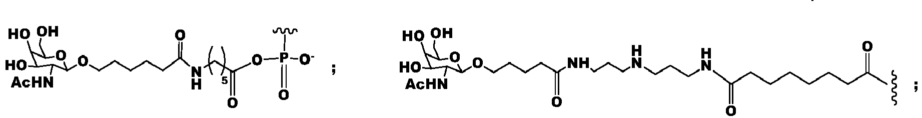

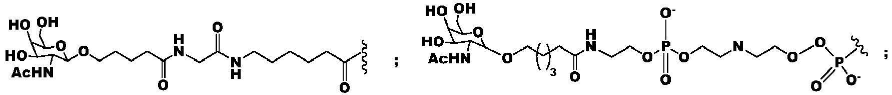

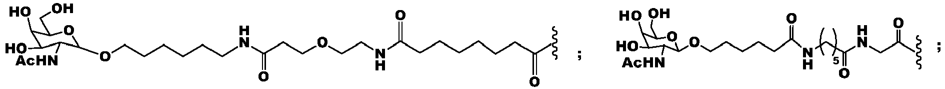

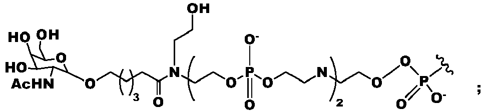

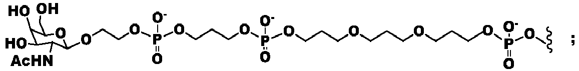

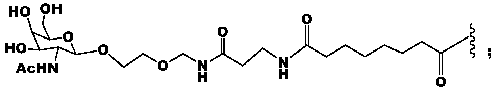

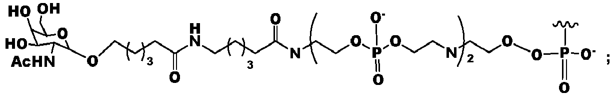

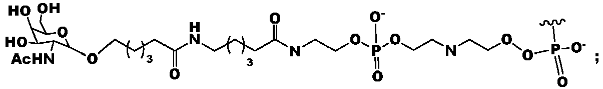

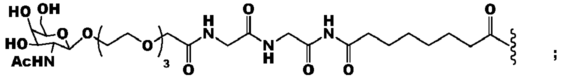

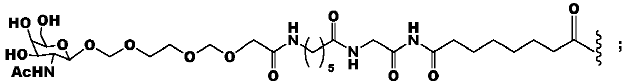

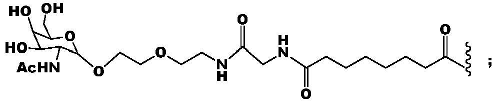

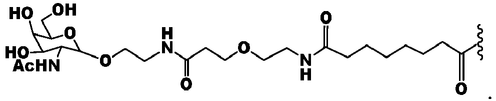

In some preferred embodiments, the (X-L)ₙ-B-D- in the structure of 5'MVIP is selected from the structural formulae as shown in Table 1.

**Table 1: (X-L)ₙ-B-D- in 5'MVIP**

| No. | Code | Formula |
|---|---|---|
| 1 | 5'YICdd-01 | 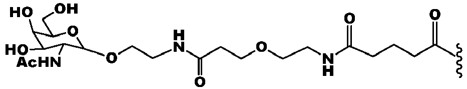 |
| 2 | 5'YICd-01 | 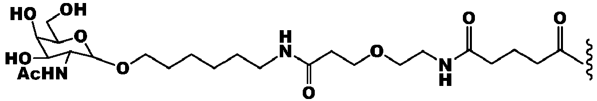 |
| 3 | 5'YICc-01 | 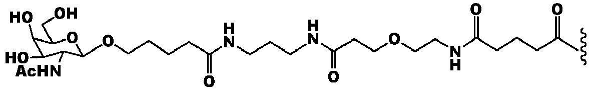 |
| 4 | 5'YICa-01 | 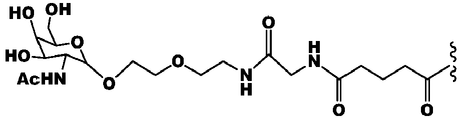 |
| 5 | 5'YICa-02 | 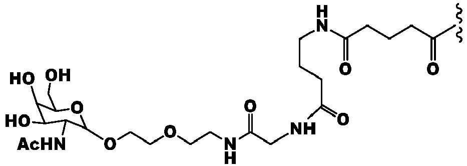 |
| 6 | 5'YICa-03 | 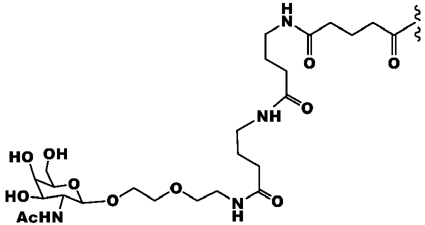 |
| 7 | 5'YICa-04 | 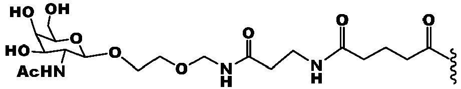 |
| 8 | 5'YICa-05 | 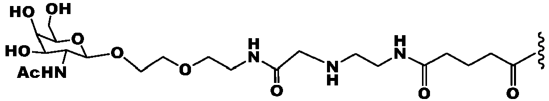 |
| 9 | 5'ERCa-01 | 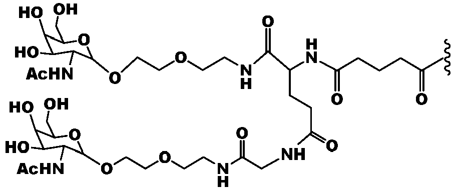 |
| 10 | 5'ERCa-02 | 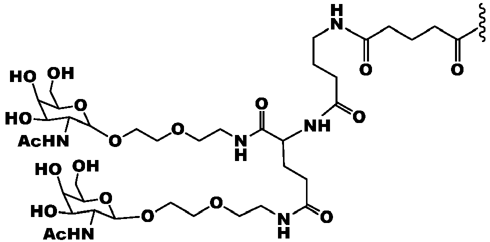 |
| 11 | 5'ERCa-03 | 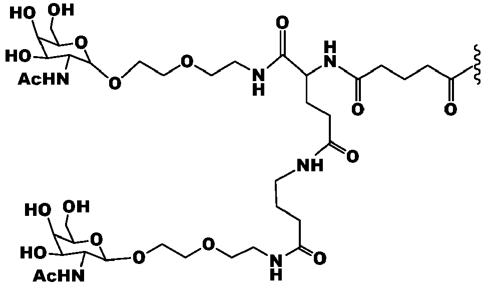 |
| 12 | 5'ERCa-04 | 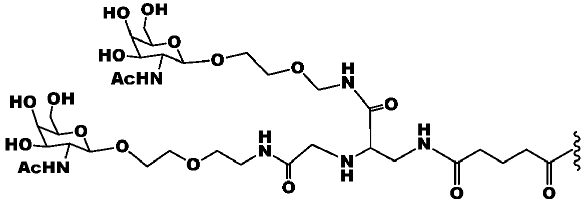 |
| 13 | 5'ERCa-05 | 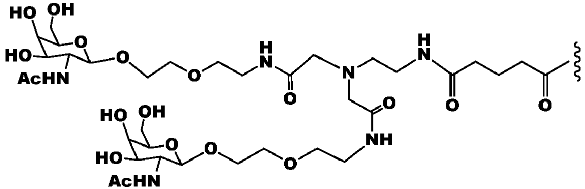 |
| 14 | 5'ERCdd-01 | 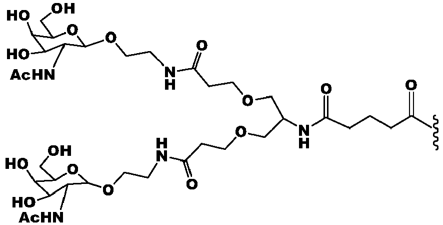 |
| 15 | 5'ERCd-01 | 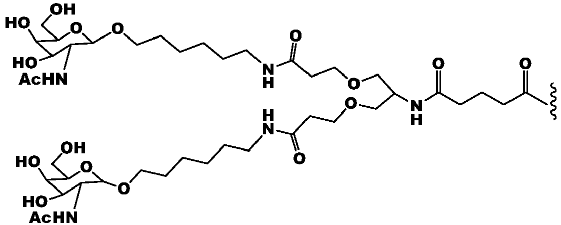 |
| 16 | 5'ERCc-01 | 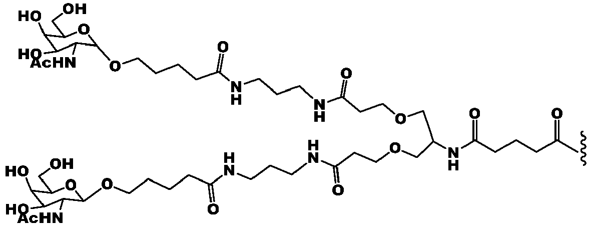 |
| 17 | 5'SANCdd-01 | 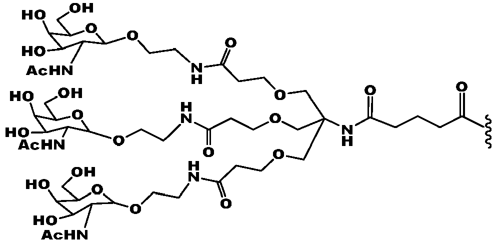 |
| 18 | 5'SANCd-01 | 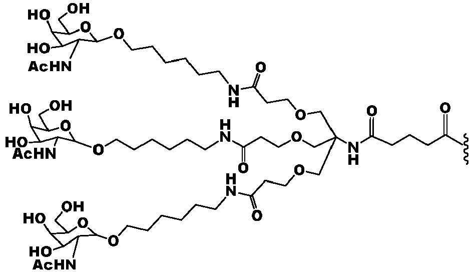 |
| 19 | 5'SANCc-01 | 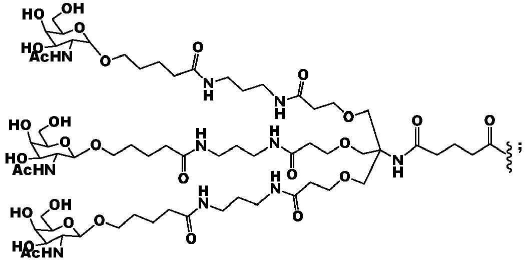 |
| 20 | 5'SANCa-01 | 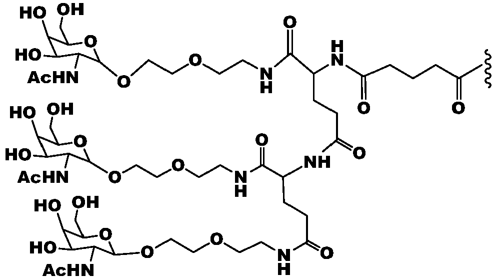 |
| 21 | 5'SANCa-02 | 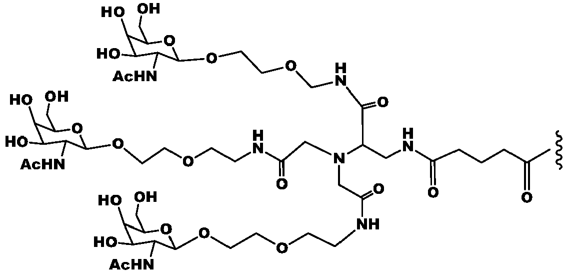 |
| 22 | 5'SANCa-03 | 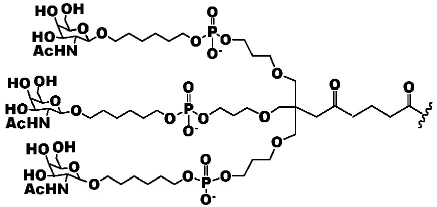 |

In some embodiments, 5'MVIP may be absent, and m may be an integer of 2 to 4.

In some preferred embodiments, the (X-L)ₘ**-**B-D**-** in the structure of 3'MVIP is selected from the structural formulae as shown in Table 2:

**Table 2: (X-L)ₘ-B-D- in 3'MVIP**

| No. | Code | Formula |
|---|---|---|
| 1 | 3'SANCdd-01 | 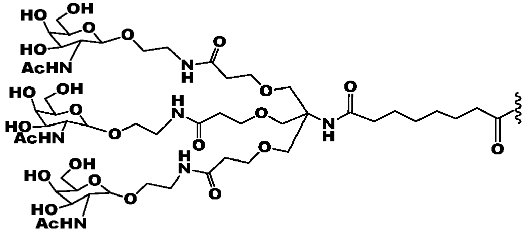 |
| 2 | 3'SANCd-01 | 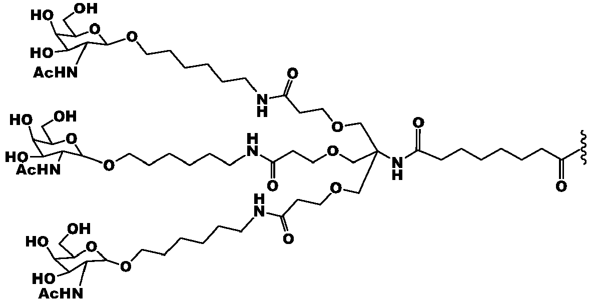 |
| 3 | 3'SANCc-01 | 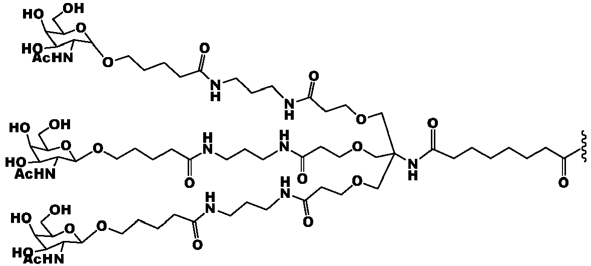 |
| 4 | 3'SANCa-01 | 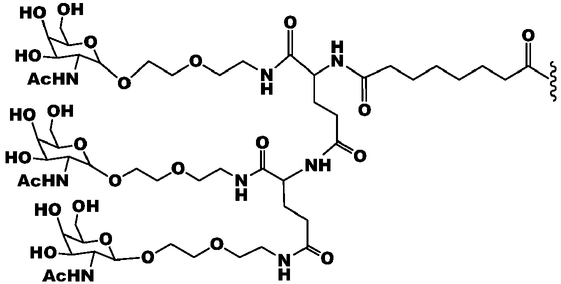 |
| 5 | 3'SANCa-02 | 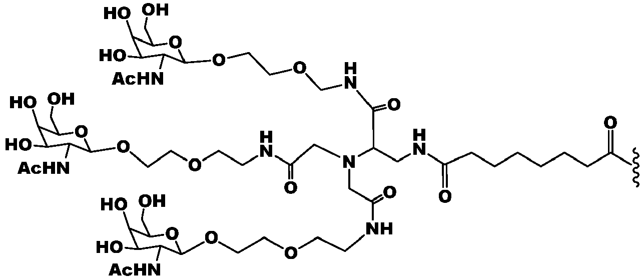 |
| 6 | 3'SANCa-03 | 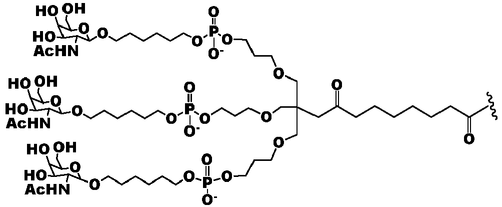 |
| 7 | 3'ERCdd-01 | 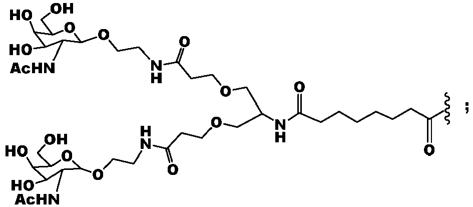 |
| 8 | 3'ERCd-01 | 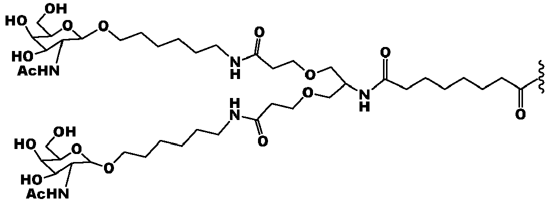 |
| 9 | 3'ERCc-01 | 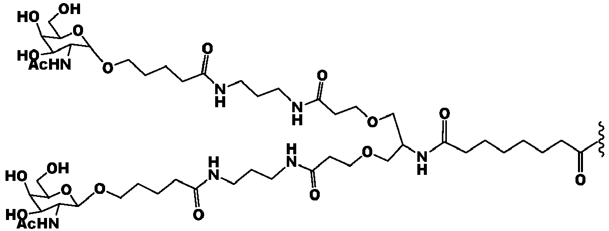 |
| 10 | 3'ERCa-01 | 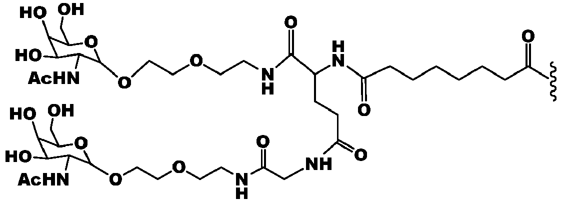 |
| 11 | 3'ERCa-02 | 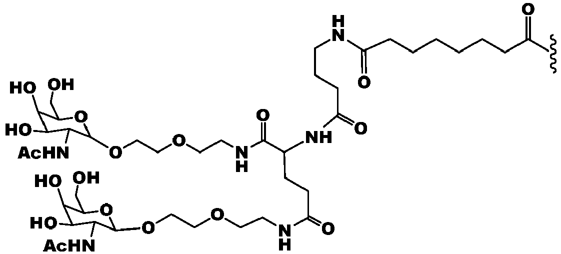 |
| 12 | 3'ERCa-03 | 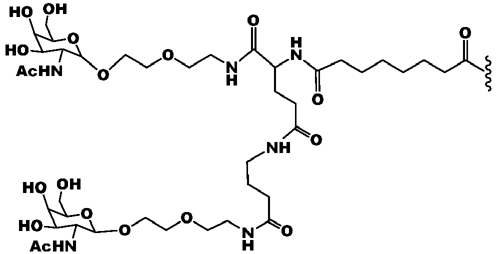 |
| 13 | 3'ERCa-04 | 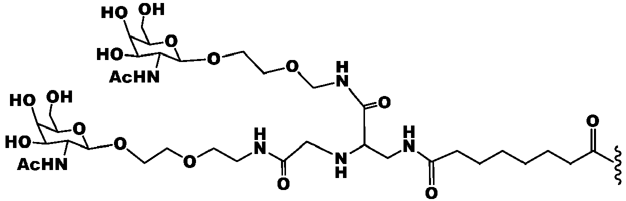 |
| 14 | 3'ERCa-05 | 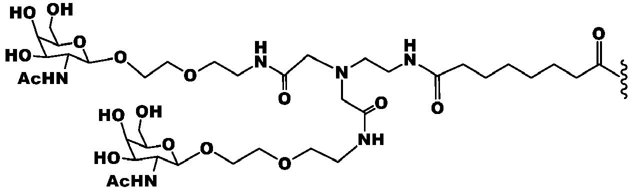 |
| 15 | 3'YICa-01 | 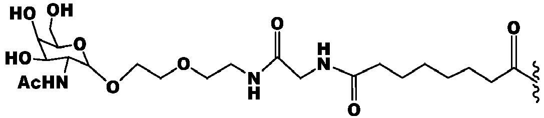 |
| 16 | 3'YICa-02 | 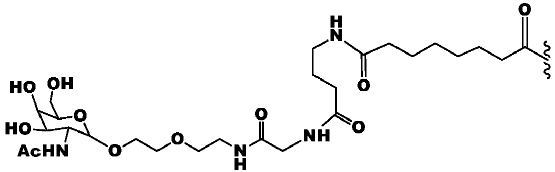 |
| 17 | 3'YICa-03 | 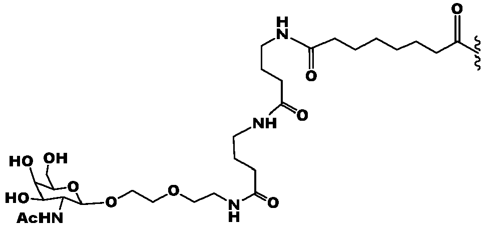 |
| 18 | 3'YICa-04 | 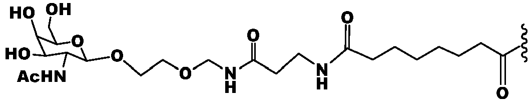 |
| 19 | 3'YICa-05 | 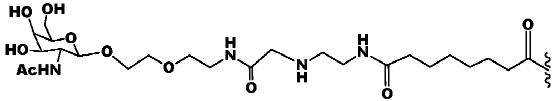 |
| 20 | 3'YICdd-01 | 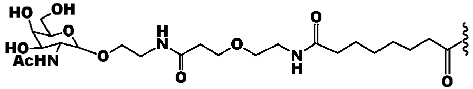 |
| 21 | 3'YICd-01 | 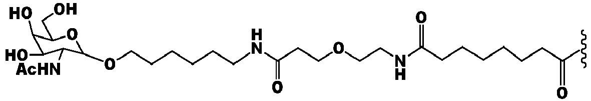 |
| 22 | 3'YICc-01 | 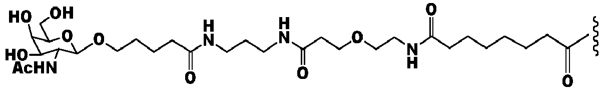 |

In the RNA inhibitor provided by the present invention, the 5'MVIP further comprises a transition point R₁ connected or coupled to the 5' end of the sense strand or antisense strand, and the transition point R₁ has -NH-, sulfur atom or oxygen atom, and generally at least one -NH-, sulfur atom or oxygen atom is in the structure. R₁ is linked to the linking chain D of 5'MVIP and the 5' end of the sense strand or antisense strand through -NH-, sulfur atom or oxygen atom in its structure, thereby introducing the liver targeting specific ligand X. The transition point R₁ may be a straight chain; a straight chain with an amide group, carboxyl or alkyl branch, or various cyclic structures, the cyclic structure may be, for example, saturated or unsaturated aliphatic carbocyclyl, or 5- or 6-membered heterocyclyl or aromatic hydrocarbonyl containing sulfur, oxygen or nitrogen atom, etc.

In some embodiments, R₁ is -B₁(CH₂)ₓCH₂B₂-, wherein x is an integer of 3 to 10, preferably 4 to 6, and the groups B₁ and B₂ may be -NH-, sulfur atom or oxygen atom, respectively.

In some embodiments, R₁ is -B₁(CH₂)ₓCH(B₃CH₃)B₂-, wherein x is an integer of 3 to 10, and the groups B₁ and B₂ may be -NH-, sulfur atom or oxygen atom, respectively, and the group B₃ is a functional group containing nitrogen, sulfur, oxygen, carboxyl or alkyl such as methyl.

In some preferred embodiments, R₁ is -NH(CH₂)ₓCH₂O-, wherein x is an integer of 3 to 10, preferably 4 to 6, and may be incorporated by the following two phosphoramidite monomers:
i. One of the oxygen or sulfur atoms is used for synthesis of the R₁ phosphoramidite monomer, which is connected to the 5' end of a single strand of the RNA inhibitor by solid-phase synthesis. The -NH-, sulfur atom or oxygen atom in the structure is used to connect with the linking chain D in the 5'MVIP, thereby introducing the liver targeting specific ligand X at the 5' end of the RNA inhibitor. An exemplary structure of the monomer introduced into the 5' end of the RNA inhibitor is as follows:

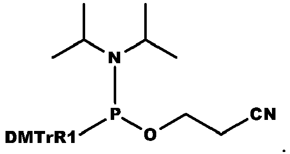

In some embodiments, the following structure is preferred:

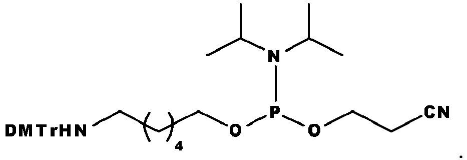

ii. -NH-, sulfur atom or oxygen atom in the structure of R₁ is first connected to the linking chain D, and the other -NH-, sulfur atom or oxygen atom is used to form an ester with phosphoramidite in the synthesis of the phosphoramidite monomer of 5'MVIP. An Example of the structure of the 5'MVIP phosphoramidite monomer of the sense strand or antisense strand is as follows:

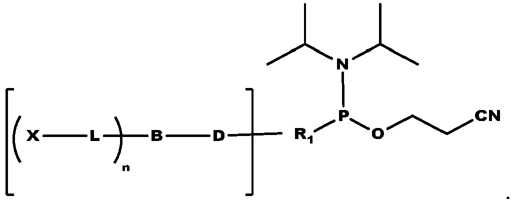

In some embodiments, R₁ is a heterocyclic or carbocyclic moiety containing nitrogen, sulfur or oxygen atom:

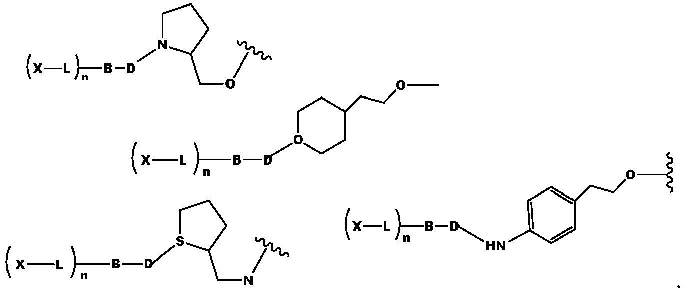

In some preferred embodiments, the 5'MVIP phosphoramidite monomer in the sense strand or antisense strand preferably has the following structure:

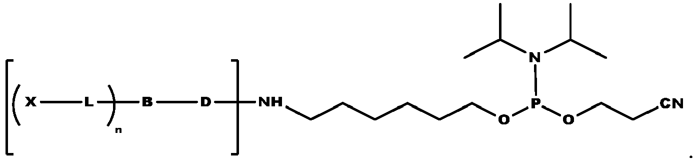

When n in the general formula is 1 to 4, the linker B moiety in the above monomers is branched 1 to 4 times respectively, to obtain the corresponding monomeric compound. By means of the monomeric compound, the liver targeting specific ligand X is introduced at the 5' end of the sense strand or antisense strand by solid-phase synthesis.

In some preferred embodiments, the transition point R₁ is preferably -NH(CH₂)ₓCH₂O-, wherein x may be an integer of 3 to 10, preferably 4 to 6, and the 5'MVIP phosphoramidite monomer has a structure selected from the following structures:

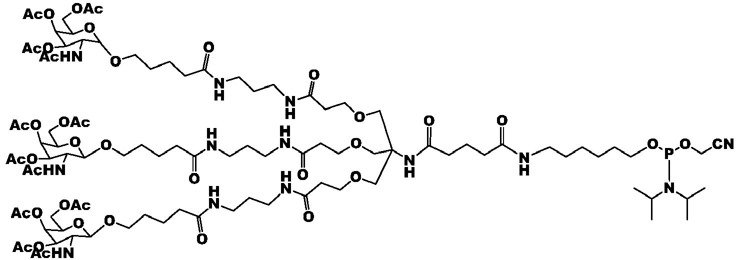

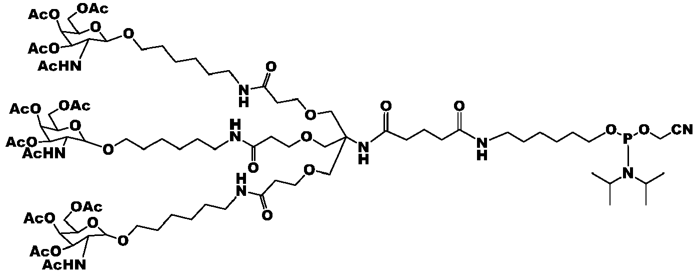

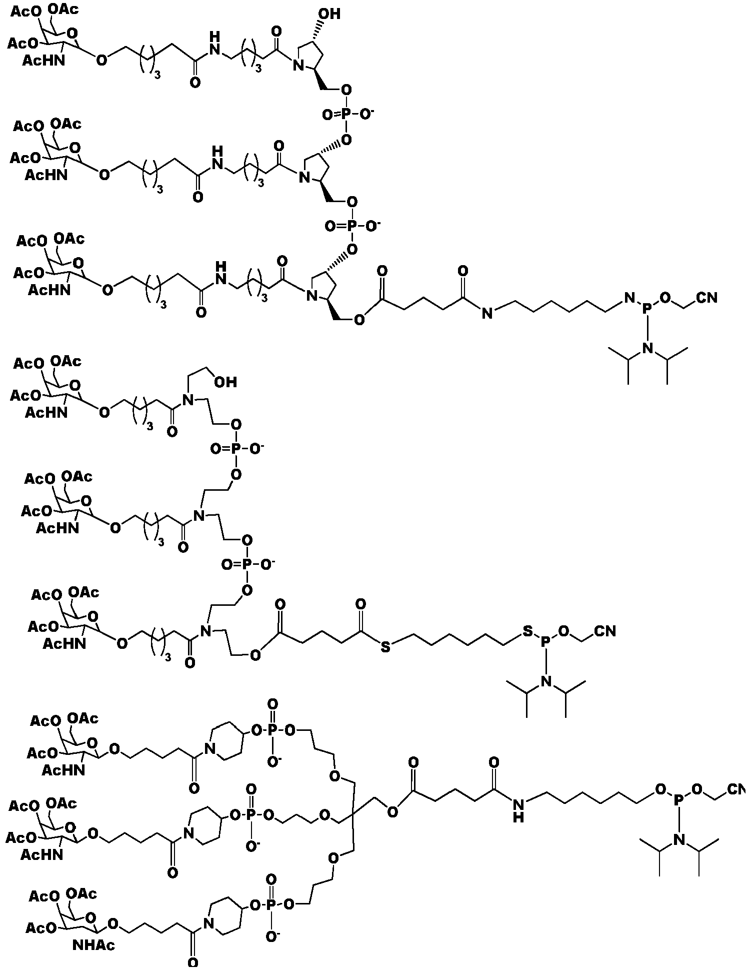

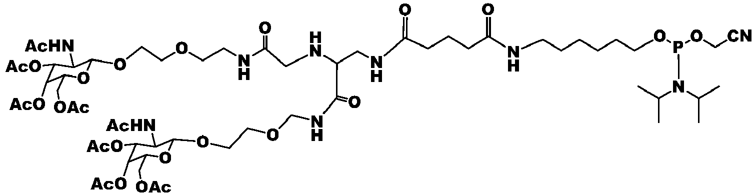

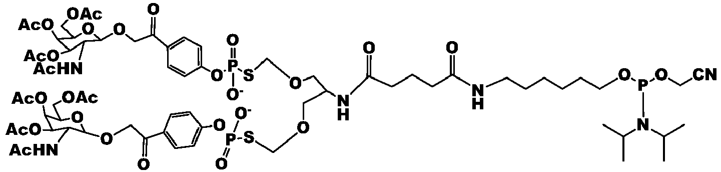

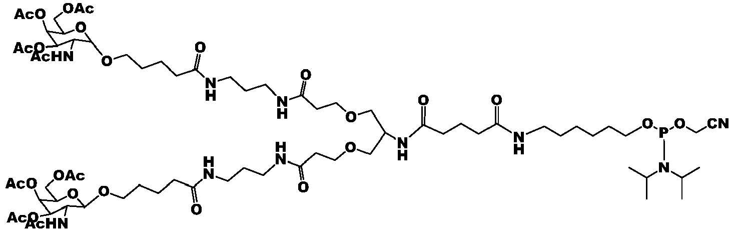

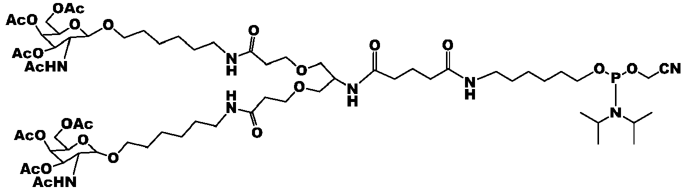

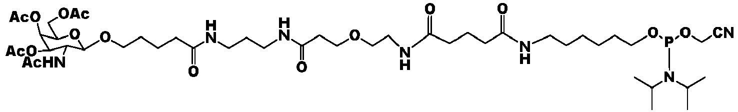

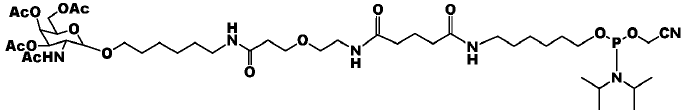

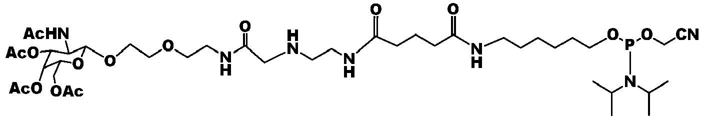

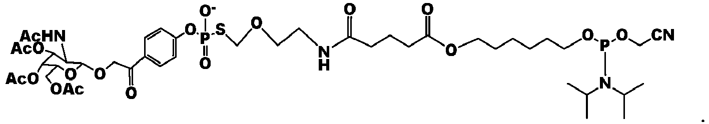

In the RNA inhibitor provided by the present invention, the 3'MVIP further comprises a transition point R₂ connected or coupled to the 3' end of the sense strand or antisense strand, and the transition point R₂ has -NH-, sulfur atom or oxygen atom, and generally at least one -NH-, sulfur atom or oxygen atom is in the structure. R₂ is linked to the linking chain D of 3'MVIP and the 3' end of the sense strand or antisense strand through -NH-, sulfur atom or oxygen atom in its structure, thereby introducing the liver targeting specific ligand X. The transition point R₂ may be a straight chain; a straight chain with an amide group, carboxyl or alkyl branch, or various cyclic structures, the cyclic structure may be, for example, saturated or unsaturated aliphatic carbocyclyl, or 5- or 6-membered heterocyclyl or aromatic hydrocarbonyl containing sulfur, oxygen or nitrogen atom, etc.

In some embodiments, the transition point R₂ containing a heterocyclic structure such as piperidinyl, pyrrolyl, thiazolyl or benzene ring has a structure as follows:

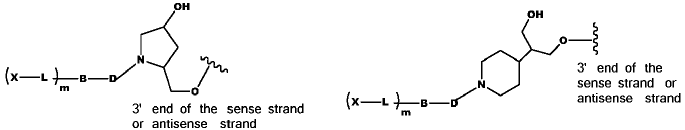

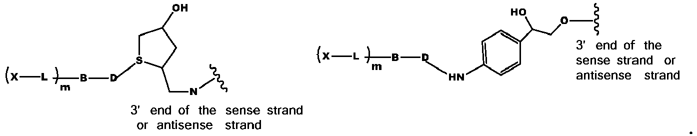

R₂ described in the present invention forms an ester or amide by reacting succinic anhydride with the - NH-, sulfur atom or oxygen atom in the structure of R₂, and also couples with the -NH- in a blank Solid Support to form a 3'MVIP solid support, followed by introducing 3'MVIP to the 3' end of the sense strand or antisense strand through a phosphoramidite solid-phase synthesis.

In some embodiments, the heterocyclic ring in the structure of R₂ is pyrrole ring or piperidine ring, which is connected to the linking chain D of the 3'MVIP through the nitrogen heteroatom in the ring, and the exemplary structure of the 3'MVIP solid support is as follows:

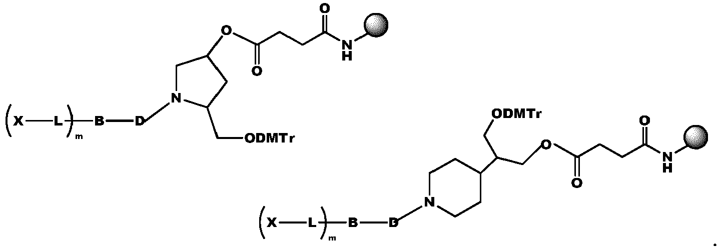

When m in the general formula is 1 to 4, the linker B moiety in the above monomers is branched 1 to 4 times respectively, to obtain a corresponding solid support.

In some embodiments, R₂ is -B₄(CH₂)ₓ₁CH(OH)(CH₂)ₓ₂CH₂B₅-, wherein x1 is an integer of 1 to 4, and x2 is an integer of 0 to 4, and B₄ and B₅ is -NH-, sulfur atom or oxygen atom, respectively.

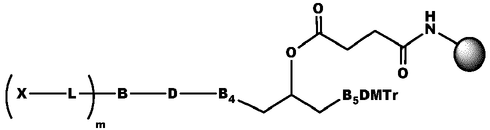

When m in the general formula is 1 to 4, the linker B moiety in the above monomers is branched 1 to 4 times respectively, to obtain a corresponding Solid Support.

In some preferred embodiments, R₂ is -NHCH₂CH(OH)CH₂O. The introduced 3'MVIP solid support has an exemplary structure as follows:

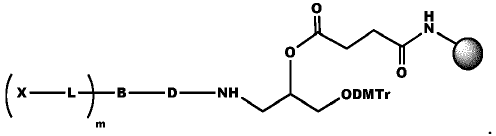

When m in the general formula is 1 to 4, the linker B moiety in the above monomers is branched 1 to 4 times respectively, to obtain a corresponding Solid Support.

In some embodiments, the 3'MVIP solid support has a structure as follows:

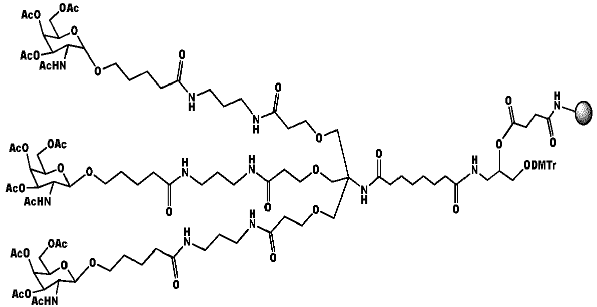

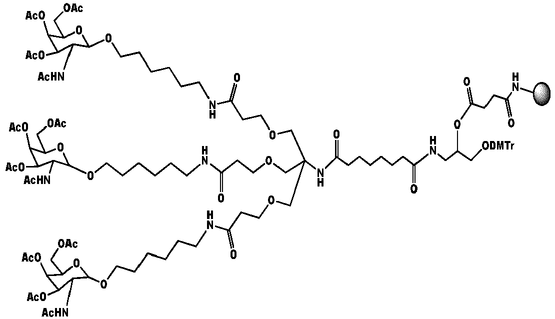

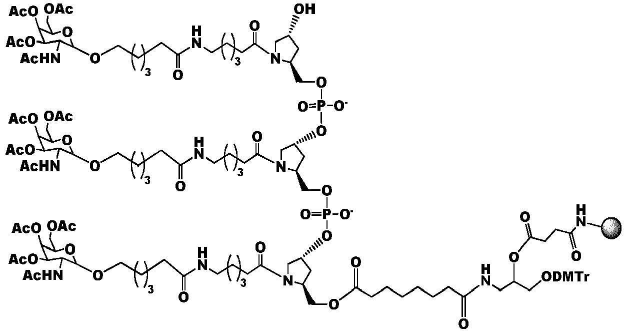

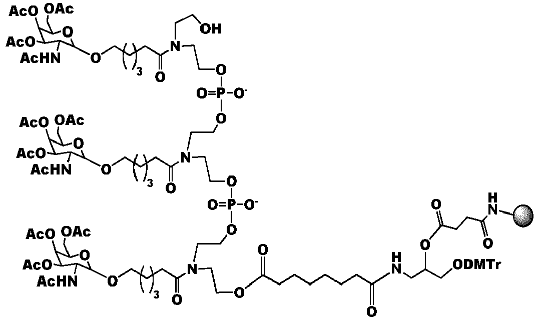

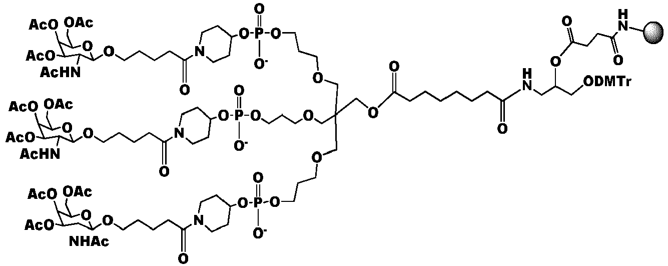

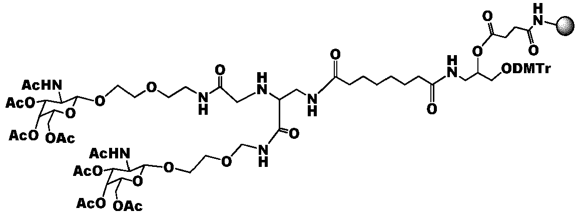

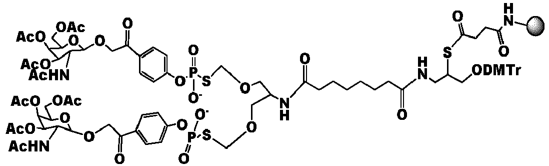

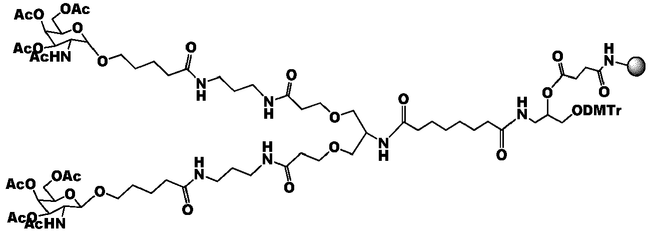

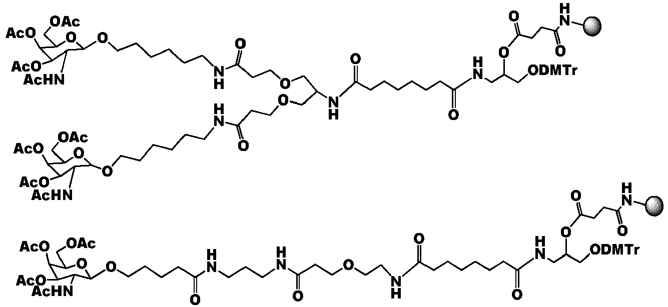

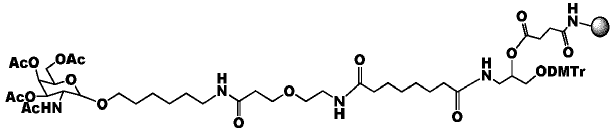

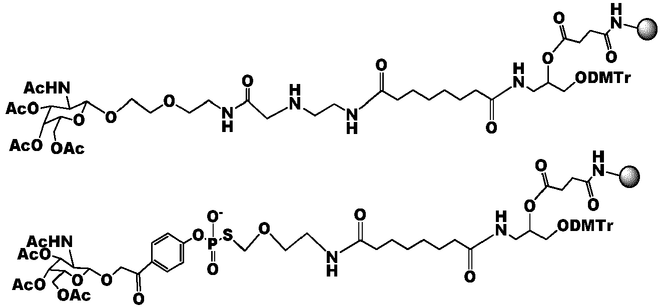

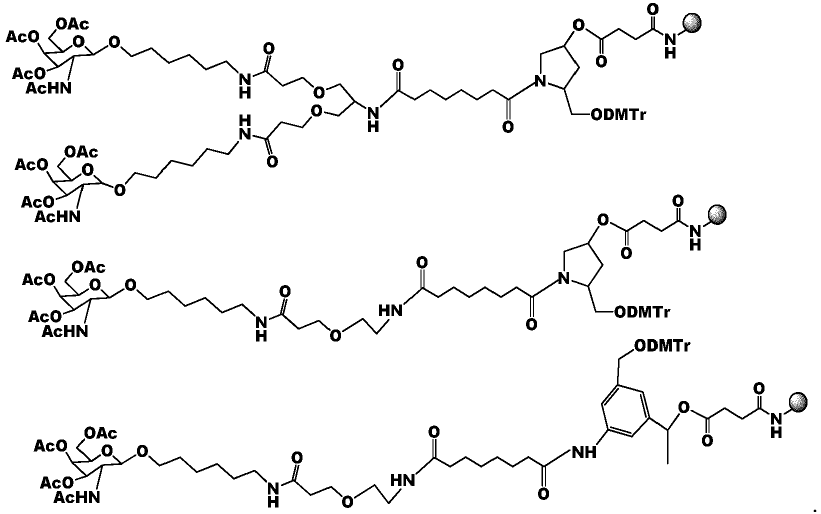

In some preferred embodiments, (X-L)ₙ-B-D- and R₁ in the structure of the 5'MVIP ligand are combined as shown in Table 3.

**Table 3: Combinations of (X-L)ₙ-B-D- and R₁ in 5'MVIP**

| No. | Code of (X-L)ₙ-B-D- | R₁ | Code of 5'MVIP |
|---|---|---|---|
| 1 | 5'YICd-01 | -NH(CH₂)₆O- | 5'MVIP01 |
| 2 | 5'YICc-01 | -NH(CH₂)₆O- | 5'MVIP02 |
| 3 | 5'YICa-01 | -O(CH₂)₆O- | 5'MVIP03 |
| 4 | 5'YICa-02 | -O(CH₂)₆O- | 5'MVIP04 |
| 5 | 5'YICa-03 | -S(CH₂)₆O- | 5'MVIP05 |
| 6 | 5'YICa-04 | -NH(CH₂)₆S- | 5'MVIP06 |
| 7 | 5'YICa-05 | -NH(CH₂)₈O- | 5'MVIP07 |
| 8 | 5'YICr-06 | -NH(CH₂)₈O- | 5'MVIP08 |
| 9 | 5'ERCd-01 | -NH(CH₂)₆O- | 5'MVIP09 |
| 10 | 5'ERCc-01 | -NH(CH₂)₆O- | 5'MVIP10 |
| 11 | 5'ERCa-01 | -NH(CH₂)₅CH(CH₂CH₃)O- | 5'MVIP11 |
| 12 | 5'ERCa-02 | -O(CH₂)₆O- | 5'MVIP12 |
| 13 | 5'ERCa-03 | -S(CH₂)₆O- | 5'MVIP13 |
| 14 | 5'ERCa-04 | -O(CH₂)₆O- | 5'MVIP14 |
| 15 | 5'ERCa-05 | -O(CH₂)₆O- | 5'MVIP15 |
| 16 | 5'ERCr-06 | -S(CH₂)₄CH(CH₃)O- | 5'MVIP16 |
| 17 | 5'SANCd-01 | -NH(CH₂)₆O- | 5'MVIP17 |
| 18 | 5'SANCc-01 | -NH(CH₂)₆O- | 5'MVIP18 |

In some embodiments, 3'MVIP may be absent, in this case n may be 2 to 4.

In some embodiments, (X-L)ₘ-B-D- and R₂ in the structure of the 3'MVIP ligand are combined as shown in Table 4.

**Table 4: Combinations of (X-L)ₘ-B-D- and R₂ in 3'MVIP**

| No. | Code of **(X-L)ₘ-B-D-** | R | Code of 3'MVIP |
|---|---|---|---|
| 1 | 3'YICd-01 | 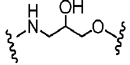 | 3'MVIP01 |
| 2 | 3'YICc-01 | 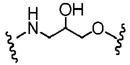 | 3'MVIP02 |
| 3 | 3'YICa-01 | 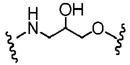 | 3'MVIP03 |
| 4 | 3'YICa-02 | 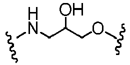 | 3'MVIP04 |
| 5 | 3'YICa-03 | 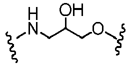 | 3'MVIP05 |
| 6 | 3'YICa-04 | 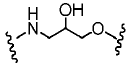 | 3'MVIP06 |
| 7 | 3'YICa-05 | 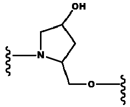 | 3'MVIP07 |
| 8 | 3'YICr-06 | 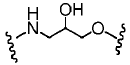 | 3'MVIP08 |
| 9 | 3'ERCd-01 | 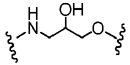 | 3'MVIP09 |
| 10 | 3'ERCc-01 | 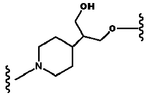 | 3'MVIP10 |
| 11 | 3'ERCa-01 | 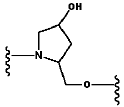 | 3'MVIP11 |
| 12 | 3'ERCa-02 | 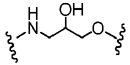 | 3'MVIP12 |
| 13 | 3'ERCa-03 | 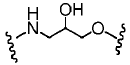 | 3'MVIP13 |
| 14 | 3'ERCa-04 | 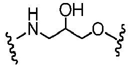 | 3'MVIP14 |
| 15 | 3'ERCa-05 | 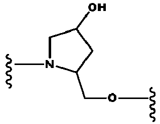 | 3'MVIP15 |
| 16 | 3'ERCr-06 | 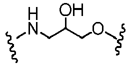 | 3'MVIP16 |
| 17 | 3'SANCd-01 | 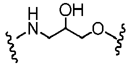 | 3'MVIP17 |
| 18 | 3'SANCc-01 | 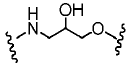 | 3'MVIP18 |
| 19 | 3'SANCa-01 | 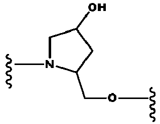 | 3'MVIP19 |
| 20 | 3'ERCd-01 | 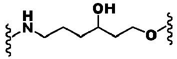 | 3'MVIP20 |
| 21 | 3'ERCd-01 | 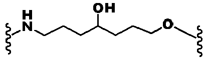 | 3'MVIP21 |
| 22 | 3'ERCd-01 | 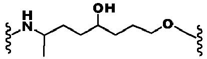 | 3'MVIP22 |
| 23 | 3'ERCd-01 | 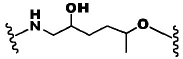 | 3'MVIP23 |
| 24 | 3'ERCd-01 | 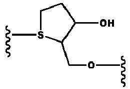 | 3'MVIP24 |
| 25 | 3'ERCd-01 | 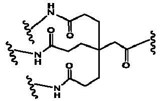 | 3'MVIP25 |
| 26 | 3'ERCd-01 | 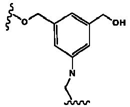 | 3'MVIP26 |
| 27 | 3'ERCd-01 | 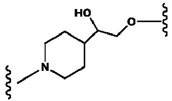 | 3'MVIP27 |

The sense strand and antisense strand in the structure of the RNA inhibitor provided by the present invention has a chain length of 15-30, preferably 19-23, and are at least 85% base complementary to each other. In order to enhance the stability of the sense strand and antisense strand *in vivo,* the sense strand and antisense strand of the RNA inhibitor may be modified in case that the activity is not affected or even enhanced. The nucleotides therein may have a modifying group, all or part of the chain may be modified, and preferably all of the chain is modified. The modification is a technique easily understood by a researcher in the art, and may be at the glycosyl moiety, and is one or more selected from deoxyribonucleotides, nucleotide mimics, abasic nucleotides, 2'-modified nucleotides, 3' to 3' linked (inverted) nucleotides, nucleotides comprising unnatural bases, bridging nucleotides, peptide nucleic acid (PNA), unlocked nucleobase analogs, locked nucleotides, 3'-O-methoxy (2' internucleoside linkage) nucleotides, 2'-F-arabinonucleotides, 5'-Me/2'-fluoronucleotides, morpholinonucleotides, vinylphosphonate deoxyribonucleotides, vinylphosphonate-containing nucleotides and cyclopropylphosphonate-containing nucleotides. Among them, 2'-modified nucleotides include, but are not limited to, 2'-O-methylnucleotides, 2'-deoxy-2'-fluoronucleotides, 2'-deoxynucleotides, 2'-methoxyethylnucleotides, 2'-aminonucleotides and 2'-alkylnucleotides. In the RNA inhibitor provided by the present invention, neither the sense strand nor the antisense strand of the RNA inhibitor needs to be uniformly modified, and more than one modification may be incorporated in a single nucleotide. Said modification may also occur in the base moiety, and the modified nucleobases include synthetic and natural nucleobases such as 5-substituted pyrimidines, 6-azapyrimidines and N-2/N-6 and O-6 substituted purine, 5-methylcytosine, 5-hydroxymethylcytosine, xanthine, hypoxanthine, 2-aminoadenine, 6-alkyl, 2-alkyl and other alkyl derivatives of adenine and guanine, 2-thiouracil, 2-thiothymine, 2-thiocytosine, 5-halouracil, cytosine, 5-propynyluracil, 5-propynylcytosine, 6-azouracil, 6-azocytosine, 6-azothymine, 5-uracil, 4-thiouracil, 8-halogen, 8-amino, 8-mercapto, 8-thioalkyl, 8-hydroxyl and other 8-substituted adenine and guanine, 5-halo, 5-trifluoromethyl and other 5-substituted uracil and cytosine, 7-methylguanine and 7-methyladenine, 8-azaguanine and 8-azaadenine, 7-deazaguanine, 7-deazaadenine, 3-deazaguanine and 3-deazaadenine.

Part or all of the sense strand and antisense strand of the RNA inhibitor of the present invention are 2'-O-methyl nucleotides and/or 2'-deoxy-2'-fluoro nucleotides, and at least two consecutive phosphorothioate bonds exist between the nucleotides at the 5' end of the sense strand and the 3' end of the antisense strand, preferably the phosphate bonds between the three consecutive nucleotides at the end are thioated.

In the RNA inhibitor provided by the present invention, when there is 3'MVIP on one single strand, the other single strand complementary to this single strand has corresponding 5'MVIP or 3'MVIP, or does not have; when there is 5'MVIP on one single strand of the RNA inhibitor, the other single strand complementary thereto has 3'MVIP or 5'MVIP, or does not have. The 5'MVIP and 3'MVIP can also be connected to the corresponding ends of the sense strand or antisense strand at the same time, that is, when the sense strand has 5'MVIP at the 5' end, it may also have 3'MVIP at the 3' end; and when the antisense strand has a 5'MVIP at the 5' end, it may also have 3'MVIP at the 3' end. Alternatively, the 5'MVIP is placed at the 5' ends of both the sense and antisense strands. Alternatively, the 3'MVIP is placed at the 3' ends of both the sense and antisense strands.

In some embodiments, it is preferred that different combinations of 5'MVIP and 3'MVIP in Table 5 below are incorporated into different positions of the sense strand and/or the antisense strand of the RNA inhibitor to investigate the impact on the HBsAg level of HBV.

**Table 5: Combinations of 5'MVIP and 3'MVIP**

| No. | Code of 5'MVIP | Structure of 5'MVIP | Code of 3'MVIP | Structure of 3'MVIP |
|---|---|---|---|---|
| 1 | 5'MVIPOl | 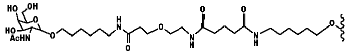 | 3'MVIP17 | 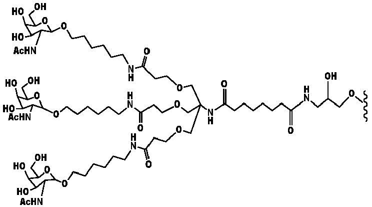 |
| 2 | 5'MVIP09 | 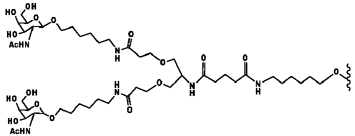 | 3'MVIP09 | 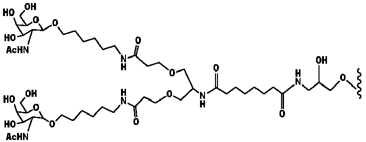 |
| 3 | 5'MVIP17 | 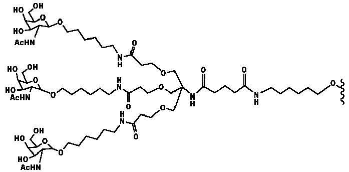 | 3'MVIPOl | 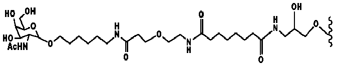 |
| 4 | 5'MVIPOl | 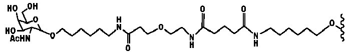 | 3'MVIPOl | 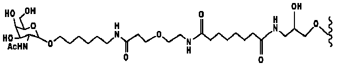 |
| 5 | 5'MVIPOl | 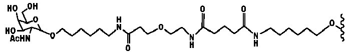 | 3'MVIP09 | 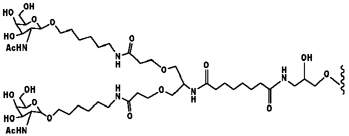 |
| 6 | 5'MVIP09 | 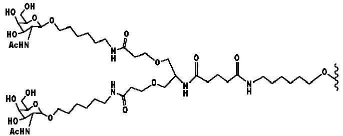 | 3'MVIPOl | 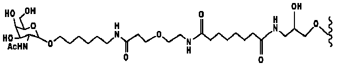 |

In some embodiments, the RNA inhibitor or a pharmaceutically acceptable salt thereof of the present invention is preferably prepared or synthesized in the form of carboxylate salts, sodium salts, triethylamine salts or other pharmaceutically acceptable salts.

In some embodiments, the RNA inhibitor or a pharmaceutically acceptable salt thereof is more preferably a sodium salt or triethylamine salt thereof.

In some embodiments, the sense strand of the RNA inhibitor is selected from the following Table 6.

**Table 6: Sense Strands of the RNA Inhibitor**

| SEQ ID NO. | Code of single chain | Sense strand sequence 5'→3'(19mer) |
|---|---|---|
| 1 | Ky-S | ggguuuuucucguugacaa |
| 2 | Ky-S0 | Gs fGs G U fU U fU fU fC U C G U U G A Cs As A |
| 3 | Ky-S1 | Gs fGs G U fU U fU fU fC U C G U U G A Cs As A- 3'MVIP17 |
| 4 | Ky-S2 | Gs fGs G U fU U fU fU fC U C G U U G A Cs As A -3'MVIP01 |
| 5 | Ky-S3 | Gs fGs G U fU U fU fU fC U C G U U G A Cs As A -3'MVIP09 |
| 6 | Ky-S4 | 5'MVIP17-Gs fGs GU fU U fU fU fC U C G U U G A Cs As A |
| 7 | Ky-S5 | 5'MVIP01-Gs fGs G U fU U fU fU fC U C G U U G A Cs As A |
| 8 | Ky-S6 | 5'MVIP09-Gs fGs G U fU U fU fU fC U C G U U G A Cs As A |
| 9 | Ky-S7 | 5'MVIP01-Gs fGs G U fU U fU fU fC U C G U U G A Cs As A -3'MVIP01 |
| 10 | Ky-S8 | 5'MVIP09-Gs fGs G U fU U fU fU fC U C G U U G A Cs As A -3'MVIP09 |
| 11 | Ky-S9 | 5'MVIP17-Gs fGs G U fU U fU fU fC U C G U U G A Cs As A -3'MVIP17 |
| 12 | Ky-S10 | 5'MVIP01-Gs fGs G U fU U fU fU fC U C G U U G A Cs As A -3'MVIP17 |
| 13 | Ky-S11 | 5'MVIP17-Gs fGs G U fU U fU fU fC U C G U U G A Cs As A -3'MVIP01 |
| 14 | Ky-S12 | 5'MVIP01-Gs fGs G U fU U fU fU fC U C G U U G A Cs As A-3'MVIP09 |
| 15 | Ky-S13 | 5'MVIP09-Gs fGs G U fU U fU fU fC U C G U U G A Cs As A -3'MVIP01 |
| 16 | Ky-S14 | 5'MVIP09-Gs fGs G U fU U fU fU fC U C G U U G A Cs As A -3'MVIP17 |
| 17 | Ky-S15 | 5'MVIP17-Gs fGs G U fU U fU fU fC U C G U U G A Cs As A -3'MVIP09 |
| 18 | Ky-S16 | 5'MVIP12-Gs fGs G U fU U fU fU fC U C G U U G A Cs As A |
| 19 | Ky-S17 | Gs fGs G U fU U fU fU fC U C G U U G A Cs As A -3'MVIP19 |
| 20 | Ky-S18 | 5'MVIP16-Gs fGs G U fU U fU fU fC U C G U U G A Cs As A -3'MVIP16 |
| 21 | Ky-S19 | Gs fGs G U fU U fU fU fC U C G U U G A Cs As A -3'MVIP17 |
| 22 | Ky-S20 | Gs fGs G U fU U fU fU fC U C G U U G A Cs As A -3'MVIP18 |
| 23 | Ky-S21 | 5' MVIP03-Gs fGs G U fU U fU fU fC U C G U U G A Cs As A |
| 24 | Ky-S22 | 5' MVIP08-Gs fGs G U fU U fU fU fC U C G U U G A Cs As A |
| 25 | Ky-S23 | 5' MVIP16-Gs fGs G U fU U fU fU fC U C G U U G A Cs As A |
| 26 | Ky-S24 | 5'MVIP13-Gs fGs G U fU U fU fU fC U C G U U G A Cs As A -3'MVIP06 |
| 27 | Ky-S25 | 5'MVIP04-Gs fGs G U fU U fU fU fC U C G U U G A Cs As A -3'MVIP06 |
| 28 | Ky-S26 | 5'MVIP11-Gs fGs G U fU U fU fU fC U C G U U G A Cs As A |
| 29 | Ky-S27 | 5'MVIP11-Gs fGs G U fU U fU fC U C G U U G A Cs As A -3'MVIP14 |
| 30 | Ky-S28 | 5'MVIP15-Gs fGs G U fU U fU fU fC U C G U U G A Cs As A |
| 31 | Ky-S29 | 5'MVIP02-Gs fGs G U fU U fU fU fC U C G U U G A Cs As A |
| 32 | Ky-S30 | 5'MVIP05-Gs fGs G U fU U fU fU fC U C G U U G A Cs As A |
| 33 | Ky-S31 | 5'MVIP06-Gs fGs G U fU U fU fU fC U C G U U G A Cs As A |
| 34 | Ky-S32 | 5'MVIP07-Gs fGs G U fU U fU fU fC U C G U U G A Cs As A |
| 35 | Ky-S33 | 5'MVIP10-Gs fGs G U fU U fU fU fC U C G U U G A Cs As A |
| 36 | Ky-S34 | 5'MVIP14-Gs fGs G U fU U fU fU fC U C G U U G A Cs As A |
| 37 | Ky-S35 | 5'MVIP18-Gs fGs G U fU U fU fU fC U C G U U G A Cs As A |
| 38 | Ky-S36 | Gs fGs G U fU U fU fU fC U C G U U G A Cs As A -3' MVIP02 |
| 39 | Ky-S37 | Gs fGs G U fU U fU fU fC U C G U U G A Cs As A -3' MVIP03 |
| 40 | Ky-S38 | Gs fGs G U fU U fU fU fC U C G U U G A Cs As A -3' MVIP04 |
| 41 | Ky-S39 | 5' MVIP04-Gs fGs G U fU U fU fU fC U C G U U G A Cs As A -3' MVIP04 |
| 42 | Ky-S40 | 5' MVIP03-Gs fGs G U fU U fU fU fC U C G U U G A Cs As A -3' MVIP19 |
| 43 | Ky-S41 | 5' MVIP18-Gs fGs G U fU U fU fU fC U C G U U G A Cs As A -3' MVIP18 |
| 44 | Ky-S42 | 5' MVIP08-Gs fGs G U fU U fU fU fC U C G U U G A Cs As A -3' MVIP18 |
| 45 | Ky-S43 | Gs fGs G U fU U fU fU fC U C G U U G A Cs As A -3' MVIP05 |
| 46 | Ky-S44 | Gs fGs G U fU U fU fU fC U C G U U G A Cs As A -3' MVIP07 |
| 47 | Ky-S45 | Gs fGs G U fU U fU fU fC U C G U U G A Cs As A -3' MVIP10 |
| 48 | Ky-S46 | Gs fGs G U fU U fU fU fC U C G U U G A Cs As A -3' MVIP11 |
| 49 | Ky-S47 | 5' MVIP11-Gs fGs G U fU U fU fU fC U C G U U G A Cs As A -3' MVIP11 |
| 50 | Ky-S48 | 5' MVIP15-Gs fGs G U fU U fU fU fC U C G U U G A Cs As A -3' MVIP15 |
| 51 | Ky-S49 | Gs fGs G U fU U fU fU fC U C G U U G A Cs As A -3' MVIP06 |
| 52 | Ky-S50 | Gs fGs G U fU U fU fU fC U C G U U G A Cs As A -3' MVIP08 |
| 53 | Ky-S51 | Gs fGs G U fU U fU fU fC U C G U U G A Cs As A -3' MVIP12 |
| 54 | Ky-S52 | Gs fGs G U fU U fU fU fC U C G U U G A Cs As A -3' MVIP13 |
| 55 | Ky-S53 | Gs fGs G U fU U fU fU fC U C G U U G A Cs As A -3' MVIP14 |
| 56 | Ky-S54 | Gs fGs G U fU U fU fU fC U C G U U G A Cs As A -3' MVIP15 |
| 57 | Ky-S55 | Gs fGs G U fU U fU fU fC U C G U U G A Cs As A -3' MVIP16 |

In some embodiments, the sense strand of the RNA inhibitor of the present invention differs from the respective sequences in Table 6 by one, two or three nucleotides.

In some embodiments, the antisense strand of the RNA inhibitor is selected from Table 7 below.

**Table 7: Antisense Strands of the RNA Inhibitor**

| SEQ ID NO. | Code of single chain | Antisense strand sequence 5'→3' (21-mer) |
|---|---|---|
| 58 | Ky-AS | uugucaacgagaaaaacccuu |
| 59 | Ky-AS0 | 5'Us Us G U C A fA C G A G fA A fA fA A C C Cs Us U 3' |
| 60 | Ky-AS1 | 5' MVIP01-Us Us G U C A fA C G A G fA A fA fA A C C Cs Us U |
| 61 | Ky-AS2 | 5' MVIP09-Us Us G U C A fA C G A G fA A fA fA A C C Cs Us U |
| 62 | Ky-AS3 | 5' MVIP17-Us Us G U C A fA C G A G fA A fA fA A C C Cs Us U |
| 63 | Ky-AS4 | Us Us G U C A fA C G A G fA A fA fA A C C Cs Us U -3' MVIP01 |
| 64 | Ky-ASS | Us Us G U C A fA C G A G fA A fA fA A C C Cs Us U -3' MVIP09 |
| 65 | Ky-AS6 | Us Us G U C A fA C G A G fA A fA fA A C C Cs Us U -3' MVIP17 |
| 66 | Ky-AS7 | 5'MVIP01-Us Us G U C A fA C GAG fA A fA fA A C C Cs Us U -3'MVIP01 |
| 67 | Ky-AS8 | 5' MVIP09-Us Us G U C A fA C G A G fA A fA fA A C C Cs Us U -3' MVIP09 |
| 68 | Ky-AS9 | 5'MVIP17-Us Us G U C A fA C GAG fA A fA fA A C C Cs Us U -3'MVIP17 |
| 69 | Ky-AS 10 | 5'MVIP01-Us Us G U C A fA C GAG fA A fA fA A C C Cs Us U -3'MVIP17 |
| 70 | Ky-ASll | 5' MVIP17-Us Us G U C A fA C G A G fA A fA fA A C C Cs Us U -3' MVIP01 |
| 71 | Ky-AS 12 | 5'MVIP01-Us Us G U C A fA C GAG fA A fA fA A C C Cs Us U -3'MVIP09 |
| 72 | Ky-AS13 | 5' MVIP09-Us Us G U C A fA C G A G fA A fA fA A C C Cs Us U -3' MVIP01 |
| 73 | Ky-AS14 | 5'MVIP09-Us Us G U C A fA C GAG fA A fA fA A C C Cs Us U -3'MVIP17 |
| 74 | Ky-AS15 | 5' MVIP17-Us Us G U C A fA C G A G fA A fA fA A C C Cs Us U -3' MVIP09 |
| 75 | Ky-AS16 | 5' MVIP12-Us Us G U C A fA C G A G fA A fA fA A C C Cs Us U |
| 76 | Ky-AS17 | Us Us G U C A fA C G A G fA A fA fA A C C Cs Us U-3' MVIP19 |
| 77 | Ky-AS18 | 5'MVIP16-Us Us G U C A fA C G A G fA A fA fA A C C Cs Us U -3'MVIP16 |
| 78 | Ky-AS19 | Us Us G U C A fA C G A G fA A fA fA A C C Cs Us U-3' MVIP17 |
| 79 | Ky-AS20 | Us Us G U C A fA C G A G fA A fA fA A C C Cs Us U-3' MVIP18 |
| 80 | Ky-AS21 | 5' MVIP03-Us Us G U C A fA C G A G fA A fA fA A C C Cs Us U |
| 81 | Ky-AS22 | 5' MVIP08-Us Us G U C A fA C G A G fA A fA fA A C C Cs Us U |
| 82 | Ky-AS23 | 5'MVIP16-Us Us G U C A fA C G A G fA A fA fA A C C Cs Us U |
| 83 | Ky-AS24 | 5'MVIP13-Us Us G U C A fA C G A G fA A fA fA A C C Cs Us U-3'MVIP06 |
| 84 | Ky-AS25 | 5'MVIP04-Us Us G U C A fA C G A G fA A fA fA A C C Cs Us U-3'MVIP06 |
| 85 | Ky-AS26 | 5' MVIP11-Us Us G U C A fA C G A G fA A fA fA A CC Cs Us U |
| 86 | Ky-AS27 | 5' MVIP11-Us Us G U C A fA C G A G fA A fA fA A C C Cs Us U-3' MVIP14 |
| 87 | Ky-AS28 | 5' MVIP15-Us Us G U C A fA C G A G fA A fA fA A C C Cs Us U |
| 88 | Ky-AS29 | 5' MVIP02-Us Us G U C A fA C G A G fA A fA fA A C C Cs Us U |
| 89 | Ky-AS30 | 5' MVIP05-Us Us G U C A fA C G A G fA A fA fA A C C Cs Us U |
| 90 | Ky-AS31 | 5' MVIP06-Us Us G U C A fA C G A G fA A fA fA A C C Cs Us U |
| 91 | Ky-AS32 | 5' MVIP07-Us Us G U C A fA C G A G fA A fA fA A C C Cs Us U |
| 92 | Ky-AS33 | 5' MVIP10- Us Us G U C A fA C G A G fA A fA fA A C C Cs Us U |
| 93 | Ky-AS34 | 5' MVIP14- Us Us G U C A fA C G A G fA A fA fA A C C Cs Us U |
| 94 | Ky-AS35 | 5' MVIP18- Us Us G U C A fA C G A G fA A fA fA A C C Cs Us U |
| 95 | Ky-AS36 | Us Us G U C A fA C G A G fA A fA fA A C C Cs Us U -3' MVIP02 |
| 96 | Ky-AS37 | Us Us G U C A fA C G A G fA A fA fA A C C Cs Us U-3' MVIP03 |
| 97 | Ky-AS38 | Us Us G U C A fA C G A G fA A fA fA A C C Cs Us U -3' MVIP04 |
| 98 | Ky-AS39 | 5' MVIP04-Us Us G U C A fA C G A G fA A fA fA A C C Cs Us U-3' MVIP04 |
| 99 | Ky-AS40 | 5'MVIP03-Us Us G U C A fA C GAG fA A fA fA A C C Cs Us U -3'MVIP19 |
| 100 | Ky-AS41 | 5' MVIP18- Us Us G U C A fA C G A G fA A fA fA A C C Cs Us U-3' MVIP18 |
| 101 | Ky-AS42 | 5' MVIP08- Us Us G U C A fA C G A G fA A fA fA A C C Cs Us U-3' MVIP18 |
| 102 | Ky-AS43 | Us Us G U C A fA C G A G fA A fA fA A C C Cs Us U -3' MVIP05 |
| 103 | Ky-AS44 | Us Us G U C A fA C G A G fA A fA fA A C C Cs Us U -3' MVIP07 |
| 104 | Ky-AS45 | Us Us G U C A fA C G A G fA A fA fA A C C Cs Us U -3' MVIP10 |
| 105 | Ky-AS46 | Us Us G U C A fA C G A G fA A fA fA A C C Cs Us U -3' MVIP11 |
| 106 | Ky-AS47 | 5' MVIP11- Us Us G U C A fA C G A G fA A fA fA A C C Cs Us U-3' MVIP11 |
| 107 | Ky-AS48 | 5' MVIP15- Us Us G U C A fA C G A G fA A fA fA A C C Cs Us U-3' MVIP15 |
| 108 | Ky-AS49 | Us Us G U C A fA C G A G fA A fA fA A C C Cs Us U -3' MVIP06 |
| 109 | Ky-AS50 | Us Us G U C A fA C G A G fA A fA fA A C C Cs Us U -3' MVIP08 |
| 110 | Ky-AS51 | Us Us G U C A fA C G A G fA A fA fA A C C Cs Us U -3' MVIP12 |
| 111 | Ky-AS52 | Us Us G U C A fA C G A G fA A fA fA A C C Cs Us U -3' MVIP13 |
| 112 | Ky-AS53 | Us Us G U C A fA C G A G fA A fA fA A C C Cs Us U -3' MVIP14 |
| 113 | Ky-AS54 | Us Us G U C A fA C G A G fA A fA fA A C C Cs Us U -3' MVIP15 |
| 114 | Ky-AS55 | Us Us G U C A fA C G A G fA A fA fA A C C Cs Us U -3' MVIP16 |

In some embodiments, the antisense strand of the RNA inhibitor of the present invention differs from the respective sequences in Table 7 by one, two or three nucleotides.

In some embodiments for investigating *in vivo* and *in vitro* effects, the sense strand or antisense strand of the RNA inhibitor is selected from the following Table 8.

**Table 8: Sense strand or antisense strand of the RNA inhibitor**

| SEQ ID NO. | Code of single chain | Single strand sequence 5'→3' |
|---|---|---|
| 115 | Ky-S56 | Gs fGs G U fU U fU fU fC U C G U U G A C A As Us A |
| 116 | Ky-S57 | Gs fGs G U fU U fU fU fC U C G U U G A C A As Us A - 3' MVIP17 |
| 117 | Ky-S58 | Gs fGs G U fU U fU fU fC U C G U U G A C A As Us A -3'MVIP01 |
| 118 | Ky-S59 | Gs fGs G U fU U fU fU fC U C G U U G A C A As Us A -3' MVIP09 |
| 119 | Ky-S60 | 5'MVIP17-Gs fGs G U fU U fU fU fC U C G U U G AC A As Us A |
| 120 | Ky-S61 | 5' MVIP01-Gs fGs G U fU U fU fU fC U C G U U G AC A As Us A |
| 121 | Ky-S62 | 5' MVIP09-Gs fGs G U fU U fU fU fC U C G U U G A C A As Us A |
| 122 | Ky-S63 | 5' MVIP01-Gs fGs G U fU U fU fU fC U C G U U G A C A As Us A -3' MVIP01 |
| 123 | Ky-S64 | 5' MVIP09-Gs fGs G U fU U fU fU fC U C G U U G A C A As Us A -3'MVIP09 |
| 124 | Ky-S65 | 5' MVIP17-Gs fGs G U fU U fU fU fC U C G U U G A C A As Us A -3' MVIP17 |
| 125 | Ky-S66 | 5' MVIP01-Gs fGs G U fU U fU fU fC U C G U U G A C A As Us A -3' MVIP17 |
| 126 | Ky-S67 | 5' MVIP17-Gs fGs G U fU U fU fU fC U C G U U G A C A As Us A -3' MVIP01 |
| 127 | Ky-S68 | 5' MVIP01-Gs fGs G U fU U fU fU fC U C G U U G A C A As Us A -3' MVIP09 |
| 128 | Ky-S69 | 5' MVIP09-Gs fGs G U fU U fU fU fC U C G U U G A C A As Us A -3' MVIP01 |
| 129 | Ky-S70 | 5'MVIP09-Gs fGs G U fU U fU fU fC U C G U U G A C A As Us A -3'MVIP17 |
| 130 | Ky-S71 | 5' MVIP17-Gs fGs G U fU U fU fU fC U C G U U G A C A As Us A -3' MVIP09 |
| 131 | Ky-S72 | 5'MVIP09-G fG G U fU U fU fU fC U C G U U G A Cs As A |
| 132 | Ky-AS56 | Us Us G U C A fA C G A G fA A fA fA A C C C U U -3' MVIP09 |
| 133 | Ky-AS57 | Us Us G U C A fA C G A G fA A fA fA A C C Cs Gs C -3'MVIP09 |
| 134 | Ky-S73 | 5'MVIP09-Gs fGs G U fU U fU fU fC U C G U U G A C A A A As Us A |
| 135 | Ky-S74 | 5' MVIP01-Gs fGs G U fU U fU fU fC U C G U U G A C A As As A |
| 136 | Ky-AS58 | Us Us U U G U fC A A C G fA G fA fA A A A C C Cs Us U -3'MVIP09 |
| 137 | Ky-S75 | 5'MVIP09-Gs fGs G U fU U fU fU fC U C G U U G A C A A A A Us Cs C |
| 138 | Ky-S76 | 5'MVIP09-Gs fGs G U fU U fU fU fC U C G U U G A C A A A A U C Cs Us A |
| 139 | Ky-AS59 | Us As G G A U fU U U G U fC A fA fC G A G A A A A A C C Cs Us U-3'MVIP09 |
| 140 | Ky-S77 | ggguuuuucuuguugacaa |
| 141 | Ky-AS60 | uugucaacaagaaaaacccuu |
| 142 | Ky-S78 | Gs Gs G U fU U fU fU fC U U G U U G A Cs As A |
| 143 | Ky-AS61 | Us Us G U C A fA C A A G fA A fA A A C C Cs Us U |
| 144 | Ky-S79 | 5'MVIP09-Gs Gs G U fU U fU fU fC U U G U U G A Cs As A |
| 145 | Ky-AS62 | Us Us G U C A fA C A A G fA A fA A A C C Cs Us U -3' MVIP09 |
| 146 | Ky-S80 | 5'MVIP09-CsUsUACGCUGAGfUACUUCGAsAsA |
| 147 | Ky-AS63 | UsCsGAAGfUACUfCAGCGfUAAGsUsU-3'MVIP09 |

In some embodiments, the sense strand or antisense strand of the RNA inhibitor of the present invention differs from respective sequences in Table 8 by one, two or three nucleotides.

In some embodiments, HepG2.2.15 cell line is used to assess 5'MVIP and 3'MVIP at the corresponding ends of the sense strand (SEQ ID NO. 2) and/or the antisense strand (SEQ ID NO. 59), and the effect of the resulting RNA inhibitors on reducing the HBsAg level of HBV. The code of the RNA inhibitors, the single strands contained and SEQ ID NO. are shown in Table 9:

**Table 9**

| Code of RNA inhibitor | Code of single strand | SEQ ID NO. |
|---|---|---|
| Ky-00 | Ky-S0 | 2 |
| | Ky-AS0 | 59 |
| Ky-01 | Ky-S1 | 3 |
| | Ky-AS0 | 59 |
| Ky-02 | Ky-S1 | 3 |
| | Ky-AS1 | 60 |
| Ky-03 | Ky-S1 | 3 |
| | Ky-AS2 | 61 |
| Ky-04 | Ky-S1 | 3 |
| | Ky-AS3 | 62 |
| Ky-05 | Ky-S0 | 2 |
| | Ky-AS3 | 62 |
| Ky-06 | Ky-S2 | 4 |
| | Ky-AS3 | 62 |
| Ky-07 | Ky-S3 | 5 |
| | Ky-AS3 | 63 |
| Ky-08 | Ky-S3 | 5 |
| | Ky-AS1 | 60 |
| Ky-09 | Ky-S3 | 5 |
| | Ky-AS2 | 61 |
| Ky-10 | Ky-S2 | 4 |
| | Ky-AS2 | 61 |
| Ky-11 | Ky-S0 | 2 |
| | Ky-AS2 | 61 |
| Ky-12 | Ky-S3 | 5 |
| | Ky-AS0 | 59 |
| Ky-13 | Ky-S2 | 4 |
| | Ky-AS1 | 60 |
| Ky-14 | Ky-S4 | 6 |
| | Ky-AS0 | 59 |
| Ky-15 | Ky-S4 | 6 |
| | Ky-AS4 | 63 |
| Ky-16 | Ky-S4 | 6 |
| | Ky-ASS | 64 |
| Ky-17 | Ky-S4 | 6 |
| | Ky-AS6 | 65 |
| Ky-18 | Ky-S0 | 2 |
| | Ky-AS6 | 65 |
| Ky-19 | Ky-S5 | 7 |
| | Ky-AS6 | 65 |
| Ky-20 | Ky-S6 | 8 |
| | Ky-AS6 | 65 |
| Ky-21 | Ky-S6 | 8 |
| | Ky-AS4 | 63 |
| Ky-22 | Ky-S6 | 8 |
| | Ky-ASS | 64 |
| Ky-23 | Ky-S5 | 7 |
| | Ky-ASS | 64 |
| Ky-24 | Ky-S0 | 2 |
| | Ky-ASS | 64 |
| Ky-25 | Ky-S6 | 8 |
| | Ky-AS0 | 59 |
| Ky-26 | Ky-S5 | 7 |
| | Ky-AS4 | 63 |
| Ky-27 | Ky-S7 | 9 |
| | Ky-AS0 | 59 |
| Ky-28 | Ky-S0 | 2 |
| | Ky-AS7 | 66 |
| Ky-29 | Ky-S8 | 10 |
| | Ky-AS0 | 59 |
| Ky-30 | Ky-S0 | 2 |
| | Ky-AS8 | 67 |
| Ky-31 | Ky-S9 | 11 |
| | Ky-AS0 | 59 |
| Ky-32 | Ky-S0 | 2 |
| | Ky-AS9 | 68 |
| Ky-33 | Ky-S10 | 12 |
| | Ky-AS0 | 59 |
| Ky-34 | Ky-S0 | 2 |
| | Ky-AS10 | 69 |
| Ky-35 | Ky-S11 | 13 |
| | Ky-AS0 | 69 |
| Ky-36 | Ky-S0 | 2 |
| | Ky-AS11 | 70 |
| Ky-37 | Ky-S12 | 14 |
| | Ky-AS0 | 59 |
| Ky-38 | Ky-S0 | 2 |
| | Ky-AS12 | 71 |
| Ky-39 | Ky-S13 | 15 |
| | Ky-AS0 | 59 |
| Ky-40 | Ky-S0 | 2 |
| | Ky-AS13 | 72 |
| Ky-41 | Ky-S14 | 16 |
| | Ky-AS0 | 59 |
| Ky-42 | Ky-S0 | 2 |
| | Ky-AS14 | 73 |
| Ky-43 | Ky-S15 | 17 |
| | Ky-AS0 | 59 |
| Ky-44 | Ky-S0 | 2 |
| | Ky-AS15 | 74 |
| Ky-45 | Ky-S5 | 7 |
| | Ky-AS1 | 60 |
| Ky-46 | Ky-S6 | 8 |
| | Ky-AS2 | 61 |
| Ky-47 | Ky-S4 | 6 |
| | Ky-AS3 | 62 |
| Ky-48 | Ky-S5 | 7 |
| | Ky-AS3 | 62 |
| Ky-49 | Ky-S5 | 7 |
| | Ky-AS2 | 61 |
| Ky-50 | Ky-S6 | 8 |
| | Ky-AS3 | 62 |
| Ky-51 | Ky-S6 | 8 |
| | Ky-AS1 | 60 |
| Ky-52 | Ky-S4 | 6 |
| | Ky-AS1 | 60 |
| Ky-53 | Ky-S4 | 6 |
| | Ky-AS2 | 61 |
| Ky-54 | Ky-S2 | 4 |
| | Ky-AS4 | 63 |
| Ky-55 | Ky-S3 | 5 |
| | Ky-ASS | 64 |
| Ky-56 | Ky-S1 | 3 |
| | Ky-AS6 | 65 |
| Ky-57 | Ky-S2 | 4 |
| | Ky-AS6 | 65 |
| Ky-58 | Ky-S2 | 4 |
| | Ky-ASS | 64 |
| Ky-59 | Ky-S3 | 5 |
| | Ky-AS6 | 65 |
| Ky-60 | Ky-ASS | 5 |
| | Ky-AS4 | 63 |
| Ky-61 | Ky-S1 | 3 |
| | Ky-ASS | 64 |
| Ky-62 | Ky-S1 | 3 |
| | Ky-AS4 | 63 |

In some embodiments, the combinations of 5'MVIP01/3'MVIP01, 5'MVIP01/3'MVIP17, and 5'MVIP09/3'MVIP09 are preferred and incorporated at the 5' end of the sense strand and the 3' end of the antisense strand.

In some embodiments, 5'MVIP01/3'MVIP09 and 5'MVIP09/3'MVIP01 are preferred and incorporated at the 5' end and the 3' end of the sense strand.

In another aspect, the present invention also provides a use of the RNA inhibitor or a pharmaceutically acceptable salt thereof in preparation of a medicament for treatment of a hepatogenic disease, which includes, but not limited to, hepatitis, liver tumors, cirrhosis, jaundice, type 2 diabetes, fatty liver, coagulation diseases of the blood system, diseases related to blood albumin and globulin, hyperlipidemia, atherosclerosis, and essential hypertension.

In a further aspect, the present invention provides a pharmaceutical composition, which comprises the RNA inhibitor or a pharmaceutically acceptable salt thereof and a pharmaceutically acceptable excipient, and its dosage form may be an oral agent, an intravenous injection or a subcutaneous or intramuscular injection, preferably a subcutaneous injection.

In yet another aspect, the present invention provides a pharmaceutical composition, which comprises the RNA inhibitor or a pharmaceutically acceptable salt thereof, and an other drug for treating hepatitis B. The other drug for treating hepatitis B includes, but not limited to, nucleoside analogues or interferons that have been used clinically, as well as some candidates for hepatitis B treatment under research, such as immunomodulators. In one embodiment, the RNA inhibitor of the present invention was compared with tenofovir, the first-line drug currently used in the treatment of chronic hepatitis B, on inhibitory effect against HBsAg of HBV on a transgenic mouse model. The test results have confirmed that the nucleoside analog as an anti-hepatitis B drug has no inhibitory effect on HBsAg of HBV, and when used in combination, it does not affect the inhibitory effect of the RNA inhibitor of the present invention on HBsAg.

In some embodiments, the RNA inhibitor of the present invention is used in combination with entecavir or interferon, the first-line drugs currently used in the treatment of chronic hepatitis B, to investigate the inhibitory effect on HBV and whether there is mutual interference. The experiment evaluated the inhibitory effect of the RNA inhibitor of the present invention combined with different concentrations of entecavir or interferon on HBV in the widely used HepG2.2.15 cell line.

In some embodiments, the inhibitory effects of the RNA inhibitor of the present invention on the four common subtypes A, B, C and D of HBV were investigated by using siRNA with a sense strand of SEQ ID NO. 146 and an antisense strand of SEQ ID NO. 147 as the negative control.

In some embodiments, the 5'MVIP and/or 3'MVIP with the different X, L, B, D, R₁ and R₂ were coupled to the corresponding ends of the sense strand (SEQ ID NO. 2) and the antisense strand (SEQ ID NO. 59) of the RNA inhibitor, and the effects of the above obtained RNA inhibitors on reducing HBsAg level of HBV were investigated by using HepG2.2.15 cell line, wherein when one of X, L, B, D, R₁ and R₂ was different, other parts of the corresponding 5'MVIP and/or 3'MVIP were the same as those of 5'MVIP09/3'MVIP09.

In some embodiments, HepG2.2.15 cell line was used to investigate the influence of different liver targeting specific ligands X on the effect of the RNA inhibitor on reducing HBsAg level of HBV.

**Table 10**

| Code of X | Code of RNA inhibitor | Structure of X |
|---|---|---|
| X1 | Ky-22 | 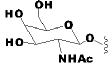 |
| X2 | Ky-22-X2 | 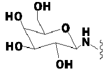 |
| X3 | Ky-22-X3 | 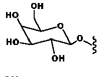 |
| X4 | Ky-22-X4 | 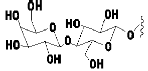 |
| X5 | Ky-22-X5 | 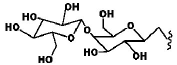 |
| X6 | Ky-22-X6 | 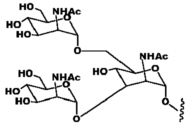 |

In some embodiments, HepG2.2.15 cell line was used to investigate the influence of different branched chains L on the action effect of the RNA inhibitor.

**Table 11**

| Code of L | Code of RNA inhibitor | Structure of L |
|---|---|---|
| L1 | Ky-22 | 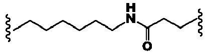 |
| L2 | Ky-22-L2 | 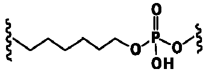 |
| L3 | Ky-22-L3 | 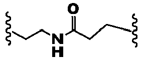 |
| L4 | Ky-22-L4 | 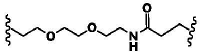 |
| L5 | Ky-22-L5 | 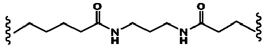 |
| L6 | Ky-22-L6 | 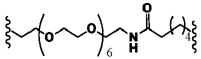 |
| L7 | Ky-22-L7 | 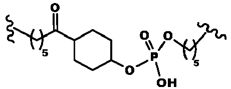 |
| L8 | Ky-22-L8 | 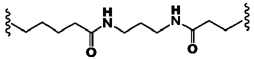 |
| L9 | Ky-22-L9 | 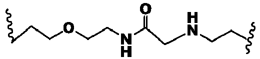 |
| L10 | Ky-22-L10 | 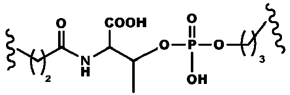 |
| L11 | Ky-22-L 11 * | 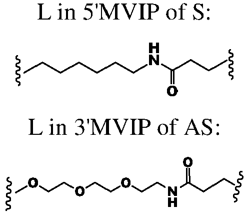 |
| L12 | Ky-22-L12* | 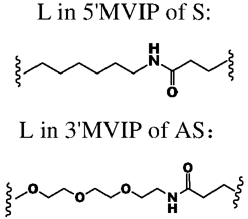 |
| L13 | Ky-22-L13* | 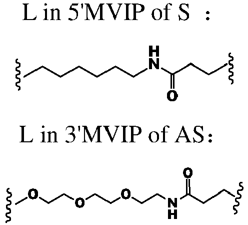 |
| L14 | Ky-22-L14 | 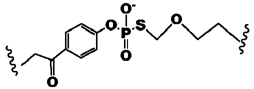 |

| | | |
|---|---|---|
| Note: RNA inhibitors marked with * indicate that the structures of L in the same 5'MVIP or 3'MVIP and between the 5'MVIP and 3'MVIP are different from each other. | | |

In some embodiments, HepG2.2.15 cell line was used to investigate the influence of different linkers B on the effect of the RNA inhibitor on reducing the HBsAg level of HBV.

**Table 12**

| Code of B | Code of RNA inhibitor | Structure of B |
|---|---|---|
| B1 | Ky-22 | 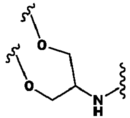 |
| B2 | Ky-22-B2 | 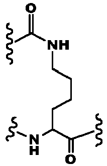 |
| B3 | Ky-22-B3 | 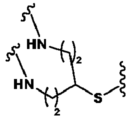 |
| B4 | Ky-22-B4 | 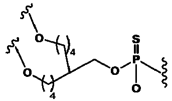 |
| B5 | Ky-22-B5 | 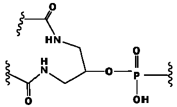 |
| B6 | Ky-22-B6 | 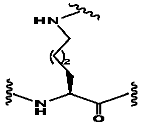 |
| B7 | Ky-22-B7 | 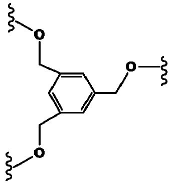 |
| B8 | Ky-19* | B in 5'MVIP of S: |
| | | 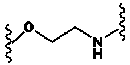 |
| | | B in 3'MVIP of AS: |
| | | 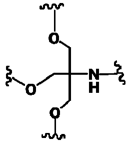 |
| B9 | Ky-19-B2* | B in 5'MVIP of S: |
| | | 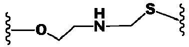 |
| | | B in 3'MVIP of AS: |
| | | 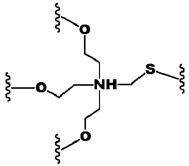 |
| B10 | Ky-19-B3* | B in 5'MVIP of S: |
| | | 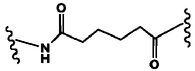 |
| | | B in 3'MVIP of AS: |
| | | 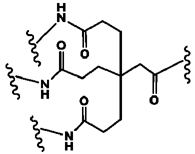 |
| B11 | Ky-19-B4* | B in 5'MVIP of S: |
| | | 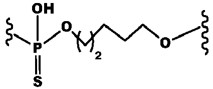 |
| | | B in 3'MVIP of AS: |
| | | 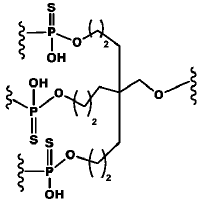 |
| B12 | Ky-19-B5* | B in 5'MVIP of S: |
| | | 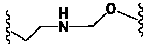 |
| | | B in 3'MVIP of AS: |
| | | 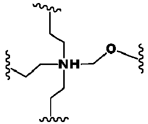 |
| B13 | Ky-19-B6* | B in 5'MVIP of S: |
| | | 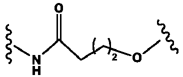 |
| | | B in 3'MVIP of AS: |
| | | 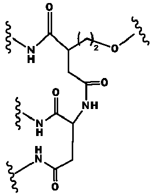 |
| B14 | Ky-19-B7* | B in 5'MVIP of S: |
| | | 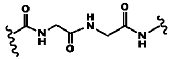 |
| | | B in 3'MVIP of AS: |
| | | 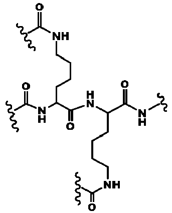 |
| B15 | Ky-19-B8* | B in 5'MVIP of S: |
| | | 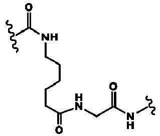 |
| | | B in 3'MVIP of AS: |
| | | 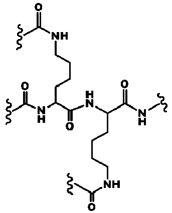 |
| B16 | Ky-19-B9* | B in 5'MVIP of S: |
| | | 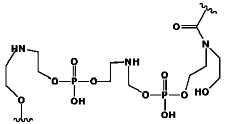 |
| | | B in 3'MVIP of AS: |
| | | 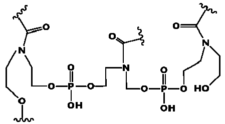 |
| B17 | Ky-19-B10* | B in 5'MVIP of S: |
| | | 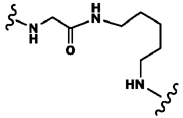 |
| | | B in 3'MVIP of AS: |
| | | 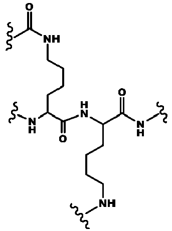 |
| B18 | Ky-19-B11* | B in 5'MVIP of S: |
| | | 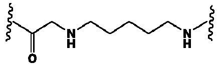 |
| | | B in 3'MVIP of AS: |
| | | 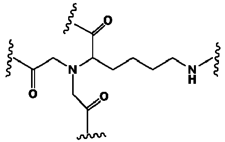 |
| B19 | Ky-19-B12* | B in 5'MVIP of S: |
| | | 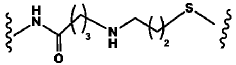 |
| | | B in 3'MVIP of AS: |
| | | 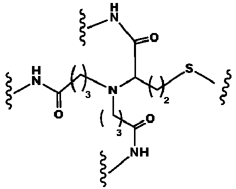 |
| B20 | Ky-26 | 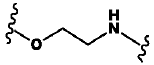 |
| B21 | Ky-26-B2 | 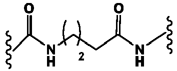 |
| B22 | Ky-26-B3 | 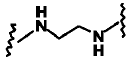 |
| B23 | Ky-26-B4 | 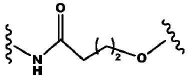 |
| B24 | Ky-26-B5 | 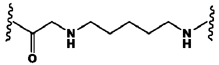 |
| B25 | Ky-26-B6 | 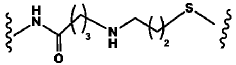 |
| B26 | Ky-26-B7 | 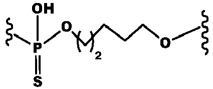 |
| B27 | Ky-37* | B in 5'MVIP of S: |
| | | 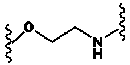 |
| | | B in 3'MVIP of AS: |
| | | 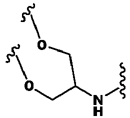 |
| B28 | Ky-37-B2* | B in 5'MVIP of S: |
| | | 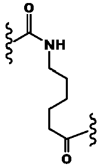 |
| | | B in 3'MVIP of AS: |
| | | 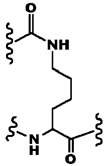 |
| B29 | Ky-37-B3* | B in 5'MVIP of S: |
| | | 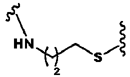 |
| | | B in 3'MVIP of AS: |
| | | 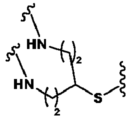 |
| B30 | Ky-37-B4* | B in 5'MVIP of S: |
| | | 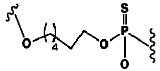 |
| | | B in 3'MVIP of AS: |
| | | 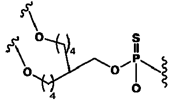 |
| B31 | Ky-37-B5* | B in 5'MVIP of S: |
| | | 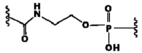 |
| | | B in 3'MVIP of AS: |
| | | 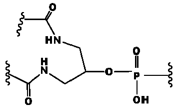 |
| B32 | Ky-37-B 6 * | B in 5'MVIP of S: |
| | | 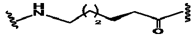 |
| | | B in 3'MVIP of AS: |
| | | 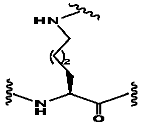 |
| B33 | Ky-39* | B in 5'MVIP of S: |
| | | 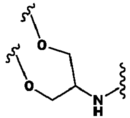 |
| | | B in 3'MVIP of AS: |
| | | 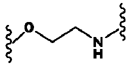 |
| B34 | Ky-39-B2* | B in 5'MVIP of S: |
| | | 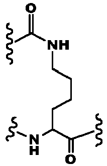 |
| | | B in 3'MVIP of AS: |
| | | 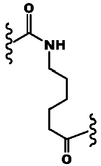 |
| B35 | Ky-39-B3* | B in 5'MVIP of S: |
| | | 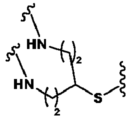 |
| | | B in 3'MVIP of AS: |
| | | 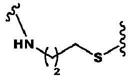 |
| B36 | Ky-39-B4* | B in 5'MVIP of S: |
| | | 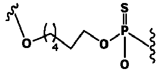 |
| | | B in 3'MVIP of AS: |
| | | 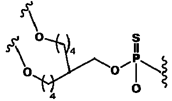 |
| B37 | Ky-39-B5* | B in 5'MVIP of S: |
| | | 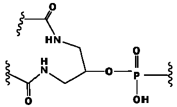 |
| | | B in 3'MVIP of AS: |
| | | 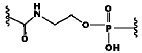 |
| B38 | Ky-39-B 6 * | B in 5'MVIP of S: |
| | | 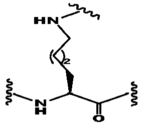 |
| | | B in 3'MVIP of AS: |
| | | 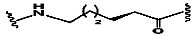 |

| | | |
|---|---|---|
| Note: RNA inhibitors marked with * indicate that the linkers B between 5'MVIP and 3'MVIP are different in structure. | | |

In some embodiments, HepG2.2.15 cell line was used to investigate the influence of different linking chains D on the effect of the RNA inhibitor on reducing the HBsAg level of HBV.

**Table 13**

| Code of D | Code of RNA inhibitor | Structure of D |
|---|---|---|
| D1 | Ky-22* | D in 5'MVIP of S: |
| | | 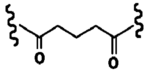 |
| | | D in 3'MVIP of AS: |
| | | 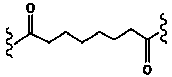 |
| D2 | Ky-22-D2* | D in 5'MVIP of S: |
| | | 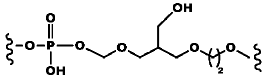 |
| | | D in 3'MVIP of AS: |
| | | 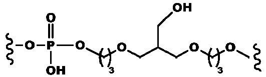 |
| D3 | Ky-22-D3* | D in 5'MVIP of S: |
| | | 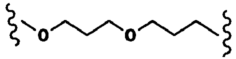 |
| | | D in 3'MVIP of AS: |
| | | 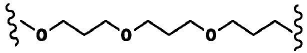 |
| D4 | Ky-22-D4* | D in 5'MVIP of S: |
| | | 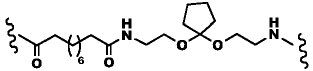 |
| | | D in 3'MVIP of AS: |
| | | 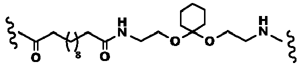 |
| D5 | Ky-22-D5* | D in 5'MVIP of S: |
| | | 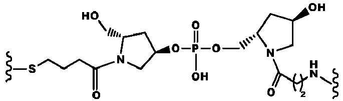 |
| | | D in 3'MVIP of AS: |
| | | 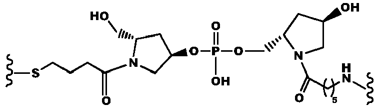 |

| | | |
|---|---|---|
| Note: RNA inhibitors marked with * indicate that the linking chains D between 5'MVIP and 3'MVIP are different in structure. | | |

In some embodiments, HepG2.2.15 cell line was used to investigate the influence of different transition points R₁ on the effect of the RNA inhibitor on reducing the HBsAg level of HBV.

**Table 14**

| Code of R₁ | Code of RNA inhibitor | Structure of R₁ |
|---|---|---|
| R1-1 | Ky-22 | -NH(CH₂)₆O- |
| R1-2 | Ky-22-R1-1 | -O(CH₂)₆O- |
| R1-3 | Ky-22-R1-2 | -S(CH₂)₆O- |
| R1-4 | Ky-22-R1-3 | -NH(CH₂)₈ O- |
| R1-5 | Ky-22-R1-4 | -NH(CH₂)₅CH(CH₂CH₃)O- |
| R1-6 | Ky-22-R1-5 | -S(CH₂)₄CH(CH₃)O- |

In some embodiments, HepG2.2.15 cell line was used to investigate the influence of different transition points R₂ on the effect of the RNA inhibitor on reducing the HBsAg level of HBV.

**Table 15**

| Code of R₂ | Code of RNA inhibitor | Structure of R₂ |
|---|---|---|
| R2-1 | Ky-22 | 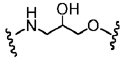 |
| R2-2 | Ky-22-R2-1 | 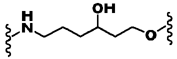 |
| R2-3 | Ky-22-R2-2 | 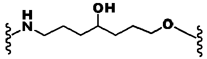 |
| R2-4 | Ky-22-R2-3 | 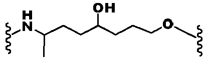 |
| R2-5 | Ky-22-R2-4 | 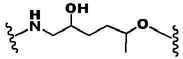 |
| R2-6 | Ky-22-R2-5 | 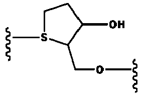 |
| R2-7 | Ky-22-R2-6 | 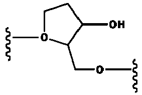 |
| R2-8 | Ky-22-R2-7 | 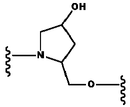 |
| R2-9 | Ky-22-R2-8 | 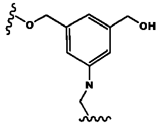 |
| R2-10 | Ky-22-R2-9 | 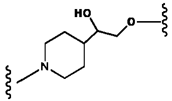 |
| R2-11 | Ky-22-R2-10 | 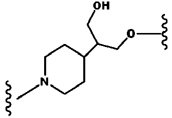 |
| R2-12 | Ky-22-R2-11 | 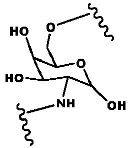 |

In some embodiments, the sequence of the RNA inhibitor Ky-22 described in the present invention was further optimized and adjusted, including the number of sequence mers, the allowable number of nucleotide differences, and the numbers of fluorine substitution and terminal thioation, the influence of these adjustments on the effect of the RNA inhibitor on reducing the level of HBsAg and the persistence of the effect was investigated, and the sequences are shown in Table 16.

**Table 16: Sequence adjustment table for Ky-22**

| Code of RNA inhibitor | Sequence 5'-3' |
|---|---|
| Ky-22 | 5'MVIP09-Gs fGs G U fU U fU fU fC U C G U U G A Cs As A |
| | Us Us G U C A fA C G A G fA A fA fA A C C Cs Us U -3' MVIP09 |
| Ky-2201 | 5'MVIP09-Gs fGs G U fU U fU fU fC U C G U U G A C A As Us A |
| | Us Us G U C A fA C G A G fA A fA fA A C C Cs Us U -3' MVIP09 |
| Ky-2202 | 5'MVIP09-Gs fGs G U fU U fU fU fC U C G U U G A C A As Us U |
| | Us Us G U C A fA C G A G fA A fA fA A C C Cs Us U -3' MVIP09 |
| Ky-2203 | 5'MVIP09-Gs fGs G U fU U fU fU fC U U G U U G A Cs As A |
| | Us Us G U C A fA C G A G fA A fA fA A C C Cs Us U -3' MVIP09 |
| Ky-2204 | 5'MVIP09-Gs fGs G U fU U fU RJ fC U U G U U G A C A As Gs C |
| | Us Us G U C A fA C G A G fA A fA fA A C C Cs Us U -3' MVIP09 |
| Ky-2205 | 5'MVIP09-Gs fGs G U fU U fU fU fC U U G U G A C A As Us A |
| | Us Us G U C A fA C G A G fA A fA fA A C C Cs Us U -3' MVIP09 |
| Ky-2206 | 5'MVIP09-Gs fGs G U fU U fU fU fC U C G U U G A Cs As A |
| | Us Us G U C A fA C G A G fA A fA fA A C C Cs Gs C -3' MVIP09 |
| Ky-2207 | 5'MVIP09-G fG G U fU U fU fU fC U C G U U G A Cs As A |
| | Us Us G U C A fA C G A G fA A fA fA A C C C U U -3' MVIP09 |
| Ky-2208 | 5'MVIP09-Gs Gs G U RJ U RJ fU fC U U G U U G A Cs As A |
| | Us Us G U C A fA C A A G fA A fA A A C C Cs Us U -3' MVIP09 |

The results of the embodiments showed that, compared with Ky-22, Ky-2201 with a sense chain length of 21-mer had no significant improvement, or even a slight decrease in the reduction of HBsAg level and the persistence of effect. Therefore, the RNA inhibitor provided by the present invention has optimally a sense chain length of 19-mer. Compared with Ky-22, Ky-2203, which has one nucleotide change in each of the sense chain and the antisense chain, had no significant influence on the reduction of HBsAg level and the persistence of effect. Ky-2204 with a sense chain length of 21-mer based on the design of Ky-2203, had no significant difference in effect from Ky-2203. Ky-2208 which was obtained by adjusting the number of fluorine substitution based on Ky-2203 and has a relatively less number of fluorine substitution, had an action effect slightly better than Ky-2203. The RNA inhibitor Ky-2205 obtain by modifying the two overhanging nucleotides at the 3' end of the sense strand of Ky-2204, the RNA inhibitor Ky-2206 obtained by modifying the two nucleotides at the 3' end of the antisense strand of Ky-22, and the RNA inhibitor Ky-2202 obtained by modifying the two overhanging nucleotides at the 3' end of the sense strand of Ky-2201 have no significant difference in effect from those before the modification, indicating that the RNA inhibitor of the present invention allows a difference of 1 to 3 nucleotides in the sense strand or antisense strand. Compared with Ky-22, Ky-2207 obtained by eliminating the thioation of the phosphate bonds between 3 consecutive nucleotides at 5' end of the sense strand and 3' end of the antisense strand, had a significant influence on the effect of reducing the HBsAg level and the persistence of effect.

In the present invention, preferred is a sequence with a sense strand length of 19-mer and an antisense strand length of 21-mer, allowing a difference of 1 to 3 nucleotides.

In some embodiments, the RNA inhibitor Ky-2208 and the nucleoside analog tenofovir (TDF) were investigated for comparison of their efficacy of anti-hepatitis B virus and their combination use in the transgenic mouse model. The test results showed that tenofovir (TDF) does not have the efficacy of reducing HBsAg level, Ky-2208 can effectively reduce the HBsAg level by up to 99.98%, and the combination with tenofovir (TDF) did not affect the effects of the RNA inhibitor of the present invention.

In some embodiments, the RNA inhibitors Ky-08, Ky-10, Ky-19, Ky-13, Ky-21, Ky-22, Ky-23, Ky-26, Ky-27, Ky-29, Ky-37 and Ky-39 of the present invention were investigated on the inhibitory effect on HBsAg in HBV transgenic mouse model. The test results showed that the RNA inhibitors Ky-19, Ky-26, Ky-37 and Ky-39 could reduce the expression level of HBsAg by 93.0% to 99.5% or more for at least 4 consecutive weeks in HBV transgenic mice.

In some embodiments, the RNA inhibitor Ky-2208 of the present invention can reduce the HBsAg level by 98.2 to 99.6% for about 140 days in AAV-HBV mice, and promote to produce surface antibody HBsAb *in vivo,* showing possibility of functionally curing hepatitis B.

### Brief Description of the Drawings

The following drawings are provided for making the objects, technical solution and beneficial effects of the present invention clearer:
Fig. 1 is a high-resolution mass spectrogram of ERCd-01-c2 synthesized in Section 1.1.5 of Example I;
Fig. 2 is a high-resolution mass spectrogram of 3'MVIP17-c1 synthesized in Section 1.2.6 of Example I;
Fig. 3 is a high-resolution mass spectrogram of 5'MVIP09-ERCd-PFP-c2 synthesized in Section 2.1.2 of Example I;
Fig. 4 is a histogram showing inhibitory effect of Ky-00 to Ky-26 on HBsAg level in HepG2.2.15 cell line in Example 1 of Example II;
Fig. 5 is a histogram showing inhibitory effect of Ky-27 to Ky-44 on HBsAg level in HepG2.2.15 cell line in Example 2 of Example II;
Fig. 6 is a histogram showing the influence of different X/L/D in the RNA inhibitor on the effect of reducing HBsAg level of HBV in Examples 3, 4 and 6 of Example II;
Fig. 7 is a histogram showing the influence of linker B in the RNA inhibitor on the effect of reducing HBsAg level of HBV in Example 5 of Example II;
Fig. 8 is a histogram showing the influence of different transition points R1/R2 in the RNA inhibitor on the effect of reducing HBsAg level of HBV in Examples 7 and 8 of Example II;
Fig. 9 is a histogram showing the inhibitory effect of Ky-22 combined with entecavir or interferon on HBsAg in HepG2.2.15 cells in Example 9 of Example II;
Fig. 10 is a histogram showing the inhibitory effect of Ky-22 combined with entecavir or interferon on HBeAg in HepG2.2.15 cells in Example 9 of Example II;
Fig. 11 is a histogram showing the inhibitory effect of Ky-22 combined with entecavir or interferon on HBV DNA in HepG2.2.15 cells in Example 9 of Example II;
Fig. 12 is a graph showing the inhibitory effect of Ky-22 on 4 different genotypes (A, B, C, D) of HBV cell lines in Example 10 of Example II;
Fig. 13 is a graph showing the inhibitory effect of the RNA inhibitor on HBsAg in the HBV transgenic mouse model in Example 1 of Example III;
Fig. 14 is a graph showing the influence of sequence adjustment of Ky-22 on the effect of inhibiting HBsAg in HBV transgenic mice in Example 2 of Example III;
Fig. 15 is a graph showing the results of investigating the dose-effect of Ky-2208 in AAV-HBV mouse model in Example 3 of Example III;
Fig. 16 is a histogram showing the effect of Ky-2208 on productiong of HBsAb in AAV-HBV mouse model in Example 3 of Example III; and
Fig. 17 is a graph showing the results of comparison and combination use of Ky-2208 and TDF on HBV-Tg mice in Example 4 of Example III.

### Detailed Description

The following examples illustrate some embodiments disclosed in the present invention, but the present invention is not limited thereto. In addition, when providing specific embodiments, the inventors anticipated application of some specific embodiments, for example, RNA inhibitors with specifically the same or similar chemical structures for treatment of different hepatogenic diseases.

### Explanations:

DMSO refers to dimethyl sulfoxide;
DMF refers to N,N-dimethylformamide;
HOBt refers to 1-hydroxybenzotriazole;
HBTU refers to O-benzotriazole-tetramethyluronium hexafluorophosphate;
DIPEA (DIEA) refers to N,N-diisopropylethylamine;
DCM refers to dichloromethane;
DMAP refers to 4-dimethylaminopyridine;
DMT-CL refers to 4,4'-dimethoxytriphenylchloromethane;
MEOH refers to methanol;
TBTU refers to O-benzotriazole-N,N,N',N'-tetramethyluronium tetrafluoroboric acid;
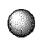 refers to a solid phase support, such as macroporous aminomethyl resin (Resin).

### Example I Synthesis of RNA Inhibitors Ky-19, Ky-22, Ky-2208, Ky-26, Ky-37 and Ky-39

The RNA inhibitor of the present invention was prepared by obtaining respective sense strands and antisense strands by solid-phase phosphoramidite method, and annealing complementarily the sense strand and the corresponding antisense strand to obtain the final product. The solid-phase phosphoramidite method includes the following basic steps: 1) deprotection: removing the protective group (DMTr) for the hydroxy on the initial monomer solid support; 2) coupling: adding a first phosphoramidite monomer, coupling in the direction of 3' to 5'; 3) oxidation: oxidizing the resulting nucleoside phosphite into a more stable nucleoside phosphate (that is, oxidization of trivalent phosphorus to pentavalent phosphorus); 4) blocking: blocking 5'-OH of the nucleotide monomer unreacted in the previous step by capping to prevent it from reacting further; the above steps were repeated until a last phosphoramidite was added. Then, the ester bond for linking the solid support and the initial monomer was cleaved with aqueous methylamine solution and aqueous ammonia, and protective groups on various bases and phosphoric acid on the oligonucleotide, including cyanoethyl (P), benzoyl (mA, fA), acetyl (mC), were removed. The resultant was purified by HPLC, filtered and sterilized, and freeze-dried to obtain the corresponding sense strand or antisense strand.

Annealing: The concentrations of the sense strand and antisense strand reconstitution solution were accurately measured, mixed in equimolar concentration, added with 1 M PBS solution by 1/20 of the volume and mixed again. The mixed system was heated to 95°C and kept for 5min, and then cooled down naturally for 3 hours to 40°C or room temperature, and performed HPLC detection. If the single-chain residue is less than 5%, the reaction is considered complete.

When there is 3'MVIP at the 3' end of the sense strand or antisense strand of the RNA inhibitor of the present invention, the 3'MVIP solid support is used as the initial monomer for solid-phase synthesis, and the 3'MVIP solid support has a general formula as follows: 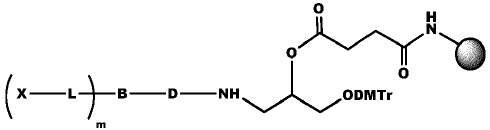 wherein, when m is 1 to 4, the linker B in the general formula is branched 1 to 4 times to obtain the corresponding 3'MVIP solid support.

When m is 1, the obtained solid support is used as the initial monomer for the solid-phase synthesis of the antisense strand of the RNA inhibitor Ky-26 and the sense strand of the RNA inhibitor Ky-39; when m is 2, the obtained solid support is used as the initial monomer for the solid-phase synthesis of the sense strand of the RNA inhibitor Ky-37 and the antisense strand of the RNA inhibitors Ky-22 and Ky-2208; and when m is 3, the obtained solid support is used as the initial monomer for the solid-phase synthesis of the antisense strand of the RNA inhibitor Ky-19.

When there is 5'MVIP at the 5' end of the sense strand or antisense strand of the RNA inhibitor of the present invention, the 5'MVIP phosphoramidite monomer is the last phosphoramidite monomer for the solid-phase synthesis of the sense strand or antisense strand. The 5'MVIP phosphoramidite monomer has a general formula as follows: 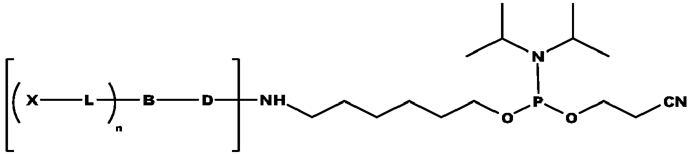 wherein, when n is 1 to 4, the linker B in the general formula is branched 1 to 4 times to obtain the corresponding 5'MVIP phosphoramidite monomer.

When n is 1, the resulting 5'MVIP phosphoramidite monomer is used as the last monomer for the solid-phase synthesis of the sense strands of the RNA inhibitors Ky-19, Ky-26 and Ky-37; when n is 2, the resulting 5'MVIP phosphoramidite monomer is used as the last monomer for the solid-phase synthesis of the sense strands of Ky-39, Ky-22 and Ky-2208.

Before the phosphoramidite solid-phase synthesis of the sense and antisense strands of the RNA inhibitors of the present invention, the corresponding 3'MVIP solid support and 5'MVIP phosphoramidite monomers need to be chemically synthesized firstly. The chemical synthesis process is described as follows:

### 1. Synthesis of 3'MVIP solid support

1.1 Synthesis of 3'MVIP09 solid support for the sense strand of the RNA inhibitor Ky-37 and the antisense strand of the RNA inhibitors Ky-22 and Ky-2208

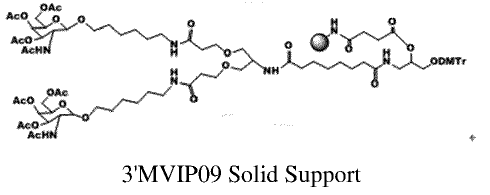

### Description of the synthesis process:

### 1.1.1. Synthesis of ERC-01-c1

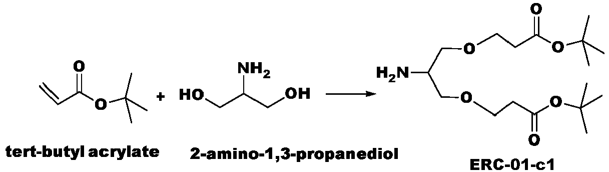

2-amino-1,3-propanediol (5.0g, 54.9mmol) was weighed and added with DMSO (50mL) and a solution of sodium hydroxide (1g/mL, 5mL), cooled down to 0°C, added dropwise with tert-butyl acrylate (20mL, 137.8mol) over 2 hours, and reacted at room temperature for 48 hours. The mixture was added with petroleum ether (100 mL). The organic phase was washed twice with saturated brine, dried and passed over a chromatography column (eluent: ethyl acetate:petroleum ether=25%-75%, containing 0.05% triethylamine) to get 6.2 g of a colorless oil.

### 1.1.2. Synthesis of ERC-01-c2

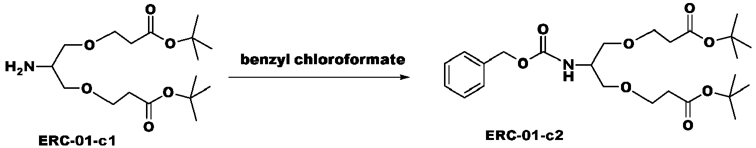

ERC-01-c1 (6.2g, 17.9mmol) was weighed, added with dichloromethane (50mL) and a solution of sodium carbonate (25%, 23mL), followed by dropwise addition of benzyl chloroformate (8.2mL, 57.4mmol) at room temperature over 2 hours. The mixture was reacted overnight at room temperature, washed with saturated brine three times, dried over anhydrous sodium sulfate, evaporated off the solvent. The residue was pass over a chromatography column (ethyl acetate:petroleum ether=5%-30%) to get 4.0 g of an oil.

### 1.1.3. Synthesis of ERC-01-c3

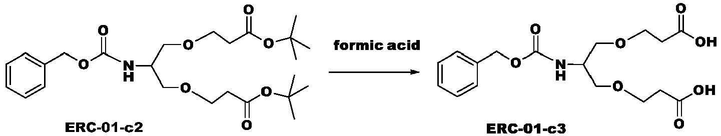

ERC-01-c2 (4.0g, 8.3mmol) was added with formic acid (12mL), reacted overnight at room temperature, and evaporated off the solvent under reduced pressure to get 2.8g of the product.

### 1.1.4. Synthesis of ERCd-01-c1

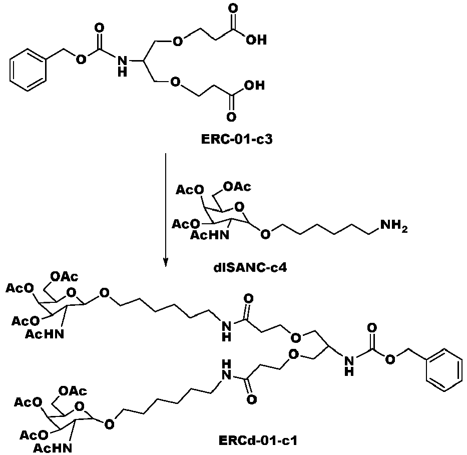

ERC-01-c3 (1.11g, 3.0mmol) and dlSANC-c4 (3.6g, 8.04mmol) were added into DMF (60 mL), added with HOBt (2.24g) and HBTU (3.36g), followed by slow addition of DIEA (4.16 mL). The reaction solution was stirred to react at room temperature for 3 hours. Water was then added and the aqueous layer was extracted with dichloromethane (2 × 10 mL). The combined organic layer was washed successively with saturated sodium bicarbonate (80 mL), water (2 × 60 mL) and saturated brine (60 mL), dried over anhydrous sodium sulfate, and evaporated to dryness under reduced pressure. The residue was purified by silica gel column chromatography (eluent: 3-15% MeOH in DCM) to get 3.24 g of a light yellow solid.

### 1.1.5. Synthesis of ERCd-01-c2

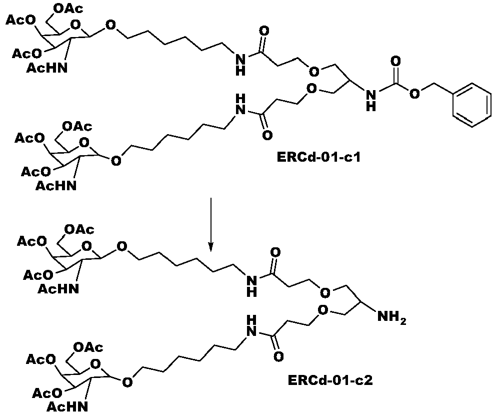

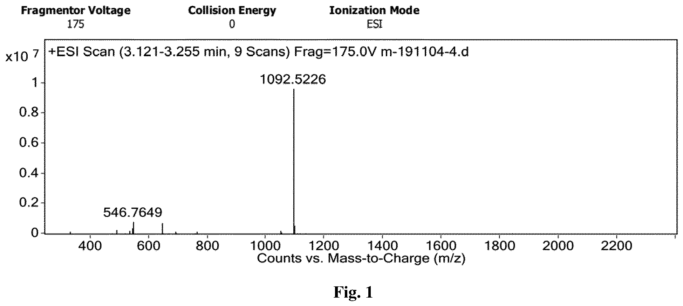

ERCd-01-c1 (3.24g, 2.6mmol) was dissolved in methanol (60mL), added with 10% Pd-C (0.3g) and acetic acid (2.0mL), and hydrogenated under normal pressure overnight. The reaction solution was filtered with diatomite, and the filtrate was evaporated to dryness under reduced pressure to get 2.9 g of ERCd-01-c2 of an oil, whose high-resolution mass spectrogram was shown in Fig. 1.

### 1.1.6. Synthesis of 3'MVIP09-c1

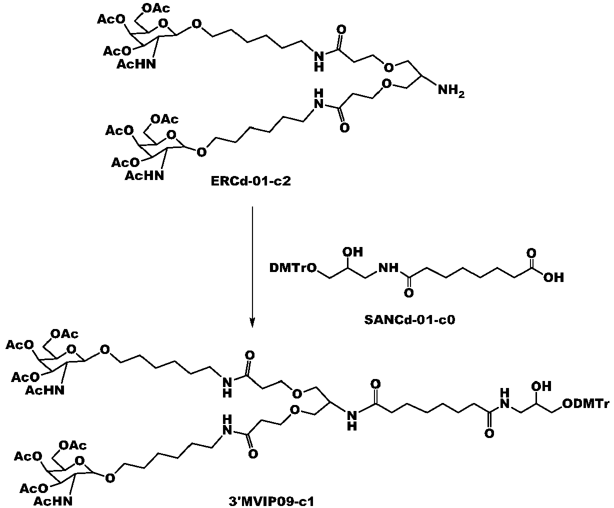

SANCd-01-c0 (0.824g, 1.5mmol) and ERCd-01-c2 (1.09g, 1.0mmol) were added in turn into a reaction flask, added with DCM (10mL) and stirred to be dissolved, and then added with TBTU (0.963g) and DIPEA (0.517g) in turn, and reacted overnight. The reaction mixture was added with water and extracted with DCM. The organic phase was washed with saturated brine, dried, filtered, concentrated, and finally purified through a silica gel column to get 1.3g of the product.

### 1.1.7. Synthesis of 3'MVIP09-c2

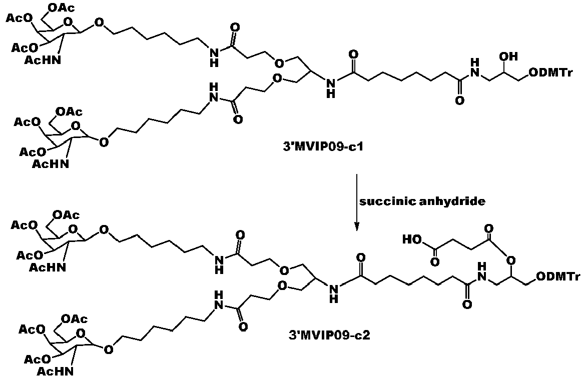

3'MVIP09-c1 (1.62g, 1 µmol) and DCM (10mL) were added in turn into a reaction flask, stirred at room temperature to be dissolved, added with DMAP (0.366g) and succinic anhydride (0.2g, 3 µmol) in turn, and stirred at room temperature to react. TLC showed the reaction is complete. The reaction mixture was concentrated to remove DCM, added with water and extracted with DCM. The organic phase was washed with saturated brine, dried over anhydrous sodium sulfate, filtered, concentrated, and finally purified through a silica gel column to get 1.55g of the product.

### 1.1.8. Synthesis of 3'MVIP09 Solid Support

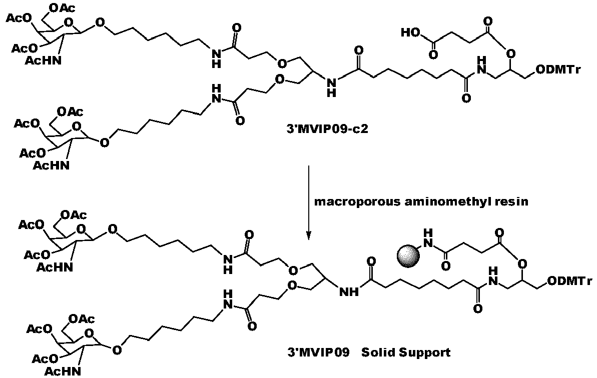

3'MVIP09-c2 (0.86g, 0.5 µmol) and DMF (10mL) were added in turn into a reaction flask, stirred to be dissolved, added with HBTU (0.19g), DIPEA (0.194g) and macroporous aminomethyl resin (2.0g) in turn, shaked for 24 hours and filtered. The resin was washed with 10% methanol/DCM, and then capped with 25% acetic acid/pyridine. The degree of substitution was 150 µmol/g.

1.2 Synthesis of 3'MVIP17 solid support for the antisense strand of the RNA inhibitor Ky-19

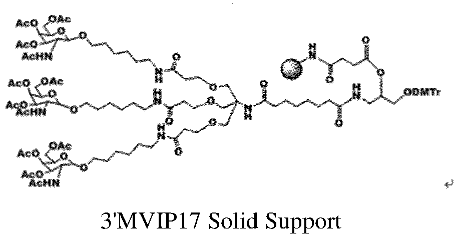

### 1.2.1. Synthesis of SANC-01-c1

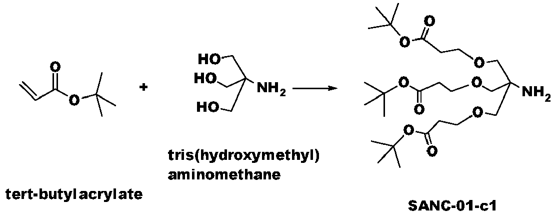

The synthesis steps referred to the synthesis of ERC-01-c1 in Section 1.1.1 of Example I.

### 1.2.2. Synthesis of SANC-01-c2

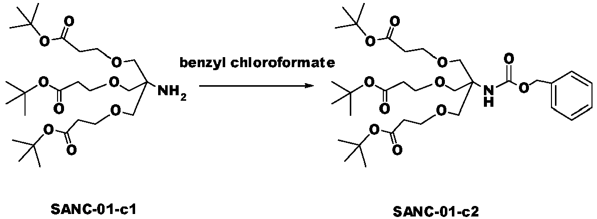

The synthesis steps referred to the synthesis of ERC-01-c2 in Section 1.1.2 of Example I.

### 1.2.3. Synthesis of SANC-01-c3

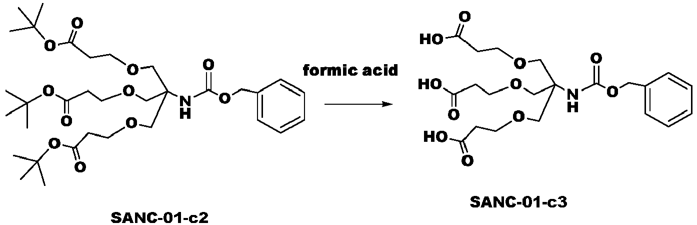

The synthesis steps referred to the synthesis of ERC-01-c3 in Section 1.1.3 of Example I.

### 1.2.4. Synthesis of SANCd-01-c1

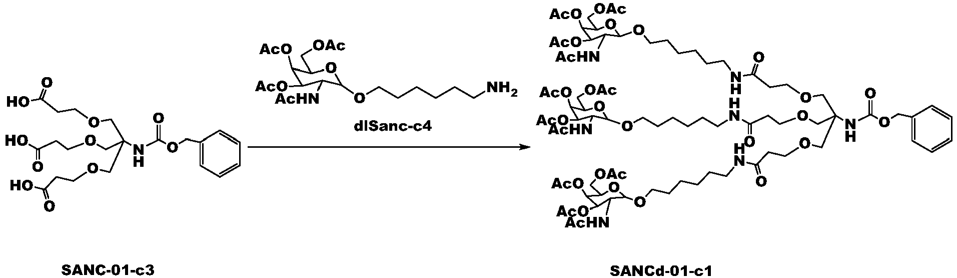

The synthesis steps referred to the synthesis of ERCd-01-c1 in Section 1.1.4 of Example I.

### 1.2.5. Synthesis of SANCd-01-c2

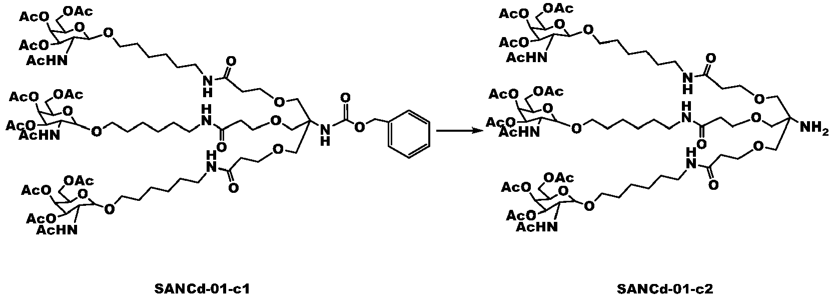

The synthesis steps referred to the synthesis of ERCd-01-c2 in Section 1.1.5 of Example I.

### 1.2.6. Synthesis of 3'MVIP17-c1

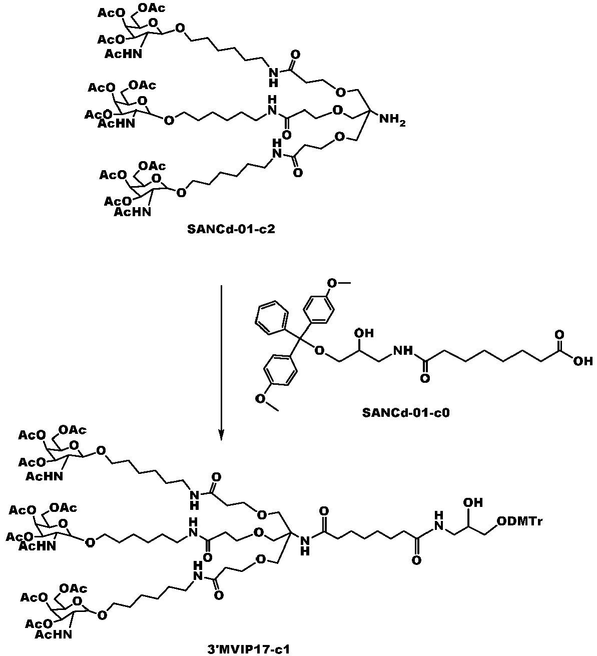

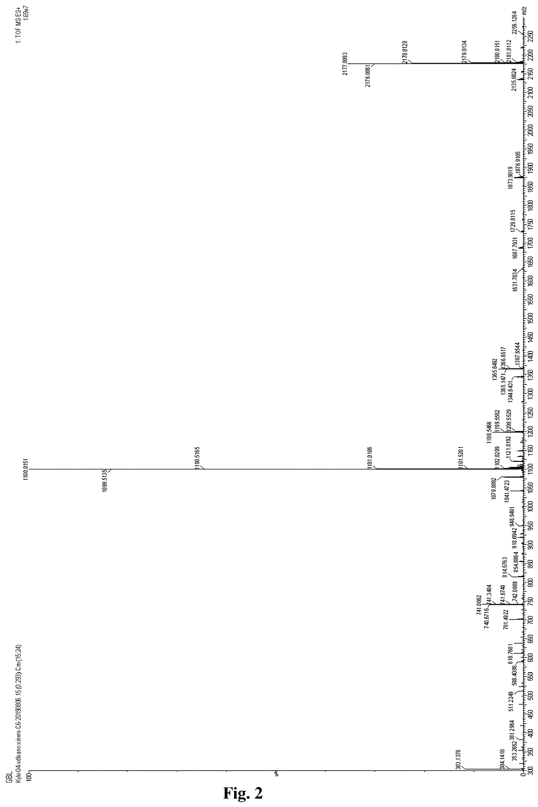

The synthesis steps referred to the synthesis of 3'MVIP09-c1 in Section 1.1.6 of Example I, and the high-resolution mass spectrogram of the synthesized 3'MVIP17-c1 was shown in Fig. 2.

### 1.2.7. Synthesis of 3'MVIP17-c2

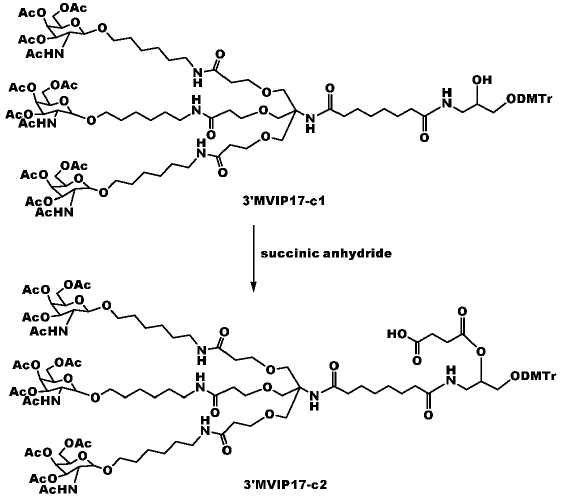

The synthesis steps referred to the synthesis of 3'MVIP09-c2 in Section 1.1.7 of Example I.

### 1.2.8. Synthesis of 3'MVIP17 Solid Support

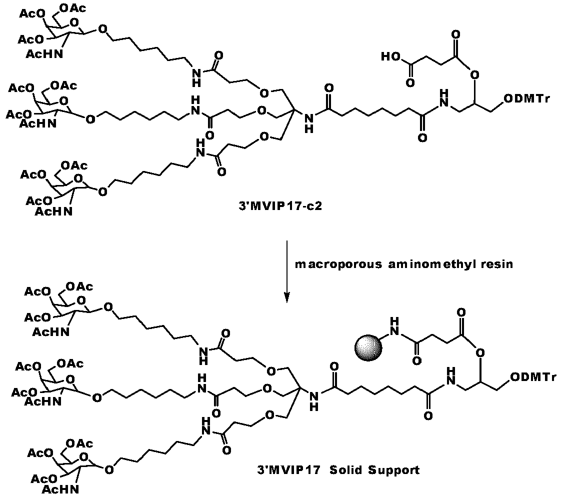

The synthesis steps referred to the synthesis of 3'MVIP09 Solid Support in Section 1.1.8 of Example I.

1.3 Synthesis of 3'MVIP01 Solid Support of the antisense strand of the RNA inhibitor Ky-26 and the sense strand of the RNA inhibitor Ky-39:

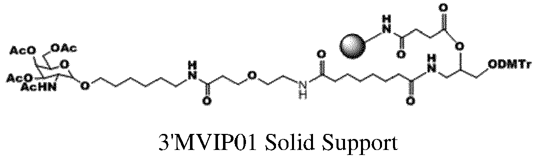

### Description of the synthesis process:

### 1.3.1. Synthesis of 3'MVIP01-c1

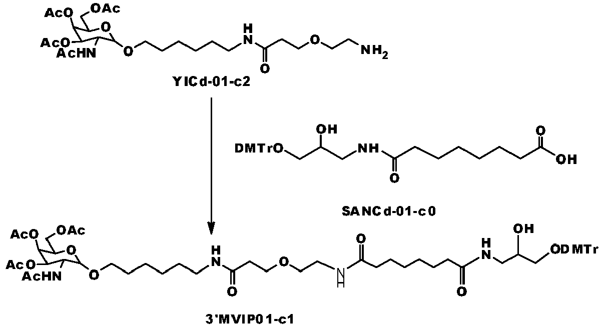

The synthesis steps referred to the synthesis of 3'MVIP09-c1 in Section 1.1.6 of Example I.

### 1.3.2. Synthesis of 3'MVIP01-c2

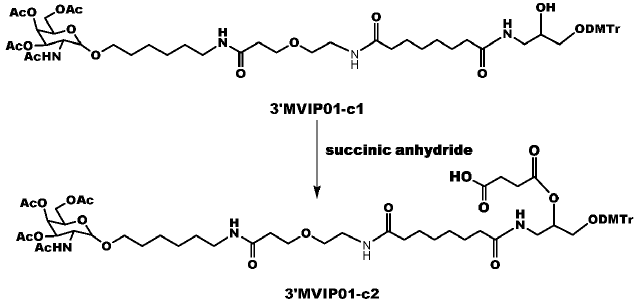

The synthesis steps referred to the synthesis of 3'MVIP09-c2 in Section 1.1.7 of Example I.

### 1.3.3. Synthesis of 3'MVIP01 Solid Support

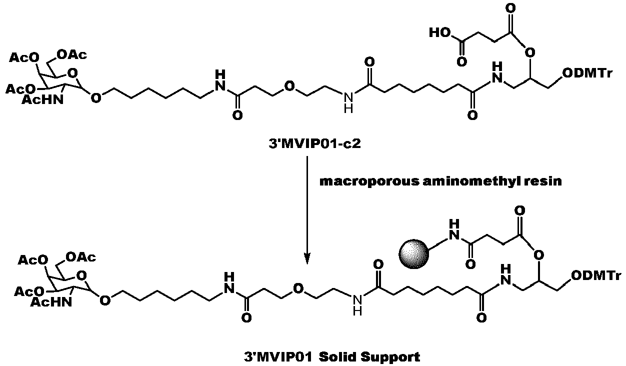

The synthesis steps referred to the synthesis of 3'MVIP09 Solid Support in Section 1.1.8 of Example I.

### 2. Synthesis of 5'MVIP phosphoramidite monomer

2.1 When n is 2, the obtained 5'MVIP phosphoramidite monomer is used as the last monomer for solid-phase synthesis of the sense strand of Ky-22, Ky-2208 and Ky-39.

### Synthesis of 5'MVIP09 phosphoramidite monomer:

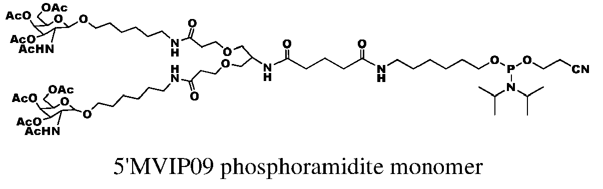

### 2.1.1. Synthesis of 5'MVIP09-ERCd-PFP-c1

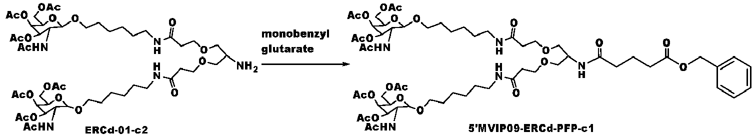

ERCd-01-c2 (2.18g, 2.0mmol) was weighed and dissolved in DMF (50mL), added with monobenzyl glutarate (0.53g, 2.4mmol), DIPEA (0.78g) and TBTU (0.84g), and stirred at room temperature overnight. The reaction mixture was quenched with water (50mL), and extracted with DCM (30mL*3). The organic phase was washed with 10% citric acid (50mL*3), saturated sodium bicarbonate (50mL) and pyridine (100mL), dried over anhydrous sodium sulfate, filtered, rotary evaporated, and purified through a column to get the product 5'MVIP09-ERCd-PFP-c1 (2.15g).

### 2.1.2. Synthesis of 5'MVIP09-ERCd-PFP-c2

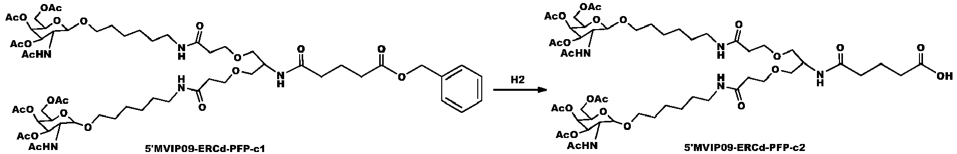

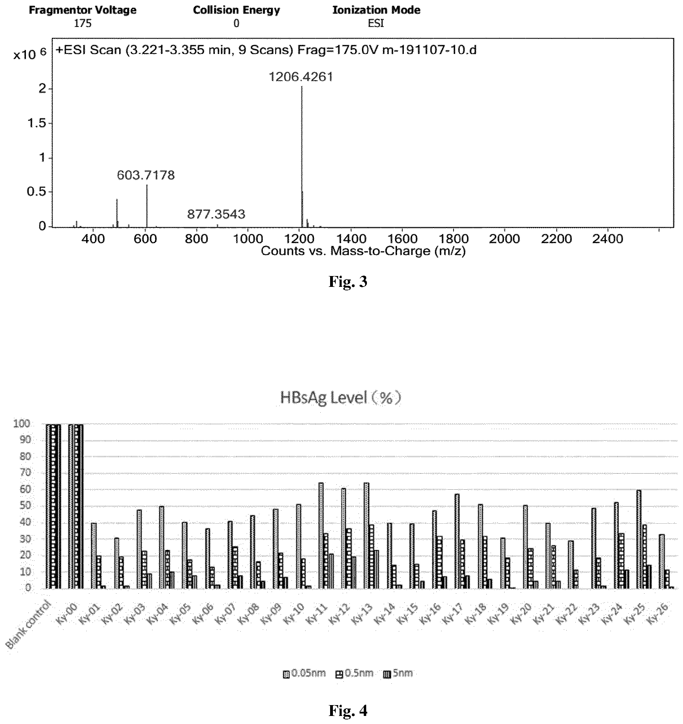

5'MVIP09-ERCd-PFP-c1 (2.15g, 1.66mmol) and 10% Pd-C (0.21g) were weighed, added with methanol (50mL), and hydrogenated under stirring at room temperature overnight. After the reaction was completed, the reaction mixture was filtered with diatomite to remove Pd-C, and the filtrate was rotary evaporated to get a crude 5'MVIP09-ERCd-PFP-c2 (1.9 g), whose high-resolution mass spectrogram was shown in Fig. 3.

### 2.1.3. Synthesis of 5'MVIP09-ERCd-PFP

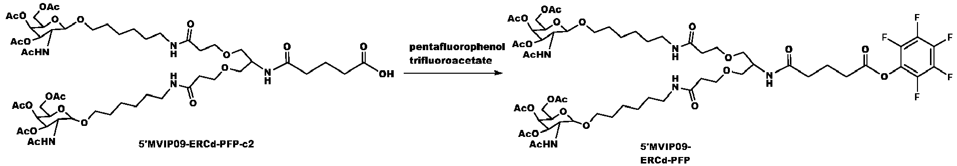

The crude 5'MVIP09-ERCd-PFP-c2 (1.9g, 1.58mmol) was weighed and dissolved in DCM (60mL), added with DIPEA (1.33g), cooled, and added with pentafluorophenol trifluoroacetate (2.21g, 7.9mmol). The mixture was reacted under stirring at room temperature for 2 hours, and then rotary evaporated. The residue was dissolved in DCM (60mL), and washed with saturated sodium bicarbonate (30mL*3), 10% citric acid (30mL*1) and saturated brine (50mL*1). The organic phase was dried over anhydrous sodium sulfate, filtered, and rotary evaporated to get a crude 5'MVIP09-ERCd-PFP (2.35g), which was sucked to dryness and directly used in the next reaction without purification.

### 2.1.4. Synthesis of 5'MVIP09 phosphoramidite monomer-c1

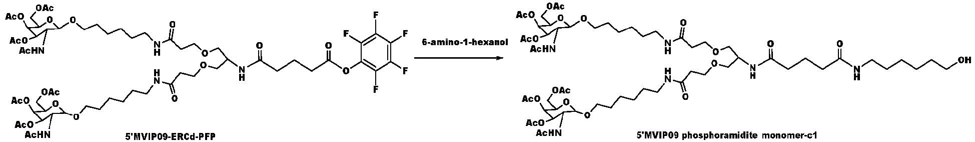

The crude 5'MVIP09-ERCd-PFP (2.35g, 1.58mmol) was dissolved in DCM (60mL), added with DIPEA (0.82g, 6.32mmol) and 6-amino-1-hexanol (0.37g, 3.16mmol), and reacted under stirring at room temperature overnight. The reaction mixture was added with 10% citric acid (30mL), and extracted with DCM (30mL*3). The organic phase was wash with saturated brine (50mL), dried over anhydrous sodium sulfate, filtered, and rotary evaporated. The residue was purified through a column to get the product 5'MVIP09 monomer-c1 (1.73 g).

### 2.1.5. 5'MVIP09 phosphoramidite monomer

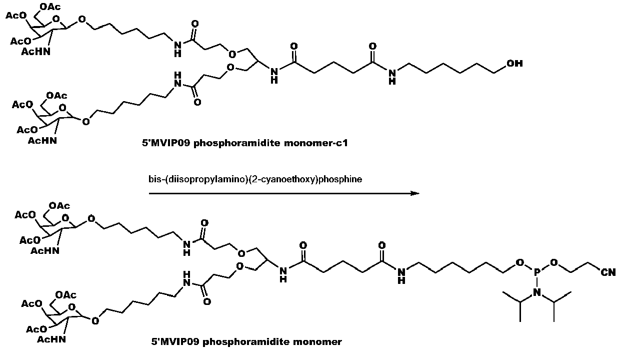

The 5'MVIP09 phosphoramidite monomer-c1 (1.3g, 1.0mmol) was weighed and dissolved in acetonitrile (30mL), followed by addtion of diisopropylamine triazole (0.22g). The mixture was added with bis-(diisopropylamino)(2-cyanoethoxy)phosphine (0.36g, 1.2mmol) under an ice bath, and reacted at room temperature for 4h. HPLC showed the reaction was complete. The reaction mixture was concentrated and purified through a column to get the product 5'MVIP09 monomer (1.2g).

2.2 When n is 1, the obtained 5'MVIP phosphoramidite monomer is used as the last monomer for solid-phase synthesis of the sense strand of Ky-19, Ky-26 and Ky-37.

### Synthesis of 5'MVIP01 phosphoramidite monomer:

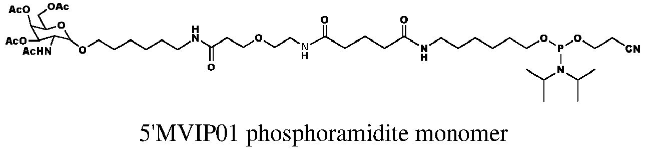

Except YICd-01-c2 (1.12g, 2.0mmol) was weighed for the 5'MVIP01 phosphoramidite monomer, the remaining operations referred to Sections 2.1.1 to 2.1.5.

### Example II: in vitro test

### Example 1: HepG2.2.15 cell line was used to evaluate the effect of reducing the HBsAg level of HBV by RNA inhibitors obtained by coupling 5'MVIP and 3'MVIP to different ends of the sense and antisense strands.

Experimental procedures: The respective RNA inhibitors Ky-00~Ky-26 were prepared according to the method described in Example I, and DMEM medium containing 10% fetal bovine serum was prepared. Media containing 0.05, 0.5, 5 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the above-mentioned RNA inhibitor samples at different concentrations for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

The experimental data obtained were shown in Fig. 4. As shown in Fig. 4, Ky-19, Ky-22 and Ky-26 showed better inhibitory effects on HBsAg than other compounds.

### Example 2: HepG2.2.15 cell line was used to evaluate the effect of reducing the HBsAg level of HBV by RNA inhibitors obtained by placing 5'MVIP and 3'MVIP on both ends of the sense strand or antisense strand of the RNA inhibitors or placing 5'MVIP or 3'MVIP simultaneously on the same end, such as the 3' end or the 5' end of the antisense and sense strand.

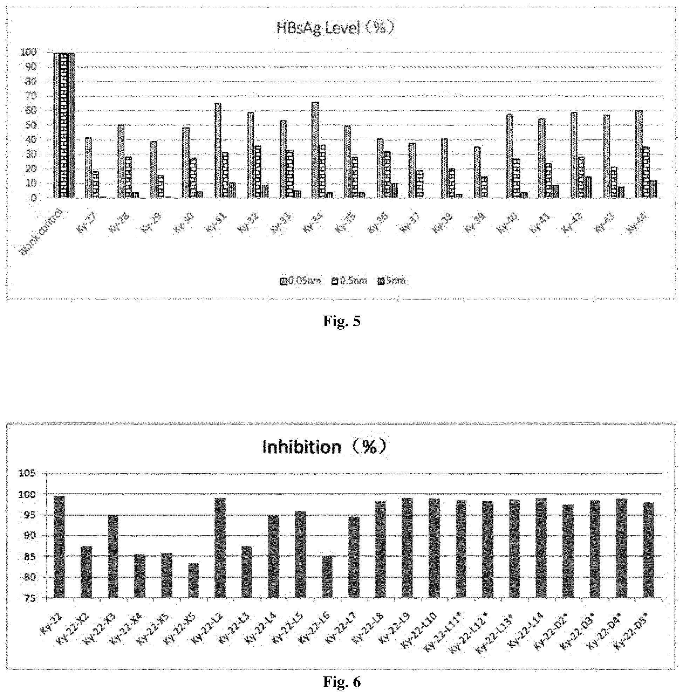

Experimental procedures: The respective RNA inhibitors Ky-27~Ky-44 were prepared according to the method described in Example I, and DMEM medium containing 10% fetal bovine serum was prepared. Media containing 0.05, 0.5, 5 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the above-mentioned RNA inhibitor samples at different concentrations for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention. The experimental data obtained were shown in Fig. 5.

As shown in Fig. 5, Ky-37 and Ky-39 showed better inhibitory effects on HBsAg than other compounds.

### Example 3 HepG2.2.15 cell line was used to evaluate the influence of different liver targeting specific ligands X on the effect of reducing the HBsAg level of HBV by RNA inhibitor.

The influence of different liver targeting specific ligands X on the effect of reducing the HBsAg level of HBV by RNA inhibitor was investigated. The obtained RNA inhibitors Ky-22 and Ky-22-X2 ~ Ky-22-X6 had the same L, B, D and R₁ /R₂ as those in the combination of 5'MVIP09/3'MVIP09, except that the structure of X was changed.

In the RNA inhibitors involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: The respective RNA inhibitors were prepared according to the method described in Example I, and DMEM medium containing 10% fetal bovine serum was prepared. Media containing 10 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

The experimental data obtained were shown in Fig. 6. The results showed that when X is galactose, galactosamine, N-acetylgalactosamine and a derivative thereof, the obtained RNA inhibitors preferably have N-acetylgalactosamine and a derivative thereof as the ligand.

### Example 4 HepG2.2.15 cell line was used to evaluate the influence of different branched chains L on the effect of reducing the HBsAg level of HBV by RNA inhibitor.

The influence of different branched chains L on the effect of reducing the HBsAg level of HBV by RNA inhibitor was investigated. The obtained RNA inhibitors Ky-22, Ky-22-L2~Ky-22-L14 had the same X, B, D and R₁ /R₂ as those in the combination of 5'MVIP09/3'MVIP09, except that the structure of L was changed.

In the RNA inhibitors involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: The respective RNA inhibitors were prepared according to the method described in Example I, and DMEM medium containing 10% fetal bovine serum was prepared. Media containing 10 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

The experimental data obtained were shown in Fig. 6. The results showed that the length of L had a large influence on the effect of RNA inhibitors, and the L chain should not be too short or too long; and there was not much difference in the effect of reducing the HBsAg level of HBV by the obtained RNA inhibitor, when -NH-, C=O, O, S, amide group, phosphoryl, thiophosphoryl, aliphatic carbocyclyl such as cyclohexane or a combination thereof was contained, or Ls in the structure of the same 5'MVIP or 3'MVIP or between 5'MVIP and 3'MVIP were different from each other, and the chain length was in the range of C7-C18.

### Example 5 HepG2.2.15 cell line was used to evaluate the influence of the linker B on the effect of reducing the HBsAg level of HBV by RNA inhibitor.

The influence of different linkers B on the effect of reducing the HBsAg level of HBV by RNA inhibitor was investigated. The obtained RNA inhibitors Ky-22, Ky-22-B2 ~ Ky-22-B7, Ky-19, Ky-19-B2 ~ Ky-19-B12, Ky-26, Ky-26-B2 ~ Ky-26-B7, Ky-37, Ky-37-B2 ~ Ky-37-B6, Ky-39, Ky-39-B2 ~ Ky-39-B6 had the same X, L, D and R₁ /R₂ as those in the combination of 5'MVIP09/3'MVIP09, except that the structure of B was changed.

In the RNA inhibitors involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: The respective RNA inhibitors were prepared according to the method described in Example I, and DMEM medium containing 10% fetal bovine serum was prepared. Media containing 10 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

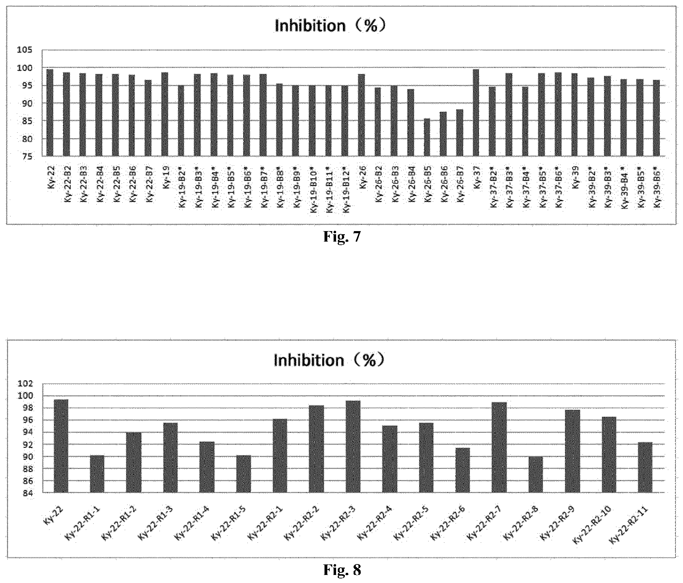

The experimental data obtained were shown in Fig. 7. The results showed that there was not much difference in the effect of reducing the HBsAg level of HBV, when except that the structure of B was changed, X, L, D and R₁ /R₂ were the same as those in the combination 5'MVIP09/3'MVIP09, A₁ and A₂ in the general formula of the linker B are each independently C, O, S, -NH-, carbonyl, amide group, phosphoryl or thiophosphoryl, r is an integer of 0 to 4, and the linkers B between 5'MVIP and 3'MVIP are the same or different.

### Example 6 HepG2.2.15 cell line was used to evaluate the influence of the linking chain D on the effect of reducing the HBsAg level of HBV by RNA inhibitor.

The influence of different linking chains D on the effect of reducing the HBsAg level of HBV by RNA inhibitor was investigated. The obtained RNA inhibitors Ky-22, Ky-22-D2 ~ Ky-22-D5 had the same X, L, B and R₁ /R₂ as those in the combination of 5'MVIP09/3'MVIP09, except that the structure of D was changed.

In the RNA inhibitors involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: The respective RNA inhibitors were prepared according to the method described in Example I, and DMEM medium containing 10% fetal bovine serum was prepared. Media containing 10 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

The experimental data obtained were shown in Fig. 6. The results showed that, in the case of the same MVIP structure and RNA inhibitors, different linking chains D had influence on the inhibitory effect of RNA inhibitors on HBsAg, and the effects of D1, D2, and D4 were close to each other and better than those of D3.

### Example 7: HepG2.2.15 cell line was used to evaluate the influence of different R₁ on the effect of reducing the HBsAg level of HBV by RNA inhibitor.

The influence of different transition points R₁ on the effect of reducing the HBsAg level of HBV by RNA inhibitor was investigated. The obtained RNA inhibitors Ky-22, Ky-22-R1-1~ Ky-22-R1-5 had the same X, L, B, D and R₂ as those in the most preferred MVIP combination of 5'MVIP09/3'MVIP09, except that the structure of R₁ was changed.

In the RNA inhibitors involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: The respective RNA inhibitors were prepared according to the method described in Example I, and DMEM medium containing 10% fetal bovine serum was prepared. Media containing 10 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

The experimental data obtained were shown in Fig. 8. The results showed that different transition points R₁ had influence on the inhibitory effect of RNA inhibitors on HBsAg, and the RNA inhibitor with R1-1 as the transition point had the best effect of reducing the HBsAg level.

### Example 8: HepG2.2.15 cell line was used to evaluate the influence of different R₂ on the effect of reducing the HBsAg level of HBV by RNA inhibitor.

The influence of different transition points R₂ on the effect of reducing the HBsAg level of HBV by RNA inhibitor was investigated. The obtained RNA inhibitors Ky-22, Ky-22-R2-1 ~ Ky-22-R2-11 had the same X, L, B, D and R₁ as those in the most preferred MVIP combination of 5'MVIP09/3'MVIP09, except that the structure of R₂ was changed. The respective RNA inhibitors were prepared according to the method described in Example I.

In the RNA inhibitors involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: The respective RNA inhibitors were prepared according to the method described in Example I, and DMEM medium containing 10% fetal bovine serum was prepared. Media containing 10 nM RNA inhibitor samples were prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled and detected with a HBsAg detection kit (Shanghai Kehua, ELISA method). The relative percentage of HBsAg in the sample intervention groups was calibrated by compared with the supernatant of HepG2.2.15 cells without intervention.

The experimental data obtained were shown in Fig. 8. The results showed that different transition points R₂ had influence on the inhibitory effect of RNA inhibitors on HBsAg, and the RNA inhibitor with R2-1 as the transition point had the best effect of reducing the HBsAg level.

### Example 9 Ky-22 was used in combination with entecavir or interferon, the first-line drugs currently used in the treatment of chronic hepatitis B, to investigate whether there was mutual interference in the inhibitory effect on HBV.

The experiment was conducted in the widely used HepG2.2.15 cell line to evaluate the inhibitory effect of the RNA inhibitor of the present invention in combination with different concentrations of entecavir (ETV) or interferon (IFN-a) on HBV.

In the RNA inhibitor Ky-22 involved in the experiment, the sense strand was SEQ ID NO. 2, the antisense strand was SEQ ID NO. 59, the 5' end of the sense strand was coupled with 5'MVIP, and the 3' end of the antisense strand was coupled with 3'MVIP.

Experimental procedures: DMEM medium containing 10% fetal bovine serum was prepared. Media containing 10 nM RNA inhibitor Ky-22 was prepared from a culture medium. HepG2.2.15 cells were inoculated at a cell density of 10⁵ and cultured in the DMEM medium with 10% fetal bovine serum at 37°C under 5% CO₂ for 24 hours, added with the drug for intervention, and incubated for 72 hours. The supernatant was sampled to detect HBsAg, HBeAg and HBV DNA. The relative percentages of HBsAg, HBeAg, and HBV DNA in the sample intervention groups were calibrated by compared with the supernatant of HepG2.2.15 cells without intervention.

### Dosage concentrations:

ETV: 10µM, 1µM, 0.1µM;
IFN-a: 1000IU/mL, 100IU/mL, 10IU/mL;
Ky-22: 0.125 µg/mL;
ETV+Ky-22: 10µM+0.125µg/mL, 1µM+0.125µg/mL, 0.1µM+0.125µg/mL;
IFN-a +Ky-22: 1000IU/mL+0.125µg/mL, 100IU/mL+0.125µg/mL, 10IU/mL+0.125µg/mL

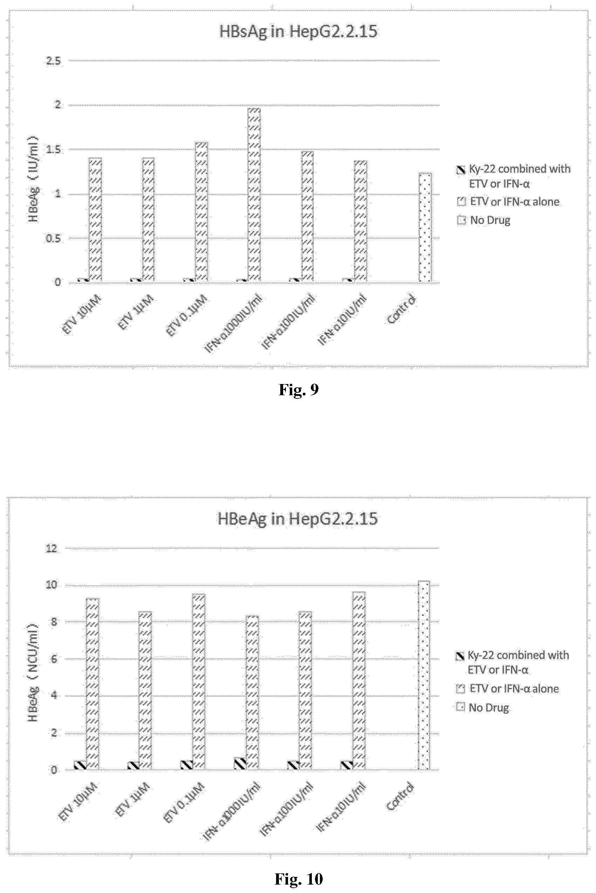

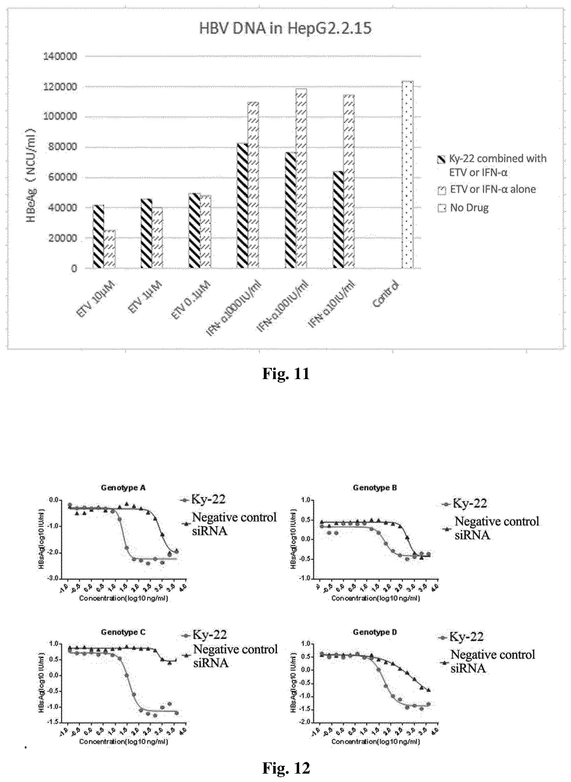

The effects of the RNA inhibitor Ky-22 on the levels of HBsAg and HBeAg in HepG2.2.15 cells were shown in Figs. 9 and 10, respectively. The results showed that entecavir or interferon alone had no significant inhibitory effect on HBsAg and HBeAg, but entecavir or interferon combined with Ky-22 showed a significant inhibitory effect on HBsAg and HBeAg, and the inhibition degree did not correlated to the concentration of entecavir or interferon. Entecavir or interferon did not affect the effect of the RNA inhibitor of the present invention on HBsAg and HBeAg; the RNA inhibitor Ky-22 combined with entecavir or interferon did not affect the inhibitory effect of entecavir or interferon on HBV DNA, even strengthen the effect of interferon on HBV DNA. The data results were shown in Fig. 11. The RNA inhibitor of the present invention can be used in combination with entecavir and interferon.

### Example 10 Study on the inhibitory effect of Ky-22 on 4 different genotypes (A, B, C, D) of HBV cell lines

### Experimental procedures:

Cell line construction: Based on HepG2 cells, the HBV gene was integrated by the transposon system of sleeping beauty. Cell culture conditions: DMEM + 10% FBS, 37 °C, 5% CO_{2.}. The HBV 1.3 ploidy genes of 4 different genotypes (A, B, C, D) were connected to the PT2/HB vectors through Gibson Assembly^{®} Master Mix, and a red fluorescent protein and puromycin resistance gene were connected at the same time as markers for cell strain screening. The constructed plasmids were co-transfected with pCMV(CAT) T7-SB100 into HepG2 cells using the X-tremeGENE HP DNA Transfection Reagent. The transfection method was as follows: A transfection system of a 10cm culture dish for the cell transfection was prepared according to the instructions, and kept still for 20 minutes. The HepG2 cells with a confluence of 70% were digested into a cell suspension, added with the prepared transfection system, mixed evenly, and placed in an incubator for cultivation. 48 hours after transfection, the cells was screened using 2 µg /mL puromycin resistance, and cells that did not express puromycin resistance, that is, cells that did not integrate with HBV, died. Cells that integrate with HBV were amplified, and cells with high red fluorescence intensity, that is, cells with high copy number of HBV integration were sorted out by flow cytometry, to get the 4 different genotypes of HBV stably integrated cell lines.

The HBV stably integrated cells of 4 genotypes A, B, C and D in the logarithmic growth phase were digested into a cell suspension, added to a 48-well plate (300 µL/well) with about 300,000 cells per well. After the confluence of the cells reached 70% (about 24 hours after plating), the following concentrations of Ky-22 or negative control siRNA (sense strand: SEQ ID NO. 146, antisense strand: SEQ ID NO. 147) was added: 4.1 µg/mL, 2.2 µg/mL, 1.1 µg/mL, 0.6 µg/mL, 0.3 µg /mL, 0.15 µg/mL, 0.0725 µg/mL, 0.03625 µg/mL, 0.018125 µg/mL, 0.0090625 µg/mL, 0.00453125 µg/mL, 0.00226563 µg/mL, 0.00113281 µg/mL, 0.000566406 µg/mL, 0.000283203 µg /mL, or no drug was added. The supernatants were collected at 24h, 48h, and 72h respectively and stored at -20°C, and replaced with fresh medium without the drug. The content of HBsAg in the cell supernatant was detected.

The experimental data were shown in Fig. 12. The results showed that, compared with the negative control siRNA treatment group (Control), Ky-22 had significant inhibitory effects on A, B, C and D genotypes of HBV, with EC50 (ng/mL) being 22.72, 25.45, 29.06 and 23.35, respectively.

### Example III in vivo efficacy studies

### Example 1: Investigation on the effect of RNA inhibitor on reducing HBsAg in HBV transgenic mouse model

The respective RNA inhibitors Ky-08, Ky-10, Ky-13, Ky-19, Ky-21, Ky-22, Ky-23, Ky-26, Ky-27, Ky-29, Ky-37 and Ky-39 were prepared according to the method described in Example I. 65 male HBV transgenic mice with a body weight of 25-35g and a week age of 8-10w were selected and raised in an animal room that meets the SPF standard at a temperature of 16-26 °C with a humidity of 40-70% and circulating light (12 hours in light and dark respectively), and were free to eat and drink water.

Animals were detected for HBV HBsAg before grouping, and randomly grouped according to the expression level of HBV HBsAg, and the average level of HBV HBsAg in various groups was kept as consistent as possible. The mice were divided into 13 groups with 5 mice in each group, including the control group (normal saline) and the administration groups 1 to 12. The administration dose was 3mg/kg with single administration, and the day of administration was set as d0. Mice in each group were administered the corresponding test solution by subcutaneous injecton at 0.04mL/10g on d0. The animals were observed for 4 to 6 weeks, and the blood was collected on d0, d7, d14, d21, d28, d35 and d42. At each blood collection time for each group, whole blood was collected through the orbital venous plexus of mice, and centrifuged at 3000×g for 5 min, and the supernatant was sampled to detect the expression level of HBV HBsAg.

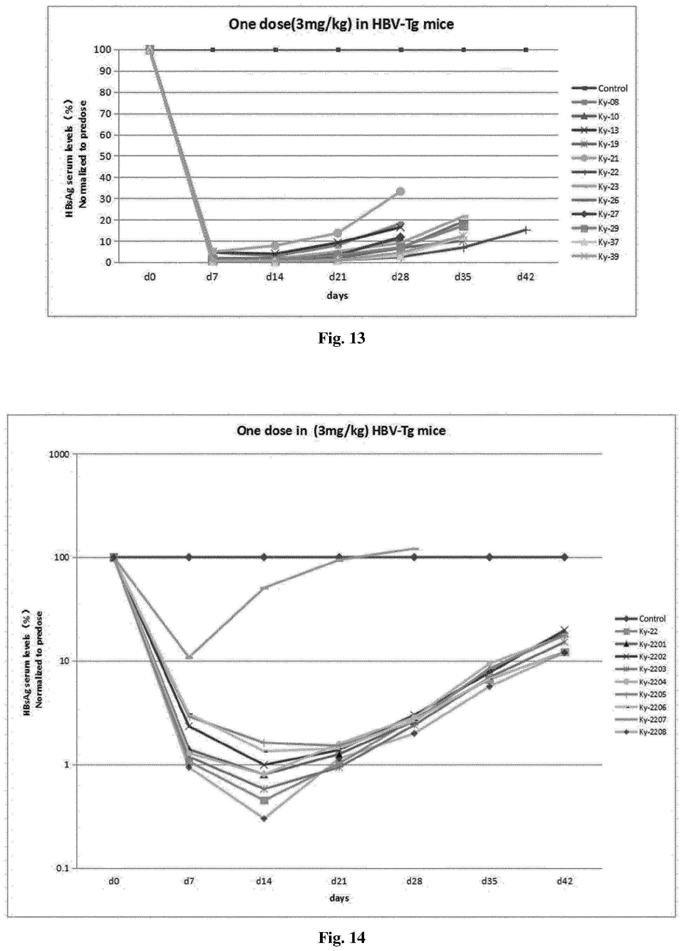

HBsAg levels of the animals in each administration group were normalized to those before administration and the control group, and the experimental data were shown in FIG. 13.

The research results showed that the RNA inhibitors of the present invention showed significant effects of reducing the HBV HBsAg level in the first three weeks, and the best reduction rate can reach 99.8%. Due to the different coupling positions with 5'MVIP and/or 3'MVIP, the respective RNA inhibitors were inconsistent in the duration of the effect of reducing HBsAg, wherein Ky-19, Ky-22, Ky-26, Ky-29, Ky-37 and Ky-39 still maintained the effect of reducing HBV HBsAg level by 93% or more on d28, and Ky-22 had the best lasting effect and maintained the effect of reducing HBV HBsAg level by 91% or more even on d35.

### Example 2: Investigation of the influence of sequence adjustment of Ky-22 on the effect of inhibiting HBsAg in HBV transgenic mice

The respective RNA inhibitors Ky-22, Ky-2201~Ky-2208 were prepared according to the method described in Example I. 50 male HBV transgenic mice with a body weight of 25-35g and a week age of 8-13w were selected and raised in an animal room that meets the SPF standard at a temperature of 16-26 °C with a humidity of 40-70% and circulating light (12 hours in light and dark respectively), and were free to eat and drink water.

Animals were detected for HBV HBsAg before grouping, and randomly grouped according to the expression level of HBV HBsAg, and the average level of HBV HBsAg in various groups was kept as consistent as possible. The mice were divided into 10 groups with 5 mice in each group, including the control group (normal saline) and the administration groups (9 groups). The administration dose was 3mg/kg with single administration, and the day of administration was set as d0. Mice in each group were administered the corresponding test solution by subcutaneous injecton at 0.04mL/10g on d0. The animals were observed for 6 weeks, and the blood was collected on d0, d7, d14, d21, d28, d35 and d42. At each blood collection time for each group, whole blood was collected through the orbital venous plexus of mice, and centrifuged at 3000×g for 5 min, and the supernatant was sampled to detect the expression level of HBV HBsAg.

| Group | Number of mice/group | Administration time | Dose | Blood collection time point | Route of administration |
|---|---|---|---|---|---|
| control | 5 | d0 | - | d0, d7, d14, d21, d35, d42 | subcutaneous injection, single administration |
| Ky-22 | 5 | | 3 mg/kg | | |
| Ky-2201 | 5 | | | | |
| Ky-2202 | 5 | | | | |
| Ky-2203 | 5 | | | | |
| Ky-2204 | 5 | | | | |
| Ky-2205 | 5 | | | | |
| Ky-2206 | 5 | | | | |
| Ky-2207 | 5 | | | | |
| Ky-2208 | 5 | | | | |

HBsAg levels of the animals in each administration group were normalized to those before administration and the control group.

The experimental data were shown in Fig. 14. The experimental results showed that, compared with Ky-22, Ky-2201 with a sense strand length of 21-mer had no significant improvement in reducing the HBsAg level and the persistence of the effect, and had even slightly decreased effects, so the length of the sense strand of the RNA inhibitor of the present invention is most preferably 19-mer. Compared with Ky-22, Ky-2203 that has one nucleotide change in each of the sense strand and the antisense strand, had no significant difference in reducing the HBsAg level and the persistence of the effect. Ky-2204 that has a sense strand with a length of 21-mer and is based on the Ky-2203 design, had no significant difference from Ky-2203 in effect. Ky-2208 which was obtained by adjusting the number of fluorine substitution on the basis of Ky-2203 and has a relatively small number of fluorine substitution, had an effect slightly better than Ky-2203. The RNA inhibitors Ky-2205 and Ky-2206 that were obtained by transforming the two overhanging nucleotides at the 3' end of the sense strand or antisense strand showed no significant difference from those before transformation in effect. The above results indicated that the RNA inhibitor of the present invention allows a difference of 1 to 3 nucleotides in the sense strand or antisense strand. Compared with Ky-22, Ky-2207 obtained by eliminating the thioation of the phosphate bonds between 3 consecutive nucleotides at 5' end of the sense chain and 3' end of the antisense strand had a significant influence on the effect of reducing the HBsAg level and the persistence of the effect.

In the present invention, preferred is a sequence having a sense strand with a length of 19-mer and an antisense strand with a length of 21-mer, allowing a difference of 1 to 3 nucleotides.

### Example 3: Investigation of the dose response of Ky-2208 and the effect of reducing HBsAg by repeated administration of a single dose and whether surface antibody HBsAb can be produced, in AAV-HBV mouse model

Experimental procedures: 36 mice of the appropriate age were raised in a barrier facility for about 7 days and observed daily, and the experiment was carried out after no obvious abnormalities were found. The HBV virus was thawed sequentially at 4 °C, and rAAV8-1.3HBV (Fiveplus Gene Technology Co. Ltd, ayw, virus batch No.: A2020051801) was injected into the tail vein of the mice with an insulin syringe, and each mouse was injected with 1×10¹¹ v.g. Blood was collected on the animals at the 4th week after modeling, and centrifuged, and serum was collected to detect the HBsAg index. At 6 weeks after modeling, blood was collected to detect HBsAg in serum. According to the results of HBsAg detection, 30 mice were selected and randomly divided into 5 groups, and the average level of HBV HBsAg in various groups was kept as consistent as possible. Drug administration began on the 2nd week after grouping, and blood was collected to detect HBsAg on the day of administration, which was set as the day of d0. The drug administration information and blood collection points of various groups were shown in the following table:

| Group | Number of mice/group | Administration time | Dosage | Blood collection time point | Route of administration |
|---|---|---|---|---|---|
| Control | 6 | d0 | - | d0, d7, d14, d21, d28, d35, d42, d49, d63, d70, d77, d91, d98, d105, d112, d126, d133, d140 | subcutaneous injection, single administration |
| Administration 1 | 6 | | 1mg/kg | d0, d7, d14, d21, d35, d42, d49, d63, d77 | subcutaneous injection, single administration |
| Administration 2 | 6 | | 3mg/kg | | subcutaneous injection, single administration |
| Administration 3 | 6 | | 9mg/kg | d0, d7, d14, d21, d35, d42, d49, d63, d77, d91, d98, d126, d133, d140 | subcutaneous injection, single administration |
| Administration 4 | 6 | | 3mg/kg | d0, d7, d14, d21, d35, d49, d63, d70, d98, d105, d112 | subcutaneous injection, once a week, three times in a row |

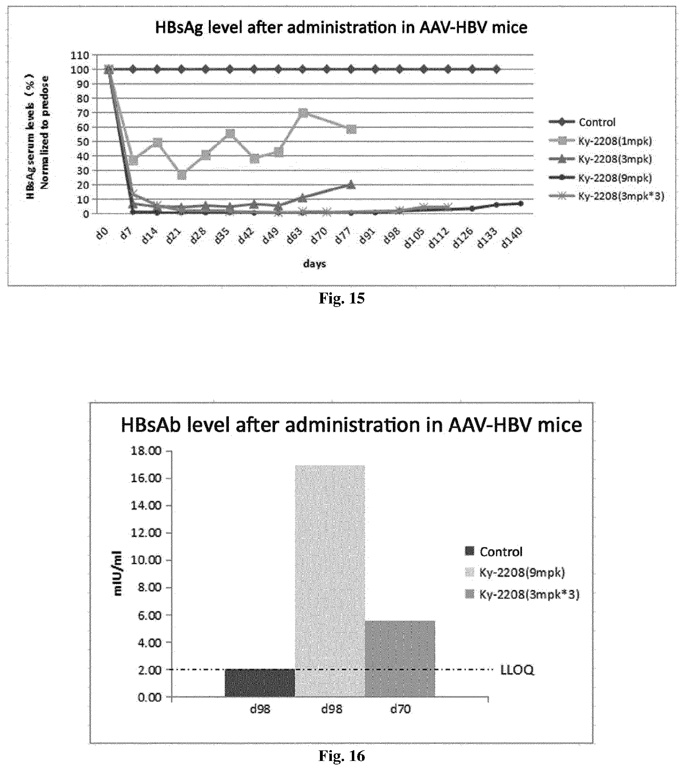

The HBsAg levels of the animals in various administration groups were normalized to those before administration and the control group, and the obtained experimental data for HBsAg and HBsAb were shown in Figs. 15 and 16, respectively.

The experimental results showed that during the entire investigation period of 140 days, the 9mg/kg group of Ky-2208 could reduce the HBsAg level by a range of 93.1% to 99.6% in the AAV-HBV mouse model; when the repeated administration group was investigated by the 112^{th} day, the inhibitory effect remained still more than 95%; by the 98^{th} day, the surface antibody HBsAb had been detected in the HBV model mice for a single administration, and new anti-HBV immunity was generated in the mice.

### Example 4: Comparative study and combined use of Ky-2208 with tenofovir (TDF), the first-line drug currently used in the treatment of chronic hepatitis B, to investigate the HBsAg inhibitory effect on HBV and whether there is interference, in the HBV transgenic mouse model

Experimental procedures: 48 HBV-Tg male mice with a body weight of 25-35g and a week age of 8-13w, were raised in an animal room that meets the SPF standard, with a temperature of 16-26 °C, a humidity of 40-70% and circulating light (12 hours in light and dark respectively), and were free to eat and drink water. The solvent for formulation of the compound was normal saline, and the concentration of the working solution was 0.75 mg/mL. HBV HBsAg was detected before animal grouping, and 48 male mice were randomly divided into 6 groups according to the expression level of HBV HBsAg with 8 mice in each group, and the average level of HBV HBsAg in various groups was kept as consistent as possible. The experiment consisted of 6 groups, including one control group (0.9% normal saline) and 5 administration groups. The drug was administered once on the day of d0, and mice in each group were subcutaneously injected with 0.04 mL/10g of the corresponding test solution on d0. Whole blood was collected through the orbital venous plexus of mice on d0 before administration and d7, d14, d21 and d28 after administration, and centrifuged at 3000×g for 5 minutes. Supernatant was sampled on d0, d7, d14, d21 and d28 to detect HBV HBsAg.

The specific dosage regimens were shown in the table below:

| No. | Test drug | Dosage | Number of mice/group | Route of administration | Dosing frequency | solvent |
|---|---|---|---|---|---|---|
| 1 | normal saline | / | 8 | sc | single | normal saline |
| 2 | TDF | 15mpk | 8 | po | daily | |
| 3 | Ky-2208 | 3mpk | 8 | sc | single | |
| 4 | Ky-2208 | 9mpk | 8 | sc | single | |
| 5 | Ky-2208 +TDF | 3 mpk +15 mpk | 8 | sc/po | Ky-2208 single /TDF daily | |
| 6 | Ky-2208 + TDF | 9 mpk + 15 mpk | 8 | sc/po | Ky-2208 single /TDF daily | |

| | | | | | | |
|---|---|---|---|---|---|---|
| Remarks: sc refers to subcutaneous injection, po refers to gavage. | | | | | | |

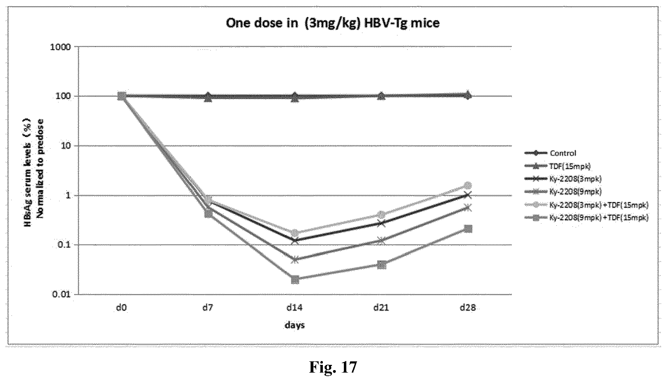

The experimental data obtained were shown in Fig. 17. The experimental results confirmed that the nucleoside analog anti-hepatitis B drug TDF had no inhibitory effect on HBV HBsAg, and when used in combination, it does not affect the inhibitory effect of the RNA inhibitor of the present invention on HBsAg. Ky-2208 used alone or combined with TDF can reduce the HBsAg level by 99.95% and 99.98%, respectively.

## Claims

1. A RNA inhibitor for inhibiting gene expression of hepatitis B virus or a pharmaceutically acceptable salt thereof, wherein, the RNA inhibitor is formed of a sense strand and an antisense strand with a chain length of 15-30, preferably 19-23, by means of base pairing.

2. The RNA inhibitor or a pharmaceutically acceptable salt thereof according to claim 1, wherein,
the sense strand and the antisense strand are at least 85% base complementary to each other;
the -OH at 2' position of glycosyl of some or all of nucleotides of the sense strand and the antisense strand may be replaced, wherein the replacing group is fluorine or methoxy; and
the phosphate bonds between at least 3 adjacent nucleotides at the end of the sense strand or antisense strand may be thioated.

3. The RNA inhibitor or a pharmaceutically acceptable salt thereof according to claim 2, wherein, the sense strand is SEQ ID NO. 1 or a sequence that differs from SEQ ID NO. 1 by one, two or three nucleotides; the antisense strand is SEQ ID NO. 58 or a sequence that differs from SEQ ID NO. 58 by one, two or three nucleotides:
| | | |
|---|---|---|
| Sense strand: | 5' ggguuuuucucguugacaa 3' | SEQ ID NO. 1 |
| Antisense strand: | 5' uugucaacgagaaaaacccuu 3' | SEQ ID NO. 58 |
wherein, g = guanosine, a = adenosine, u = uridine, c = cytidine.

4. The RNA inhibitor or a pharmaceutically acceptable salt thereof according to claim 3, wherein, the sense strand is SEQ ID NO. 2 or a sequence that differs from SEQ ID NO. 2 by one, two or three nucleotides; the antisense strand is SEQ ID NO. 59 or a sequence that differs from SEQ ID NO. 59 by one, two or three nucleotides:
| | | |
|---|---|---|
| sense strand: | 5' Gs fGs G U fU U fU fU fC U C G U U G A Cs As A 3' | SEQ ID NO. 2 |
| antisense strand: | 5' Us Us G U C A fA C G A G fA A fA fA A C C Cs Us U 3' | SEQ ID NO. 59 |
wherein, G=2'-O-methylguanosine, A=2'-O-methyladenosine, U=2'-O-methyluridine, C=2'-O-methylcytidine; Gs=2'-O-methylguanosine-3'-phosphorothioate, As=2'-O-methyladenosine-3'-phosphorothioate, Us=2'-O-methyluridine-3'-phosphorothioate, Cs=2'-O-methylcytidine-3'-phosphorothioate; fG=2'-fluoroguanosine, fA=2'-fluoroadenosine, fU=2'-fluorouridine, fC=2'-fluorocytidine; fGs=2'-fluoroguanosine-3'-phosphorothioate, fAs=2'-fluoroadenosine-3'-phosphorothioate, fUs=2'-fluorouridine-3'-phosphorothioate, fCs=2'-fluorocytidine-3'-phosphorothioate.

5. The RNA inhibitor or a pharmaceutically acceptable salt thereof according to claim 2 or 3, wherein, the sense strand is SEQ ID NO. 140 or a sequence that differs from SEQ ID NO. 140 by one, two or three nucleotides; the antisense strand is SEQ ID NO. 141 or a sequence that differs from SEQ ID NO. 141 by one, two or three nucleotides:
| | | |
|---|---|---|
| Sense strand: | 5' ggguuuuucuuguugacaa 3' | SEQ ID NO. 140 |
| Antisense strand: | 5' uugucaacaagaaaaacccuu 3' | SEQ ID NO. 141 |
wherein, g = guanosine, a = adenosine, u = uridine, c = cytidine.

6. The RNA inhibitor or a pharmaceutically acceptable salt thereof according to claim 5, wherein, the sense strand is SEQ ID NO. 142 or a sequence that differs from SEQ ID NO. 142 by one, two or three nucleotides; the antisense strand is SEQ ID NO. 143 or a sequence that differs from SEQ ID NO. 143 by one, two or three nucleotides:
| | | |
|---|---|---|
| Sense strand: | 5' Gs Gs G U fU U fU fU fC U U G U U G A Cs As A 3' | SEQ ID NO. 142 |
| Antisense strand: | 5'Us Us G U C A fA C A A G fA A fA A A C C Cs Us U 3' | SEQ ID NO. 143 |
wherein, G=2'-O-methylguanosine, A=2'-O-methyladenosine, U=2'-O-methyluridine, C=2'-O-methylcytidine; Gs=2'-O-methylguanosine-3'-phosphorothioate, As=2'-O-methyladenosine-3'-phosphorothioate, Us=2'-O-methyluridine-3'-phosphorothioate, Cs=2'-O-methylcytidine-3'-phosphorothioate; fG=2'-fluoroguanosine, fA=2'-fluoroadenosine, fU=2'-fluorouridine, fC=2'-fluorocytidine; fGs=2'-fluoroguanosine-3'-phosphorothioate, fAs=2'-fluoroadenosine-3'-phosphorothioate, fUs=2'-fluorouridine-3'-phosphorothioate, fCs=2'-fluorocytidine-3'-phosphorothioate.

7. The RNA inhibitor or a pharmaceutically acceptable salt thereof according to any one of claims 1-6, wherein the RNA inhibitor further comprises a combination of 5'MVIP and 3'MVIP, wherein,
the 5'MVIP and 3'MVIP are ligand structures with a liver targeting specific ligand X, and further comprise a branched chain L, a linker B and a linking chain D;
the 5'MVIP is coupled to the 5' end of the sense strand and/or the antisense strand, and further comprises a transition point R₁ connected to the 5' end of the sense strand or antisense strand;
the 3'MVIP is coupled to the 3' end of the antisense strand and/or the sense strand, and further comprises a transition point R₂ connected to the 3' end of the sense strand or antisense strand;
the 5'MVIP has a structure as shown in general formula I, and the 3'MVIP has a structure as shown in general formula II,
**(X-L)ₙ-B-D-R₁-** I
**(X-L)ₘ-B-D-R₂-** II
wherein,
n and m are respectively an integer of 0 to 4, preferably 1 to 3, and n+m is an integer of 2 to 6, preferably 2, 3 or 4;
the transition points R₁ and R₂ have a structure containing -NH-, sulfur atom or oxygen atom, and generally at least one -NH-, sulfur atom or oxygen atom is in the structure, R₁ and R₂ are linked to the linking chain D of 5'MVIP and 3'MVIP, and the 5' end and the 3' end of the sense strand and/or the antisense strand respectively through the -NH-, sulfur atom or oxygen atom in the structure; the transition points R₁ and R₂ may be a straight chain; a straight chain with an amide, carboxyl or alkyl branch, or various cyclic structures, such as saturated or unsaturated aliphatic carbocyclyl, or 5- or 6-membered heterocyclyl or aromatic hydrocarbonyl containing sulfur, oxygen or nitrogen atom;
R₁ is preferably -NH(CH₂)ₓCH₂O-, wherein x is an integer of 3 to 12, preferably 4 to 6;
R₂ is preferably -NH(CH₂)ₓ₁CH(OH)(CH₂)ₓ₂CH₂O-, wherein x1 is an integer of 1 to 4, and x2 is an integer of 0 to 4;
the liver targeting specific ligand X is selected from galactose, galactosamine, N-acetylgalactosamine and derivatives thereof, preferably selected from N-acetylgalactosamine and derivatives thereof, and the liver target specific ligands X within each of the 5'MVIP and the 3'MVIP or between the 5'MVIP and the 3'MVIP may be the same or different;
the branched chain L is a C4-C18 straight chain containing -NH-, C=O, O, S, amide group, phosphoryl, thiophosphoryl, C4-C10 aliphatic carbocyclyl, phenyl or a combination thereof, the C4-C18 straight chain may have a side chain of ethyl alcohols or carboxylic acids, the branched chain L is preferably a C7-C18 straight chain containing an amide group or a six-membered aliphatic carbocyclyl, and the branched chains L within each of the 5'MVIP and the 3'MVIP or between the 5'MVIP and the 3'MVIP may be the same or different;
the linker B is selected from the following structural formulae: 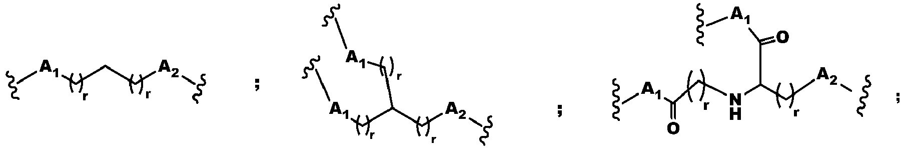 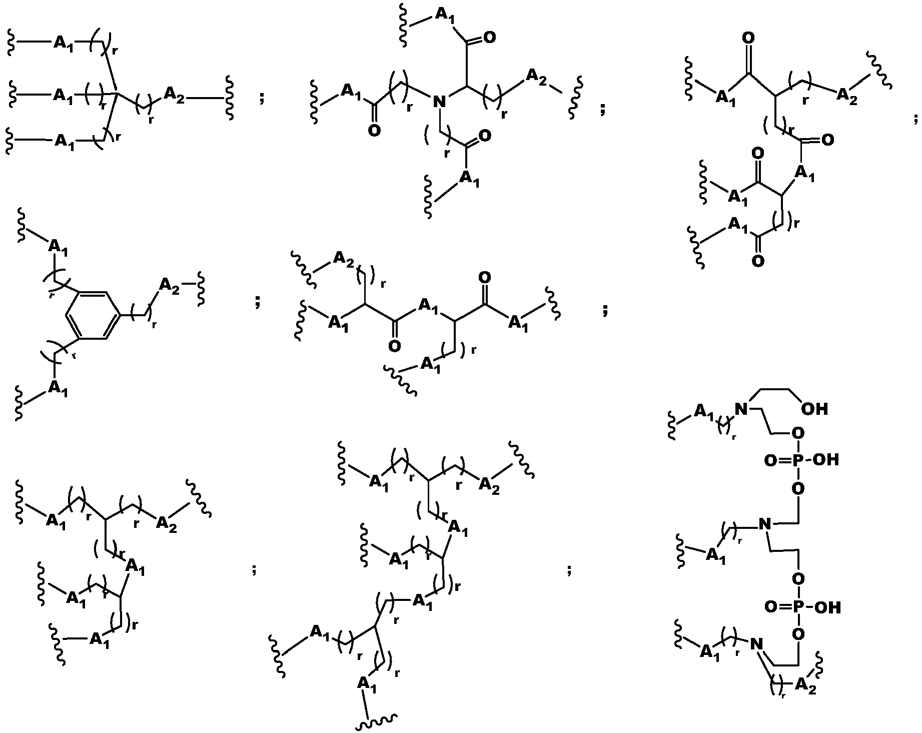
wherein, A₁ and A₂ are each independently C, O, S, -NH-, carbonyl, amide group, phosphoryl or thiophosphoryl, r is an integer of 0 to 4, and the linkers B between the 5'MVIP and the 3'MVIP may be the same or different;
the linking chain D is a C3-C18 straight chain containing -NH-, C=O, O, S, amide group, phosphoryl, thiophosphoryl, aromatic hydrocarbonyl, C4-C10 aliphatic carbocyclyl, 5- or 6-membered heterocyclyl containing 1 to 3 nitrogens or a combination thereof, the C3-C18 straight chain may have a side chain of methyl alcohol, methyl tert-butyl, methyl phenol, or C5-C6 alicyclyl, the linking chain D is preferably a C3-C10 straight chain containing two C=O, 6-membered aliphatic carbocyclyl or phenyl, most preferably a C3-C10 straight chain containing two C=O.

8. The RNA inhibitor or a pharmaceutically acceptable salt thereof according to claim 7, wherein, the 5'MVIP is 5'MVIP01 or 5'MVIP09 as shown below, and the 3'MVIP is 3'MVIP01, 3'MVIP09 or 3'MVIP17 as shown below:

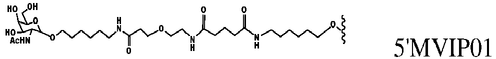

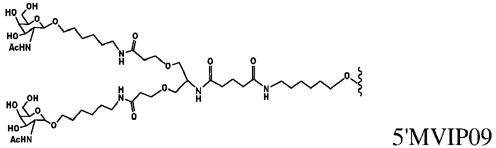

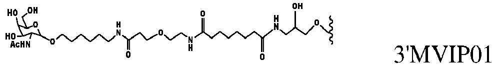

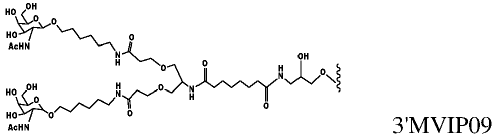

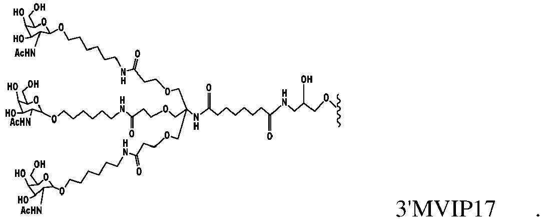

9. The RNA inhibitor or a pharmaceutically acceptable salt thereof according to claim 8, wherein, the combination of the sense strand 5'MVIP and the antisense strand 3'MVIP is 5'MVIP01/3'MVIP01, 5'MVIP01/3'MVIP17 or 5'MVIP09/3'MVIP09, or the combination of the sense strand 5'MVIP and the sense strand 3'MVIP is 5'MVIP01/3'MVIP09 or 5'MVIP09/3'MVIP01.

10. Use of the RNA inhibitor or a pharmaceutically acceptable salt thereof according to any one of claims 1 to 9 in preparation of a medicament for treatment of a hepatogenic disease, which includes, but not limited to, hepatitis, liver tumors, cirrhosis, jaundice, type 2 diabetes, fatty liver, coagulation diseases of blood system, diseases related to blood albumin and globulin, hyperlipidemia, atherosclerosis, and essential hypertension.

11. A pharmaceutical composition comprising the RNA inhibitor or a pharmaceutically acceptable salt thereof according to any one of claims 1 to 9, and a pharmaceutically acceptable auxiliary material, the dosage form of which is an oral agent, an intravenous injection or a subcutaneous or intramuscular injection, preferably a subcutaneous injection.

12. A pharmaceutical composition comprising the RNA inhibitor or a pharmaceutically acceptable salt thereof according to any one of claims 1 to 9, and a nucleoside analog or interferon that is a drug for treatment of chronic hepatitis B.
